# EUROPEAN PATENT APPLICATION

(11) **EP 4 778 920 A1**
(43) Date of publication of application: **22.07.2026**
(21) Application number: 24864788.5
(22) Date of filing: 14.09.2024
(51) Int. Cl.: C07D 401/04, C07D 401/14, C07D 417/14, C07D 413/14, C07D 405/14, A61K 31/454, A61K 31/4545, A61K 31/4709, A61P 35/00, A61P 35/02

(54) **INDAZOLONE COMPOUND, PREPARATION METHOD THEREFOR, PHARMACEUTICAL COMPOSITION, AND USE**

(30) Priority: 14.09.2023 CN 202311187240
(71) Applicant: Shanghai Institute of Materia Medica, Chinese Academy of Sciences, Shanghai 201203 (CN)
(72) Inventor: CHEN, Xiaohua, Shanghai 201203 (CN); LI, Jia, Shanghai 201203 (CN); NIE, Huijun, Shanghai 201203 (CN); ZHOU, Chucheng, Shanghai 201203 (CN); WANG, Jiamin, Shanghai 201203 (CN); HU, Tengfei, Shanghai 201203 (CN); KAN, Weijuan, Shanghai 201203 (CN)
(74) Representative: Lavoix
(86) International application number: PCT/CN2024/119210
(87) International publication number: WO 2025/056073

(57) **Abstract**

Disclosed in the present invention are an indazolone compound, a preparation method therefor, a pharmaceutical composition, and a use. Specifically, provided are a compound of formula (I), or a pharmaceutically acceptable salt, tautomer, stereoisomer, isotopic compound, pharmaceutically usable salt, ester, prodrug or hydrate thereof, a preparation method therefor, a pharmaceutical composition, and a use. The compound of the present invention has good anti-tumor activity as a CRL4^{CRBN}E3 ubiquitin ligase modulator, and can be used in the preparation of a drug for treating CRL4^{CRBN}E3 ubiquitin ligase-related diseases.

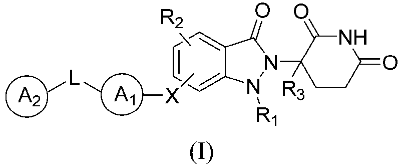

## Description

### Technical Field

The present invention relates to the field of medicinal chemistry, and in particular to a class of indazolone derivatives, preparation methods thereof, pharmaceutical compositions comprising the same, and uses thereof.

### Background

Targeted protein degradation is a promising field for the discovery and development of innovative therapeutics. Targeted protein degradation technology breaks through the mechanism of action of traditional small-molecule inhibitors and can directly induce the degradation of substrate proteins through the ubiquitin-proteasome system (UPS) in vivo. At present, targeted protein degradation technologies based on the ubiquitin-proteasome system (UPS) mainly include proteolysis targeting chimeras (PROTACs) and molecular glues.

A molecular glue is a small molecule that induces protein-protein interactions and can precisely control various biological processes in a time-dependent manner. Molecular glues act at protein interfaces to form new protein-protein interactions. Molecular glue-mediated proximity-induced protein degradation possesses inherent advantages over heterobifunctional proteolysis targeting chimeras, including unprecedented mechanisms of action, unique biological activities, and favorable physicochemical properties. However, the scarcity of serendipitously discovered molecular glues has hindered their development as reagents for biological detection and disease treatment. Therefore, rational discovery and design strategies are essential for the development of molecular glue drugs.

At present, CRBN-based molecular glue degraders are mainly immunomodulatory drugs (IMiDs), such as thalidomide, lenalidomide, and pomalidomide. Due to the favorable drug-like properties of CRBN ligands, immunomodulatory imide drugs (IMiDs), targeting CRBN has become a major approach for molecular degraders. However, IMiDs are inherently unstable and are prone to hydrolysis in body fluids. For example, thalidomide can undergo racemization and hydrolysis in body fluids, and the rate increases with increasing pH. Accordingly, the hydrolysis half-life of thalidomide is about 11, 5, and 1.25 hours at pH 7, 7.4, and 8, respectively. Therefore, the development of small molecules targeting CRBN with favorable pharmaceutical properties is an important direction in the design of molecular glue degraders.

Lenalidomide is currently mainly used for the treatment of multiple myeloma and myelodysplastic syndrome, but its efficacy for other indications is not satisfactory. Therefore, the development of a class of structurally novel molecular glue degraders may improve therapeutic effects against tumors and expand new indications, thereby addressing unmet clinical needs. Accordingly, the development of novel ligands and new molecular glues targeting the CRL4^{CRBN} E3 ubiquitin ligase is of significant research value and practical importance for new drug development.

### Summary of the Invention

Through literature investigation, the inventors found that indazolone compounds and their derivatives possess unique biological activities and exhibit good metabolic stability. In addition, based on analysis of the crystal structures of thalidomide-like compounds bound to the CRL4^{CRBN} E3 ubiquitin ligase, the inventors conducted molecular docking and computational simulations. The analyses revealed that derivatives formed by combining an indazolone moiety with a glutarimide ring to generate a new scaffold, namely 3-(3-oxo-1,3-dihydro-2H-indazol-2-yl)piperidine-2,6-dione, can bind more favorably to the pocket of the CRL4^{CRBN} E3 ubiquitin ligase while reducing steric repulsion with carbonyl groups on nearby peptide chains of the binding protein. Therefore, this scaffold can serve as a novel ligand structure with favorable binding to the CRL4^{CRBN} E3 ubiquitin ligase.

Accordingly, based on the favorable bioavailability and drug metabolism properties of indazolone structures, the inventors designed a new scaffold by combining indazolone with a glutarimide ring. The resulting indazolone-derived glutarimide structure can bind effectively to the CRL4^{CRBN} E3 ubiquitin ligase. Through structural modification, a variety of structurally novel molecular glue degraders targeting the CRL4^{CRBN} E3 ubiquitin ligase can be obtained.

The objective of the present invention is to provide a class of CRL4^{CRBN} E3 ubiquitin ligase molecular glue degraders having a novel indazolone scaffold, which exhibit improved antitumor therapeutic effects and broaden the therapeutic indications of molecular glue degraders. In addition, the invention provides ligands for the CRL4^{CRBN} E3 ubiquitin ligase with improved physicochemical properties for the design and development of degraders targeting disease-related proteins.

In one aspect, the present invention provides a compound represented by general formula (I), or an enantiomer, diastereomer, racemate, isotopic compound, metabolic precursor, metabolite, pharmaceutically acceptable salt, ester, prodrug, or hydrate thereof.

In another aspect, the present invention provides methods for preparing the compounds represented by general formula (I), as well as important intermediates for preparing such compounds and methods for preparing the intermediates.

In another aspect, the present invention provides the compounds represented by general formula (I), and their tautomers, enantiomers, diastereomers, racemates, metabolites, metabolic precursors, isotopic compounds, pharmaceutically acceptable salts, esters, prodrugs, or hydrates, characterized in that the compounds are used in the preparation of a medicament or diagnostic reagent for preventing or treating diseases associated with the CRL4^{CRBN} E3 ubiquitin ligase; preferably, the diseases associated with the CRL4^{CRBN} E3 ubiquitin ligase include cancer, pain, central nervous system diseases, and immune system diseases.

In order to achieve the above objectives, the present invention provides a compound represented by general formula (I), or pharmaceutically acceptable salts, tautomers, stereoisomers, isotopic compounds, pharmaceutically acceptable salts, esters, prodrugs, or hydrates thereof:

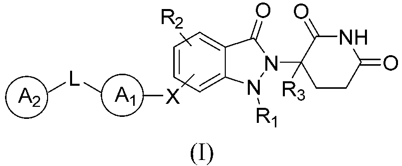

Wherein:
R₁ is hydrogen, methyl or ethyl;
R₂ is hydrogen or fluorine;
R₃ is hydrogen, deuterium, or fluorine;
L is absent, -CH₂-, S, -CO-, or -CH(CH₃)-;
The X-A₁ combination is selected from -(CH₂)ₙ-O-CH₂-A₁, -(CH₂)ₙ-NHCONH-A₁, -(CH₂)ₙ-NHCOCF₂-A₁, -CONH-(CH₂)ₙ-A₁, -CONH-CO-A₁, -CONHCH(CH₃)-A₁, and -CONHCH(CF₃)-A₁;
n is 0 or 1;
When the X-A₁ combination is -(CH₂)ₙ-O-CH₂-A₁, L is absent, -CH₂-, S, -CO-, or -CH(CH₃)-;
A₁ is selected from halogen-substituted or unsubstituted phenyl, halogen-substituted or unsubstituted pyridyl, and five-membered heteroaryl containing N, O, or S;
A₂ is selected from phenyl, pyridyl, piperazinyl, piperidinyl, morpholinyl, or azetidinyl; wherein the phenyl, pyridyl, piperazinyl, piperidinyl, or morpholinyl is optionally substituted with one or more groups selected from hydrogen, deuterium, halogen, cyano, nitro, hydroxyl, carboxyl, aminocarbonyl, a 4-10 membered heterocyclic group, C₁-C₆ alkyl, C₁-C₆ alkoxy, halogen-substituted C₁-C₆ alkyl, halogen-substituted C₁-C₆ alkoxy, substituted or unsubstituted phenyl, substituted or unsubstituted pyridyl, C₃-C₈ heterocycloalkoxy, C₃-C₈ cycloalkoxy, hydroxy-substituted C₁-C₆ alkoxy, and C₁-C₆ alkoxy-substituted C₁-C₆ alkoxy;
wherein the substituted or unsubstituted phenyl or pyridyl is optionally substituted with one or more groups selected from hydrogen, halogen, cyano, a 4-10 membered heterocyclic group, C₁-C₆ alkyl, C₁-C₆ alkoxy, halogen-substituted C₁-C₆ alkyl, and halogen-substituted C₁-C₆ alkoxy.

When the X-A₁ combination is -(CH₂)ₙ-NHCONH-A₁ or -(CH₂)ₙ-NHCOCF₂-A₁, L and A₂ are both absent;
A₁ is phenyl optionally substituted with one or more groups selected from hydrogen, halogen, cyano, or C₁-C₆ alkyl.

When the X-A₁ combination is -CONHCH(CH₃)-A₁, -CONH-(CH₂)ₙ-A₁, -CONH-CO-A₁, or-CONHCH(CF₃)-A₁, L and A₂ are both absent;
A₁ is selected from phenyl, pyridyl, 5-10 membered heteroaryl, C₃-C₁₀ cycloalkyl, 4-6 membered heterocyclyl, benzofused 4-6 membered heterocyclyl, or C₁-C₆ alkyl; wherein the C₆-₁₀ aryl, 5-10 membered heteroaryl, C₃-C₁₀ cycloalkyl, 4-6 membered heterocyclyl, or C₁-C₆ alkyl is optionally substituted with one or more groups selected from hydrogen, deuterium, halogen, cyano, a 4-10 membered heterocyclic group, C₁-C₆ alkyl, C₁-C₆ alkoxy, halogen-substituted C₁-C₆ alkyl, and halogen-substituted C₁-C₆ alkoxy.

In another preferred embodiment:
R₁ is hydrogen, methyl or ethyl;
R₂ is hydrogen or fluorine;
R₃ is hydrogen, deuterium, or fluorine;
L is absent, -CH₂-, S, -CO-, or -CH(CH3)-;
The X-A₁ combination is selected from -(CH₂)ₙ-O-CH₂-A₁, -(CH₂)ₙ-NHCONH-A₁, -(CH₂)ₙ-NHCOCF₂-A₁, -(CH₂)ₙ-NHCH₂-A₁, -CONH-(CH₂)ₙ-A₁, -CONHCH(CH₃)-A₁, -CONHCH(CF₃)-A₁, or X is absent;
n is 0 or 1.
When the X-A₁ combination is -(CH₂)ₙ-O-CH₂-A₁, L is absent, -CH₂-, S, -CO-, or -CH(CH₃)-;
A₁ is selected from halogen-substituted or unsubstituted phenyl, halogen-substituted or unsubstituted pyridyl, and five-membered heteroaryl containing N, O, or S;
A₂ is selected from phenyl, pyridyl, piperazinyl, piperidinyl, or morpholinyl; wherein the phenyl, pyridyl, piperazinyl, piperidinyl, or morpholinyl is optionally substituted with one or more groups selected from hydrogen, deuterium, halogen, cyano, nitro, hydroxyl, carboxyl, aminocarbonyl, a 4-10 membered heterocyclic group, C₁-C₆ alkyl, C₁-C₆ alkoxy, halogen-substituted C₁-C₆ alkyl, halogen-substituted C₁-C₆ alkoxy, substituted or unsubstituted phenyl, substituted or unsubstituted pyridyl, C₃-C₈ heterocycloalkoxy, C₃-C₈ cycloalkoxy, hydroxy-substituted C₁-C₆ alkoxy, and C₁-C₆ alkoxy-substituted C₁-C₆ alkoxy;
wherein the substituted or unsubstituted phenyl or pyridyl is optionally substituted with one or more groups selected from hydrogen, halogen, cyano, a 4-10 membered heterocyclic group, C₁-C₆ alkyl, C₁-C₆ alkoxy, halogen-substituted C₁-C₆ alkyl, and halogen-substituted C₁-C₆ alkoxy.

When the X-A₁ combination is -(CH₂)ₙ-NHCONH-A₁ or -(CH₂)ₙ-NHCOCF₂-A1, L and A₂ are both absent;
A₁ is phenyl optionally substituted with one or more groups selected from hydrogen, halogen, cyano, or C₁-C₆ alkyl.

When the X-A₁ combination is -CONHCH(CH₃)-A₁, -CONH-(CH₂)ₙ-A₁, or -CONHCH(CF₃)-A₁, L and A₂ are both absent;
A₁ is selected from phenyl, pyridyl, 5-10 membered heteroaryl, C₃-C₁₀ cycloalkyl, 4-6 membered heterocyclyl, or C₁-C₆ alkyl; wherein the C₆₋₁₀ aryl, 5-10 membered heteroaryl, C₃-C₁₀ cycloalkyl, 4-6 membered heterocyclyl, or C₁-C₆ alkyl is optionally substituted with one or more groups selected from hydrogen, deuterium, halogen, cyano, a 4-10 membered heterocyclic group, C₁-C₆ alkyl, C₁-C₆ alkoxy, halogen-substituted C₁-C₆ alkyl, and halogen-substituted C₁-C₆ alkoxy.

In another preferred embodiment, the compound has a structure represented by formulae (I-1)-(I-11):

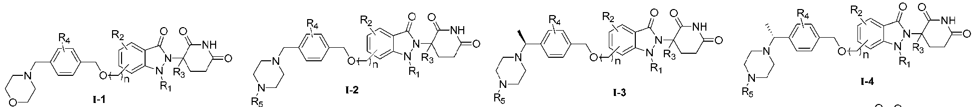

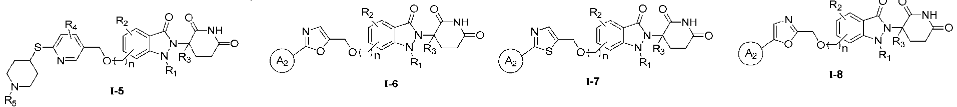

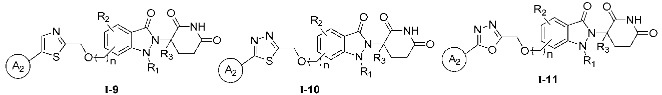

Wherein:
R₁ is hydrogen, methyl or ethyl;
R₂ is hydrogen or fluorine;
R₃ is hydrogen, deuterium or fluorine;
R₄ is H or halogen;
n is 0 or 1;
R₅ is selected from phenyl, pyridyl or morpholinyl, wherein the phenyl, pyridyl or morpholinyl is optionally substituted with one or more groups selected from hydrogen, halogen, cyano, a 4-10 membered heterocyclic group, C₁-C₆ alkyl, C₁-C₆ alkoxy, halogen-substituted C₁-C₆ alkyl, and halogen-substituted C₁-C₆ alkoxy;
A₂ is selected from phenyl or pyridyl, wherein the phenyl or pyridyl is optionally substituted with one or more groups selected from hydrogen, deuterium, halogen, cyano, a 4-10 membered heterocyclic group, C₁-C₆ alkyl, C₁-C₆ alkoxy, halogen-substituted C₁-C₆ alkyl, halogen-substituted C₁-C₆ alkoxy, C₃-C₈ heterocycloalkoxy, C₃-C₈ cycloalkoxy, hydroxy-substituted C₁-C₆ alkoxy, and C₁-C₆ alkoxy-substituted C₁-C₆ alkoxy.

In another preferred embodiment, the compound has a structure represented by formulae (I-29)-(I-30):

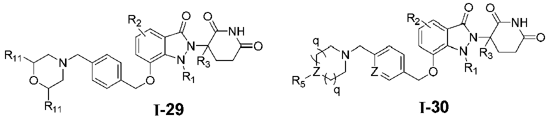

Wherein:
R₁ is hydrogen, methyl or ethyl;
R₂ is hydrogen or fluorine;
R₃ is hydrogen, deuterium or fluorine;
R₁₁ is hydrogen or methyl;
each q is independently 0 or 1;
Z is N or -CH-;
R₅ is selected from phenyl, pyridyl or morpholinyl, wherein the phenyl, pyridyl or morpholinyl is optionally substituted with one or more groups selected from hydrogen, halogen, cyano, a 4-10 membered heterocyclic group, C₁-C₆ alkyl, C₁-C₆ alkoxy, halogen-substituted C₁-C₆ alkyl, and halogen-substituted C₁-C₆ alkoxy;
A₂ is selected from phenyl or pyridyl, wherein the phenyl or pyridyl is optionally substituted with one or more groups selected from hydrogen, deuterium, halogen, cyano, a 4-10 membered heterocyclic group, C₁-C₆ alkyl, C₁-C₆ alkoxy, halogen-substituted C₁-C₆ alkyl, halogen-substituted C₁-C₆ alkoxy, C₃-C₈ heterocycloalkoxy, C₃-C₈ cycloalkoxy, hydroxy-substituted C₁-C₆ alkoxy, and C₁-C₆ alkoxy-substituted C₁-C₆ alkoxy.

In another preferred embodiment, R₁ is hydrogen.

In another preferred embodiment, R₃ is hydrogen or deuterium.

In another preferred embodiment, L is -CH₂-.

In another preferred embodiment, the X-A₁ combination is -(CH₂)ₙ-O-CH₂-A₁.

In another preferred embodiment, n is 0.

In another preferred embodiment, A₁ is halogen-substituted or unsubstituted phenyl.

In another preferred embodiment, R₅ is phenyl, wherein the phenyl is optionally substituted with one or more groups selected from hydrogen, halogen, cyano, C₁-C₆ alkyl, and halogen-substituted C₁-C₆ alkyl.

In another preferred embodiment, the compound has a structure represented by formulae (I-12)-(I-13):

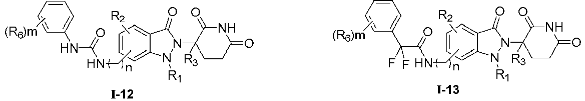

Wherein:
R₁ is hydrogen, methyl or ethyl;
R₂ is hydrogen or fluorine;
R₃ is hydrogen, deuterium or fluorine;
n is 0 or 1;
each R₆ is independently selected from hydrogen, halogen, cyano, or C₁-C₆ alkyl;
m is 0, 1, 2, 3, 4 or 5.

In another preferred embodiment, the compound has a structure represented by formulae (I-31)-(I-32):

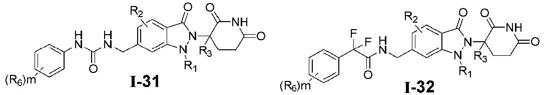

Wherein:
R₁ is hydrogen, methyl or ethyl;
R₂ is hydrogen or fluorine;
R₃ is hydrogen, deuterium or fluorine;
each R₆ is independently selected from hydrogen, halogen, cyano, or C₁-C₆ alkyl;
m is 0, 1, 2, 3, 4 or 5.

In another preferred embodiment, the compound has a structure represented by formulae (I-20)-(I-26):

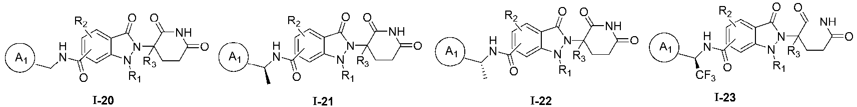

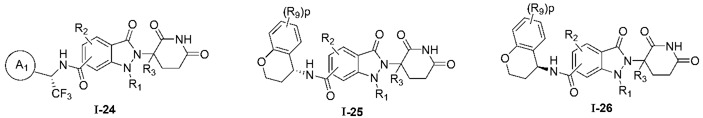

Wherein:
R₁ is hydrogen, methyl or ethyl;
R₂ is hydrogen or fluorine;
R₃ is hydrogen, deuterium or fluorine;
each R₉ is independently selected from hydrogen, halogen, cyano, C₁-C₆ alkyl, C₁-C₆ alkoxy, halogen-substituted C₁-C₆ alkyl, or halogen-substituted C₁-C₆ alkoxy;
p is 0, 1, 2, 3, 4 or 5;
A₁ is selected from phenyl, pyridyl, substituted or unsubstituted C₃-C₁₀ cycloalkyl, 4-6 membered heterocyclyl, or C₁-C₆ alkyl; wherein the phenyl, pyridyl, substituted or unsubstituted C₃-C₁₀ cycloalkyl, 4-6 membered heterocyclyl, or C₁-C₆ alkyl is optionally substituted with one or more groups selected from hydrogen, deuterium, halogen, cyano, a 4-10 membered heterocyclic group, C₁-C₆ alkyl, C₁-C₆ alkoxy, halogen-substituted C₁-C₆ alkyl, halogen-substituted C₁-C₆ alkoxy, C₃-C₈ heterocycloalkoxy, and C₃-C₈ cycloalkoxy.

In another preferred embodiment, 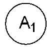 is selected from: 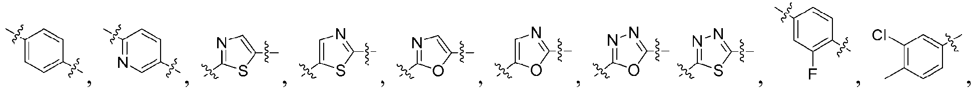 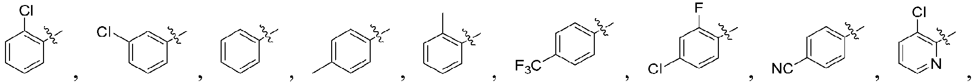 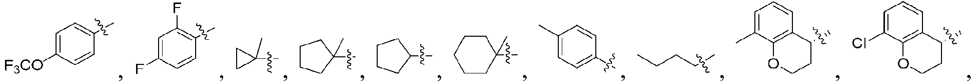 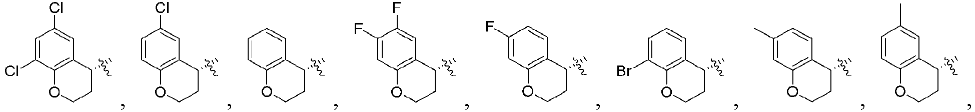 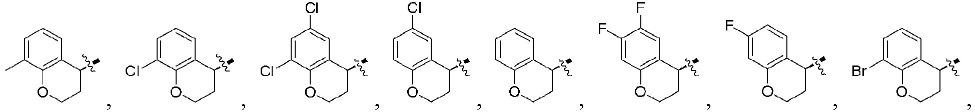 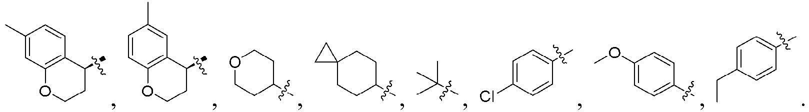 In another preferred embodiment, 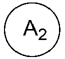 is selected from:

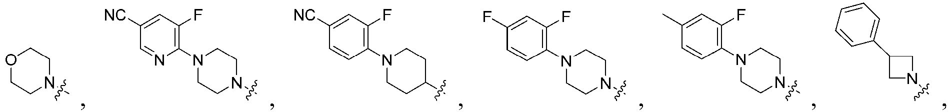

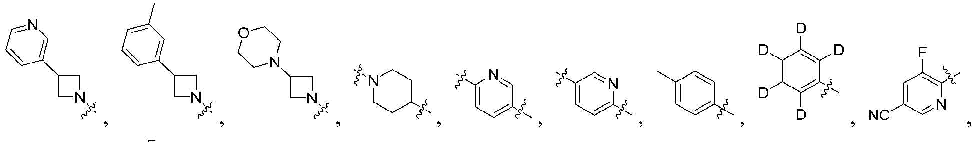

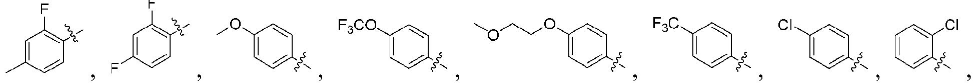

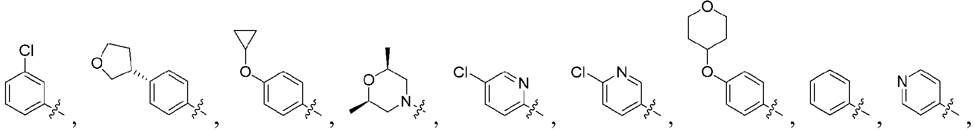

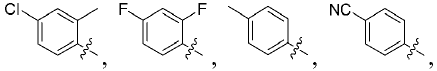

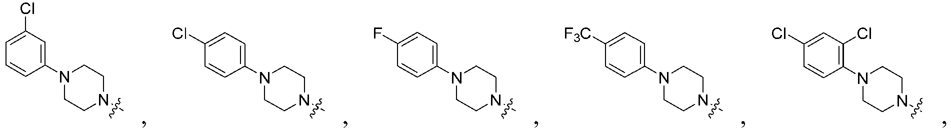

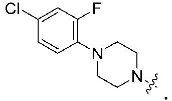

In another preferred embodiment, the compound represented by general formula (I) is one of the following compounds:

| **Compound No.** | **Structure** | **Compound No.** | **Structure** |
|---|---|---|---|
| **D1** | 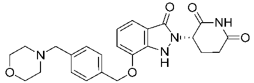 | **D2** | 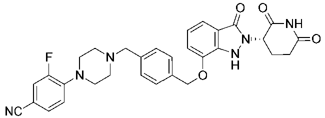 |
| **D3** | 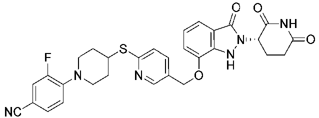 | **D4** | 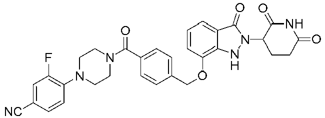 |
| **D5** | 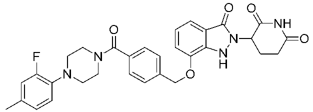 | **D6** | 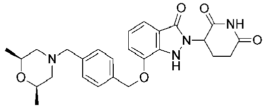 |
| **D7** | 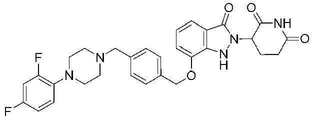 | **D8** | 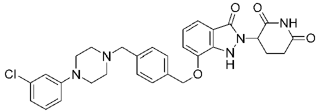 |
| **D9** | 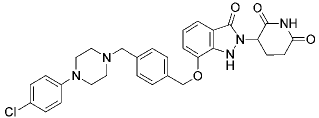 | **D10** | 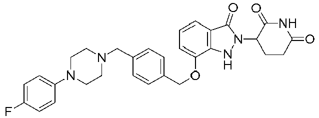 |
| **D11** | 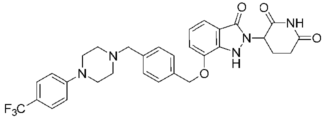 | **D12** | 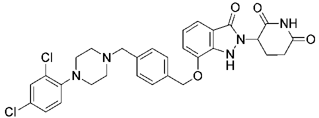 |
| **D13** | 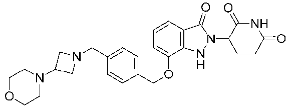 | **D14** | 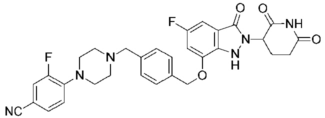 |
| **D15** | 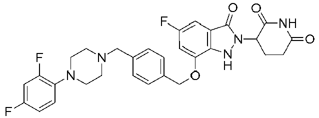 | **D16** | 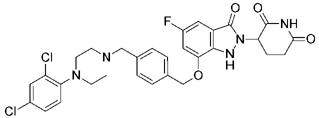 |
| **D17** | 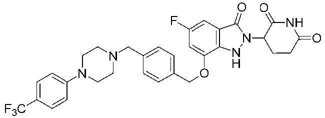 | **D18** | 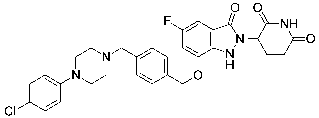 |
| **D19** | 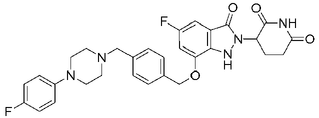 | **D20** | 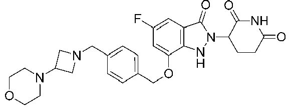 |
| **D21** | 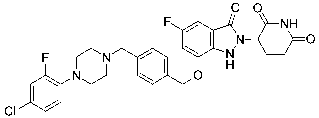 | **D22** | 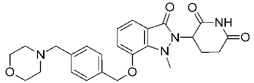 |
| **D23** | 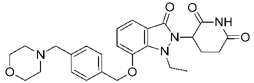 | **D24** | 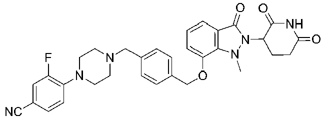 |
| **D25** | 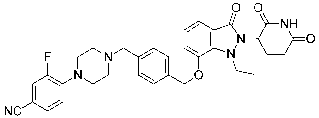 | **D26** | 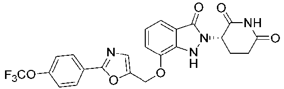 |
| **D27** | 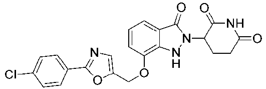 | **D28** | 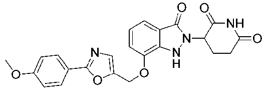 |
| **D29** | 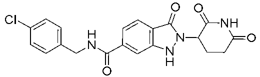 | **D30** | 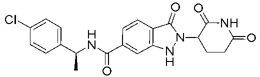 |
| **D31** | 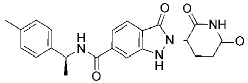 | **D32** | 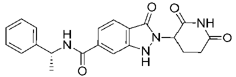 |
| **D33** | 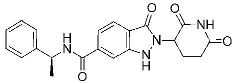 | **D34** | 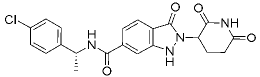 |
| **D35** | 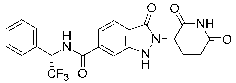 | **D36** | 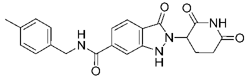 |
| **D37** | 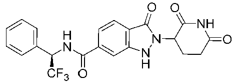 | **D38** | 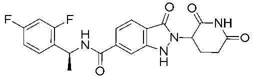 |
| **D39** | 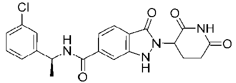 | **D40** | 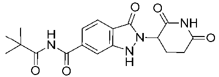 |
| **D41** | 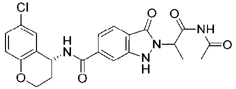 | **D42** | 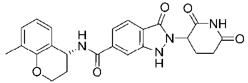 |
| **D43** | 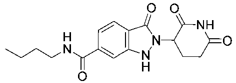 | **D44** | 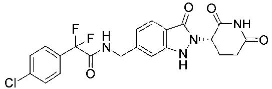 |
| **D45** | 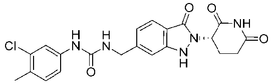 | **D46** | 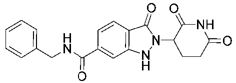 |
| **D47** | 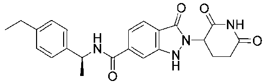 | | |

Compounds, or pharmaceutically acceptable salts, tautomers, stereoisomers, isotopic compounds, pharmaceutically acceptable salts, esters, prodrugs, or hydrates thereof.

In one aspect, the present invention provides a method for preparing the compound represented by general formula (I), wherein the method is selected from one of the following methods:
The synthesis of compounds 1I and 3E may be carried out with reference to the following literature: Organic Letters, 2020, 22(16): 6277-6282; Nature Communications, 2020, 11(1): 1-13; Bioorganic & Medicinal Chemistry Letters, 2013, 23(10): 2936-2940.

### Synthetic Method 1:

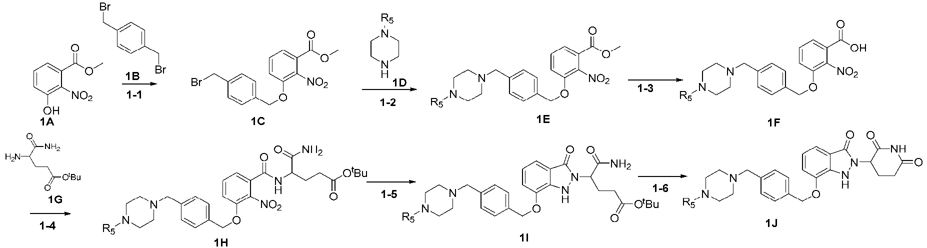

Step 1-1: Compound 1A and compound 1B undergo a nucleophilic substitution reaction to give compound 1C;
Step 1-2: Compound 1C and compound 1D undergo a nucleophilic substitution reaction to give compound 1E;
Step 1-3: Compound 1E is subjected to alkaline hydrolysis to give compound 1F;
Step 1-4: Compound 1F and compound 1G undergo a condensation reaction to give compound 1H;
Step 1-5: Compound 1H reacts with tetrahydroxydiboron and sodium hydroxide to give compound 1I;
Step 1-6: Compound 1I undergoes a reflux reaction with p-toluenesulfonic acid to give compound 1J.
R₅ is as defined above;
Synthetic Method 2: 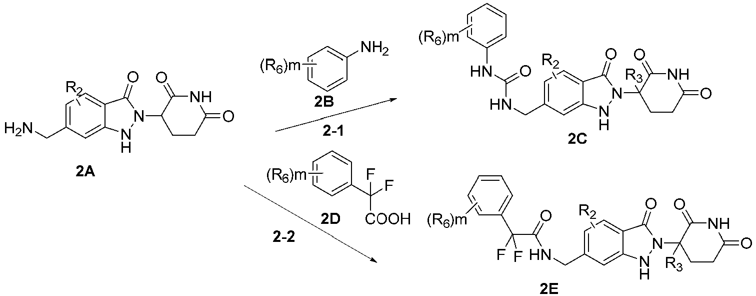
Step 2-1: Compound 2A and compound 2B react in the presence of N,N'-carbonyldiimidazole and triethylamine to give compound 2C;
Step 2-2: Compound 2A and compound 2D react in the presence of HATU and N,N-diisopropylethylamine to give compound 2E.
R₂, R₃, R₆, and m are as defined above.

### Synthetic Method 3:

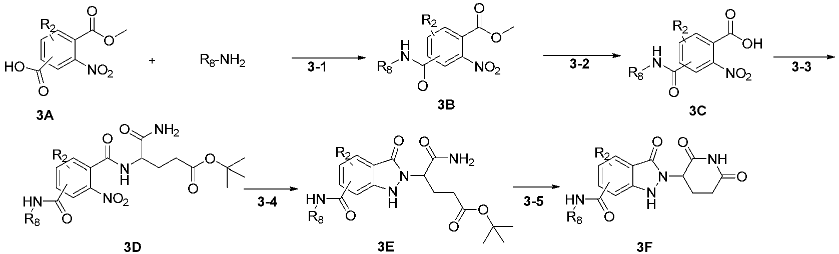

Step 3-1: Compound 3A and R₈-NH₂ undergo a condensation reaction to give compound 3B;
Step 3-2: Compound 3B is hydrolyzed in the presence of lithium hydroxide to give compound 3C;
Step 3-3: Compound 3C and compound 1G undergo a condensation reaction to give compound 3D;
Step 3-4: Compound 3D reacts with tetrahydroxydiboron and sodium hydroxide to give compound 3E;
Step 3-5: Compound 3E undergoes a reflux reaction with p-toluenesulfonic acid to give compound 3F;
R₈-NH₂ is selected from: 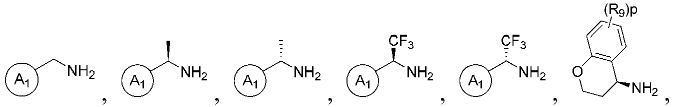 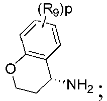 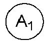 and R₉ are as defined above.

In another aspect, the present invention provides the use of the compound represented by general formula (I), and their tautomers, enantiomers, diastereomers, racemates, metabolites, metabolic precursors, isotopic compounds, pharmaceutically acceptable salts, esters, prodrugs, hydrates, crystalline hydrates, or solvates, in the preparation of a medicament or diagnostic reagent for preventing or treating diseases associated with the CRL4^{CRBN} E3 ubiquitin ligase.

In another aspect, the present invention provides the use of the compound represented by general formula (I), and their tautomers, enantiomers, diastereomers, racemates, metabolites, metabolic precursors, isotopic compounds, pharmaceutically acceptable salts, esters, prodrugs, hydrates, crystalline hydrates, or solvates, in the preparation of a medicament for treating or preventing diseases, disorders, or conditions associated with abnormal production of TNF-α or regulation of TNF-α activity, abnormal production of IL-2 or regulation of IL-2 activity, or abnormal production of IFN-γ or regulation of IFN-γ activity.

In another aspect, the present invention provides a pharmaceutical composition comprising a therapeutically effective amount of the compound represented by general formula (I), and their tautomers, enantiomers, diastereomers, racemates, metabolites, metabolic precursors, isotopic compounds, pharmaceutically acceptable salts, esters, prodrugs, hydrates, crystalline hydrates, or solvates, together with one or more pharmaceutically acceptable carriers.

In another aspect, the present invention provides a pharmaceutical composition comprising a therapeutically effective amount of the compound represented by general formula (I), and their tautomers, enantiomers, diastereomers, racemates, metabolites, metabolic precursors, isotopic compounds, pharmaceutically acceptable salts, esters, prodrugs, hydrates, crystalline hydrates, or solvates, and one or more additional therapeutically active agents.

The compounds represented by general formula (I), and their tautomers, enantiomers, diastereomers, racemates, metabolites, metabolic precursors, isotopic compounds, pharmaceutically acceptable salts, esters, prodrugs, hydrates, crystalline hydrates, or solvates may exhibit synergistic effects when used in combination with one or more additional therapeutically active agents for the prevention or treatment of particular diseases or disorders.

In addition, the compounds represented by general formula (I), and their tautomers, enantiomers, diastereomers, racemates, metabolites, metabolic precursors, isotopic compounds, pharmaceutically acceptable salts, esters, prodrugs, hydrates, crystalline hydrates, or solvates may reduce or eliminate adverse effects caused by other therapeutically active agents during treatment or prevention of particular diseases or disorders, and vice versa.

In another aspect, the present invention provides a pharmaceutical composition in which the additional therapeutically active agent may include macromolecular compounds such as proteins (e.g., antibodies or polypeptides), polysaccharides, and nucleic acids (DNA or RNA), or small molecules such as inorganic compounds, organometallic compounds, or synthetic or naturally derived organic small molecules.

In a preferred embodiment, the pharmaceutical composition further comprises other therapeutic agents selected from dexamethasone, rituximab, trastuzumab, PD-1 inhibitors, PD-L1 inhibitors, pemetrexed, topotecan, doxorubicin, bortezomib, gemcitabine, dacarbazine, clarithromycin, vincristine, cytarabine, prednisone, docetaxel, clofarabine injection, HDAC inhibitors, androgen receptor inhibitors, androgen biosynthesis inhibitors, BTK inhibitors, erythropoietin, minocycline, elotuzumab, palbociclib, nivolumab, pembrolizumab, panobinostat, ublituximab, romidepsin, eltrombopag, CAR-T, and melphalan.

In another aspect, the present invention provides the use of the compound represented by general formula (I) for preparing a medicament for treating or preventing diseases associated with the CRL4^{CRBN} E3 ubiquitin ligase, including but not limited to cancer, pain, neurological diseases, and immune system diseases. Wherein the diseases, disorders, or conditions include: myelodysplastic syndrome, multiple myeloma, mantle cell lymphoma, non-Hodgkin lymphoma, chronic lymphocytic leukemia, chronic myelomonocytic leukemia, myelofibrosis, Burkitt lymphoma, Hodgkin lymphoma, large cell lymphoma, diffuse large B-cell lymphoma, follicular lymphoma, ciliary body melanoma, choroidal melanoma, iris melanoma, recurrent intraocular melanoma, T-cell lymphoma, erythroid lymphoma, monoblastic and monocytic leukemia, myeloid leukemia, central nervous system lymphoma, brain tumors, meningioma, spinal cord tumors, thyroid cancer, non-small cell lung cancer, ovarian cancer, skin cancer, renal cell carcinoma, astrocytoma, amyloidosis, complex regional pain syndrome type I, malignant melanoma, radiculopathy, glioblastoma, gliosarcoma, malignant glioma, refractory plasmacytoma, extraocular extension melanoma, solid tumors, papillary and follicular thyroid carcinoma, breast cancer, prostate cancer, hepatocellular carcinoma, and primary macroglobulinemia.

In another aspect, the present invention provides a pharmaceutical composition comprising a therapeutically effective amount of the compound represented by general formula (I), or one or more of its stereoisomers, pharmaceutically acceptable salts, prodrugs, solvates, hydrates, or polymorphs, together with at least one excipient, diluent, or carrier. Typical formulations are prepared by mixing the compounds represented by formula (I) with suitable carriers, diluents, or excipients known in the art. Suitable carriers include carbohydrates, waxes, water-soluble or swellable polymers, hydrophilic or hydrophobic substances, gelatin, oils, solvents, and water. The particular carrier, diluent, or excipient selected will depend on the intended mode of administration and purpose. Generally, safe solvents are non-toxic aqueous solvents such as water, as well as water-miscible solvents including ethanol, propylene glycol, and polyethylene glycol (e.g., PEG-400 or PEG-300). The formulation may further comprise buffers, stabilizers, surfactants, wetting agents, lubricants, emulsifiers, suspending agents, preservatives, antioxidants, light-shielding agents, glidants, processing aids, coloring agents, sweeteners, flavoring agents, or other known additives.

When used in combination therapy, the compound represented by formula (I) and one or more additional therapeutic agents may be administered separately or together, preferably in the form of a pharmaceutical composition. Administration may be carried out by any known route, including oral, intravenous, rectal, vaginal, transdermal, or other local or systemic routes.

The pharmaceutical compositions may further contain buffers, stabilizers, surfactants, wetting agents, lubricants, emulsifiers, suspending agents, preservatives, antioxidants, light-protective agents, glidants, processing aids, coloring agents, sweeteners, flavoring agents, or other additives to provide an acceptable dosage form.

The pharmaceutical compositions are preferably administered orally. Solid dosage forms suitable for oral administration include capsules, tablets, powders, or granules. In such forms, the compounds of the invention may be mixed with inert excipients, diluents, or carriers such as sodium citrate, dicalcium phosphate, starch, lactose, sucrose, mannitol, silica, binders (e.g., carboxymethyl cellulose, alginates, gelatin, polyvinylpyrrolidone, sucrose, gum arabic), wetting agents such as glycerol, disintegrants such as agar, calcium carbonate, potato or tapioca starch, alginic acid, complex silicates, sodium carbonate, and lubricants such as talc, calcium stearate, magnesium stearate, solid polyethylene glycol, or sodium lauryl sulfate, and the like. In the case of capsules and tablets, the dosage forms may also comprise buffering agents. Solid compositions of a similar type may also be employed as fillers in soft and hard-filled gelatin capsules, using excipients such as lactose and high-molecular-weight polyethylene glycols.

The above liquid dosage forms for oral administration include pharmaceutically acceptable emulsions, solutions, suspensions, syrups, and elixirs. In addition to the compound of the present invention or a composition thereof, the liquid dosage forms may contain inert diluents commonly used in the art, such as water or other solvents; solubilizers and emulsifiers such as ethanol, isopropyl alcohol, ethyl carbonate, ethyl acetate, benzyl alcohol, benzyl benzoate, propylene glycol, 1,3-butanediol, dimethylformamide; oils (such as cottonseed oil, peanut oil, corn germ oil, olive oil, castor oil, sesame oil, and the like); glycerol; tetrahydrofurfuryl alcohol; fatty acid esters of polyethylene glycol and sorbitan; or mixtures of two or more thereof.

In addition to these inert diluents, the compositions may also include one or more excipients such as wetting agents, emulsifying agents, suspending agents, sweetening agents, flavoring agents, and perfuming agents. For suspensions, in addition to the compound or composition of the present invention, there may be further included carriers such as suspending agents, e.g., ethoxylated isostearyl alcohol, polyoxyethylene sorbitol, sorbitan esters, microcrystalline cellulose, aluminum metahydroxide, bentonite, agar-agar, and tragacanth, or mixtures thereof.

Compositions for rectal or vaginal administration are preferably suppositories, which may be prepared by mixing the compound or composition of the present invention with a suitable non-irritating excipient or carrier, such as cocoa butter, polyethylene glycol, or suppository wax, which are solid at ordinary room temperature but liquid at body temperature and thus melt in the rectum or vagina to release the active compound.

The compounds or pharmaceutical compositions of the present invention may be administered via other topical dosage forms, including ointments, powders, sprays, and inhalants. The medicament may be mixed under sterile conditions with a pharmaceutically acceptable excipient, diluent, or carrier, and any preservatives, buffers, or propellants as required. Ophthalmic formulations, ophthalmic ointments, powders, and solutions are also intended to be within the scope of the present invention.

The present invention further provides the use of a compound represented by Formula (I), as well as tautomers, enantiomers, diastereomers, racemates, metabolic precursors, metabolites, isotopic compounds, pharmaceutically acceptable salts, esters, prodrugs, or hydrates, solvates, or crystal forms thereof, as selective modulators of CRL4^{CRBN} E3 ubiquitin ligase, for regulating the activity of CUL4^{CRBN} E3 ubiquitin ligase.

The present invention also provides the use of a compound represented by Formula (I), as well as tautomers, enantiomers, diastereomers, racemates, metabolic precursors, metabolites, isotopic compounds, pharmaceutically acceptable salts, esters, prodrugs, or hydrates, solvates, or crystal forms thereof, in the manufacture of a medicament for the treatment or prevention of diseases associated with the involvement of CRL4^{CRBN} E3 ubiquitin ligase. Diseases associated with the involvement of CRL4^{CRBN} E3 ubiquitin ligase include, but are not limited to, tumors, central nervous system diseases, and immune diseases.

In a preferred embodiment, the present invention relates to a method for treating or preventing a disease, disorder, or condition associated with abnormal TNFα production or regulation of TNFα activity, IL-2 production, or regulation of IL-2 activity, said method comprising administering to a subject a therapeutically or prophylactically effective amount of one or more of the isoindoline derivatives represented by Formula (I), or pharmaceutically acceptable salts, solvates, stereoisomers, isotopic compounds, metabolites, and prodrugs thereof. According to the methods of the present invention, examples of such diseases, disorders, and conditions to be treated or prevented include, but are not limited to: cancer, including solid tumors; TNFα-related disorders; diseases and conditions associated with undesired angiogenesis; pain; macular degeneration (MD)-related syndromes; skin diseases; keratoses; respiratory diseases (e.g., pulmonary diseases); immunodeficiency diseases; central nervous system (CNS) diseases; autoimmune diseases; atherosclerosis; genetic, allergic, viral, sleep disorders and related syndromes; inflammatory diseases; PDE4-related diseases; or IL-2-related diseases. Examples of such diseases, disorders, or conditions well known in the art include, but are not limited to, those described in PCT Patent Publications WO2012015986 and WO2006018182, and U.S. Patent Publication US20100204227, some of which are incorporated herein by reference in their entirety.

The compound represented by Formula (I) of the present invention, as well as stereoisomers, pharmaceutically acceptable salts, prodrugs, solvates, hydrates, or crystal forms thereof, may be used in monotherapy or combination therapy. When used in combination therapy, the composition comprises a therapeutically effective dose of a compound of Formula (I), enantiomers, diastereomers, racemates, and mixtures thereof, as well as pharmaceutically acceptable salts, crystalline hydrates, and solvates thereof, and one or more additional pharmaceutically therapeutically active ingredients. Said one or more additional pharmaceutically therapeutically active ingredients include macromolecular compounds such as proteins (antibodies or polypeptides), polysaccharides, and nucleic acids (DNA or RNA); and small molecule compounds such as inorganic compounds, organometallic compounds, synthetic or naturally occurring small organic molecules; and further include radiation therapy, surgery, cell therapy, hormone therapy, or cytokine therapy. The compound of Formula (I) of the present invention, prodrugs, enantiomers, diastereomers, racemates, and mixtures thereof, as well as pharmaceutically acceptable salts, crystalline hydrates, and solvates thereof, may exert a synergistic effect with one or more additional pharmaceutically therapeutically active ingredients in the prevention or treatment of a particular disease or disorder. The compound of Formula (I) of the present invention, prodrugs, enantiomers, diastereomers, racemates, and mixtures thereof, as well as pharmaceutically acceptable salts, crystalline hydrates, and solvates thereof, may also reduce or eliminate toxic or adverse effects produced by one or more additional pharmaceutically therapeutically active ingredients in the prevention or treatment of a particular disease or disorder, and vice versa.

In another preferred embodiment, the diseases or disorders include, but are not limited to: cancer, angiogenesis-related diseases or disorders, pain (including but not limited to complex regional pain syndrome), macular degeneration and related disorders, skin diseases, pulmonary disorders, immunodeficiency diseases, central nervous system injuries and disorders, and TNFα-related diseases or disorders.

In another preferred embodiment, the cancer includes, but is not limited to: skin cancer (e.g., melanoma), lymphatic system cancer, breast cancer, cervical cancer, uterine cancer, gastrointestinal cancer, lung cancer, ovarian cancer, prostate cancer, colon cancer, rectal cancer, oral cancer, brain tumor, head and neck cancer, throat cancer, testicular cancer, kidney cancer, pancreatic cancer, spleen cancer, liver cancer, bladder cancer, laryngeal cancer, and AIDS-related cancer. The compounds provided by the present invention are also effective against hematological neoplasms and myelomas, such as multiple myeloma and acute and chronic leukemia. The compounds provided by the present invention can be used for the prevention or treatment of primary tumors and metastatic tumors.

The compounds represented by general formula (I) may contain one or more asymmetric centers and therefore may exist in different stereoisomeric forms. All such stereoisomers-including diastereomers, enantiomers, atropisomers, and mixtures thereof (such as racemic mixtures)-as well as tautomers, metabolites, metabolic precursors, isotopic derivatives, pharmaceutically acceptable salts, esters, prodrugs, hydrates, solvates, or crystalline forms are within the scope of the present invention.

It should be understood that the technical features described above may be combined in any suitable manner within the scope of the present invention to form new or preferred embodiments. The foregoing description is not intended to limit the scope of the invention in any way.

### Detailed Description of the Invention

The inventors of the present invention, through extensive and in-depth research, and after substantial screening and testing, have provided a class of structurally novel CRL4^{CRBN} E3 ubiquitin ligase modulator compounds exhibiting excellent antitumor activity. By analyzing the crystal structures of complexes formed between CRL4^{CRBN} E3 ubiquitin ligase and small molecules (PDB IDs: 4CI2 and 5HXB), the inventors obtained the following important information: the CRL4^{CRBN} E3 ubiquitin ligase contains multiple binding pockets for small molecules. Therefore, structurally complex small molecules capable of interacting with multiple binding sites can be developed to achieve effective binding between CRL4^{CRBN} E3 ubiquitin ligase and small molecules. In addition, molecular dynamics simulations were employed to analyze the structural dynamics and binding sites at the interface between model molecules and the E3 ubiquitin ligase. Combined with molecular docking and complex-based pharmacophore matching strategies, scoring functions were used to evaluate the binding modes and interactions of compounds at the active site of the E3 ubiquitin ligase. Through computational simulation and structural optimization during the design process, novel and specific small-molecule modulators of the CRL4^{CRBN} E3 ubiquitin ligase were obtained.

Based on this information, the inventors designed and synthesized a series of novel CRL4^{CRBN} E3 ubiquitin ligase small-molecule modulators with an indazolone scaffold as described in the present application, and evaluated their biological activities. Representative compounds were tested in multiple myeloma cell line (MM.1S), mantle cell lymphoma cell line (Mino), and acute myeloid leukemia cell line (MV-4-11). The results showed that the novel small-molecule modulators exhibit very strong inhibitory activity against cell proliferation. In contrast, previously known CRBN modulators based on isoindolinone structures, including marketed or clinical-stage immunomodulatory drugs (IMiDs), do not show significant cell growth inhibitory activity in mantle cell lymphoma cell line (Mino) or acute myeloid leukemia cell line (MV-4-11). Therefore, the indazolone compounds developed in the present application act in biological systems by regulating the ubiquitin-proteasome-mediated protein degradation pathway, thereby controlling degradation of substrate proteins or inducing degradation of pathogenic proteins. As a result, effective disease treatment based on the CRBN target can be achieved. On this basis, the present invention has been completed.

### Terms

When a substituent is described by a conventional chemical formula written from left to right, the substituent also includes the chemically equivalent substituent obtained when the structural formula is written from right to left. For example, -CH₂O- is equivalent to -OCH₂-.

Those skilled in the art will understand that the combinations of substituents contemplated by the present invention are those that are stable or chemically feasible. In the present invention, substitutions on the relevant structures, including substituted and unsubstituted groups, such as groups "optionally" or "optionally substituted" with a certain substituent, mean that the group may either be substituted with the substituent or remain unsubstituted.

In the present invention, when the number of substituents is greater than one, the substituents R may be the same or different. This means that when multiple substituents are present in a given structure, the substituent combinations for R may be selected from different types of substituents.

The term "substituted" applies only to positions capable of being substituted and does not include substitutions that cannot be achieved according to known chemical principles. The term "substituted" means that one or more hydrogen atoms on a specific group are replaced by a specified substituent. The specific substituent may be one described hereinabove or one appearing in the embodiments. Unless otherwise specified, any optionally substituted group may have one substituent selected from a specified group at any substitutable position of the group, and the substituents at different positions may be the same or different. Cyclic substituents, such as heterocycloalkyl groups, may be connected to another ring, such as a cycloalkyl group, to form a spirocyclic system, for example two rings sharing a single carbon atom. Unless otherwise specified, substitution may refer to substitution with one or more groups selected from hydrogen, halogen, cyano, nitro, hydroxyl, carboxyl, a 4-10 membered heterocyclic group, C₁-C₆ alkyl, C₁-C₆ alkoxy, halogen-substituted C₁-C₆ alkyl, halogen-substituted C₁-C₆ alkoxy, C₂-C₆ alkenyl, C₃-C₈ heterocycloalkoxy, C₃-C₈ cycloalkoxy, hydroxy-substituted C₁-C₆ alkoxy, and C₁-C₆ alkoxy-substituted C₁-C₆ alkoxy.

The term "halogen" refers to fluorine, chlorine, bromine, or iodine.

The term "plurality" refers to two or more, such as 2, 3, 4, 5, or 6.

The term "C₁-C₆ alkyl" refers to a straight-chain or branched saturated hydrocarbon group having 1 to 6 carbon atoms in the chain, such as alkyl groups containing 1, 2, 3, 4, 5, or 6 carbon atoms. As used herein, an alkyl group or an alkyl moiety as part of another group has 1 to 6 carbon atoms (i.e., C₁-C₆ alkyl) or 1 to 3 carbon atoms (i.e., C₁-C₃ alkyl), including but not limited to methyl, ethyl, propyl, isopropyl, butyl, isobutyl, sec-butyl, and tert-butyl.

The term "C₁-C₆ alkoxy" refers to a straight-chain or branched alkoxy group having 1 to 6 carbon atoms, including but not limited to methoxy, ethoxy, propoxy, isopropoxy, and butoxy.

The term "aryl" refers to a 6-14 membered carbocyclic monocyclic or fused polycyclic group having a conjugated π-electron system, preferably a 6-10 membered ring (i.e., C₆-C₁₀ aryl), more preferably phenyl and naphthyl, and most preferably phenyl. The aryl ring may be fused with a heteroaryl ring, heterocyclic ring, or cycloalkyl ring, wherein the ring connected to the parent structure is the aryl ring.

The term "halogen-substituted C₁-C₆ alkoxy" refers to a straight-chain or branched C₁-C₆ alkoxy group substituted with one or more halogens, including but not limited to -OCH₂F,-OCHF₂, and -OCF₃.

The term "halogen-substituted C₁-C₆ alkyl" refers to a straight-chain or branched C₁-C₆ alkyl group substituted with one or more halogens, including but not limited to 2-bromoethyl and 2-bromopropyl.

The term "C₃-C₁₀ cycloalkyl" refers to a saturated or partially unsaturated monocyclic or polycyclic cyclic hydrocarbon substituent. C₃-C₁₀ cycloalkyl preferably refers to C₄-C₇ cycloalkyl or C₅-C₆ cycloalkyl. Monocyclic cycloalkyl groups include, but are not limited to, cyclopropyl, cyclobutyl, cyclopentenyl, and cyclohexyl. Polycyclic cycloalkyl groups include spirocyclic, fused, and bridged cycloalkyl structures.

The term "4-10 membered heterocyclyl" refers to a saturated and/or partially saturated ring containing 4 to 10 ring atoms, wherein one or more (e.g., 2 or 3) ring atoms are heteroatoms selected from nitrogen, oxygen, sulfur, or S(O)ₘ (where m is an integer from 0 to 2), and the remaining ring atoms are carbon atoms. Preferably, the heterocycle is a 4-6 membered heterocycle, such as oxiranyl, tetrahydrofuranyl, pyrrolidinyl, tetrahydropyranyl, piperidinyl, piperazinyl, morpholinyl, or thiomorpholinyl.

The term "C₂-C₆ alkenyl" refers to a straight-chain or branched hydrocarbon group consisting only of carbon and hydrogen atoms, containing at least one double bond, having 2 to 6 carbon atoms, and connected to the rest of the molecule by one or more single bonds, such as but not limited to vinyl, allyl, butenyl, styryl, and cinnamyl.

The term "heteroaryl" refers to a 5-14 membered aromatic ring containing 1-4 heteroatoms (e.g., 2 or 3 heteroatoms) as ring atoms, with the remaining ring atoms being carbon atoms, wherein the heteroatoms are selected from oxygen, sulfur, and nitrogen. Preferably the heteroaryl ring is 5-10 membered, more preferably 5- or 6-membered, such as thienyl, furyl, pyridyl, pyrrolyl, N-alkylpyrrolyl, pyrimidinyl, pyrazinyl, imidazolyl, and tetrazolyl.

The term "hydrate" refers to a compound containing water.

The term "pharmaceutically acceptable salt" refers to salts formed between a drug molecule and a corresponding organic acid, inorganic acid, organic base, or inorganic base. Inorganic acids from which salts may be derived include hydrochloric acid, hydrobromic acid, sulfuric acid, nitric acid, and phosphoric acid. Organic acids from which salts may be derived include formic acid, acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, trifluoroacetic acid, and succinic acid salts.

By way of example, salts derived from inorganic bases include sodium salts, potassium salts, lithium salts, ammonium salts, calcium salts, and magnesium salts. Salts derived from organic bases include, but are not limited to, salts of primary, secondary, and tertiary amines, such as alkylamines (i.e., NH₂(alkyl)), dialkylamines (i.e., HN(alkyl)₂), trialkylamines (i.e., N(alkyl)₃), substituted alkylamines, di(substituted alkyl)amines, and tri(substituted alkyl)amines. Suitable examples of amines include isopropylamine, trimethylamine, diethylamine, triisopropylamine, tripropylamine, ethanolamine, 2-dimethylaminoethanol, piperazine, piperidine, morpholine, and N-ethylpiperidine.

Unless otherwise specified, the terms used herein have their generally accepted meanings as understood by those skilled in the art. In the present invention, the definitions of identical groups appearing in different general formulae or in the same general formula are independent of one another.

### Active Ingredient

As used herein, the term "compound of the present invention" or "active ingredient" refers to the compound represented by formula (I), and also includes pharmaceutically acceptable salts, tautomers, stereoisomers, and prodrugs thereof.

The compounds represented by formula (I) may also exist in different tautomeric forms, and all such forms are included within the scope of the present invention.

The term "tautomer" refers to structural isomers having different energies that interconvert via a low-energy barrier, typically involving migration of a hydrogen atom or proton accompanied by rearrangement of a single bond and an adjacent double bond.

The compounds of the present invention may exist in particular geometric or stereoisomeric forms. All such compounds contemplated by the present invention-including cis and trans isomers, (-) and (+) enantiomers, (R) and (S) enantiomers, diastereomers, racemates, and other mixtures-are within the scope of the present invention. Additional asymmetric carbon atoms may also be present in substituents such as alkyl groups. All such isomers and mixtures thereof are included within the scope of the present invention.

The compounds represented by formula (I) may contain one or more asymmetric or chiral centers and therefore may exist in different stereoisomeric forms. The compounds of the present invention include all stereoisomeric forms, including but not limited to diastereomers, enantiomers, atropisomers, and mixtures thereof (such as racemates), all of which fall within the scope of the present invention.

Unless otherwise specified, the term "enantiomer" refers to stereoisomers that are mirror images of each other.

Unless otherwise specified, the term "diastereomer" refers to stereoisomers that contain two or more chiral centers and are not mirror images of each other.

The term "racemate" refers to a mixture of two mirror-image stereoisomers with opposite optical rotations that cancel each other.

The general formulae and example compounds of the present invention may contain chiral centers; therefore, both single stereochemical configurations and racemic mixtures fall within the scope of the present invention. In addition, existing studies have shown that compounds containing thalidomide-like structures may interconvert between R-configuration, S-configuration, and racemic forms in biological systems, ultimately forming a 1:1 racemic mixture in vivo. Accordingly, those skilled in the art may reasonably expect that the enantiomers or racemates of the compounds of the present invention will achieve comparable technical effects. If a single stereochemical configuration of an example compound is desired, it may be obtained using a single-configuration intermediate 1B via Synthetic Method 1 described in the preparation methods. Thus, enantiomerically pure compounds may be obtained. Similarly, diastereomeric compounds may also be obtained using single-configuration intermediate 1B. All such different isomeric forms are considered stereoisomers and fall within the scope of the present invention.

The compounds of the present invention may contain atoms having non-natural isotopic abundances. For example, compounds may be labeled with radioactive isotopes such as deuterium (D), tritium (T), ¹²C, ¹³C, or ¹⁴C. In another example, hydrogen atoms may be replaced with deuterium to form deuterated drugs. Because the carbon-deuterium bond is stronger than the carbon-hydrogen bond, deuterated drugs may exhibit advantages such as reduced toxicity and side effects, improved stability, enhanced therapeutic efficacy, and extended biological half-life compared with non-deuterated drugs. All isotopic variations of the compounds of the present invention, whether radioactive or not, are included within the scope of the present invention.

As used herein, unless otherwise specified, the term "prodrug" refers to a derivative of a compound that contains a bioreactive functional group such that, under biological conditions (in vitro or in vivo), the functional group may be cleaved or otherwise transformed to release the active compound. Typically, prodrugs are inactive or less active than the parent compound until the compound is released from the bioreactive functional group. The bioreactive functional group may be hydrolyzed or oxidized under biological conditions to generate the active compound. For example, a prodrug may contain a biologically hydrolyzable group. Examples of such groups include, but are not limited to, biologically hydrolyzable phosphates, esters, amides, carbonates, carbamates, and acylureas. When the compounds of the present invention contain basic groups, they may be converted into pharmaceutically acceptable salts, including inorganic acid salts and organic acid salts. Suitable acids for salt formation include, but are not limited to: inorganic acids: hydrochloric acid, hydrobromic acid, hydrofluoric acid, sulfuric acid, nitric acid, and phosphoric acid; organic acids: formic acid, acetic acid, propionic acid, oxalic acid, malonic acid, succinic acid, fumaric acid, maleic acid, lactic acid, malic acid, tartaric acid, citric acid, picric acid, methanesulfonic acid, benzenesulfonic acid, and benzoic acid; acidic amino acids, such as aspartic acid and glutamic acid.

The present invention also encompasses any novel intermediates disclosed herein.

### Pharmaceutical Compositions and Methods of Administration

As used herein, the term "pharmaceutical composition" refers to a formulation comprising a compound of the present invention together with a medium commonly accepted in the art for delivering biologically active compounds to mammals (e.g., humans). The medium includes pharmaceutically acceptable carriers. The purpose of the pharmaceutical composition is to facilitate administration to the organism and promote absorption of the active ingredient so that its biological activity can be exerted.

The compound represented by formula (I) may be administered in combination with other drugs known to treat or improve similar conditions. In combination therapy, the administration mode and dosage of the original drug may remain unchanged, while the compound of formula (I) may be administered simultaneously or subsequently. When the compound of formula (I) is administered simultaneously with one or more other drugs, a pharmaceutical composition containing both the compound of formula (I) and one or more known drugs may preferably be used. Combination therapy also includes administering the compound of formula (I) together with one or more known drugs during overlapping treatment periods. When the compound of formula (I) is used in combination with other drugs, the dosage of the compound of formula (I) and/or the known drug may be lower than the dosage used in monotherapy.

As used herein, the term "pharmaceutically acceptable" refers to substances (such as carriers or diluents) that do not adversely affect the biological activity or properties of the compounds of the present invention and are relatively non-toxic, meaning that they may be administered to an individual without causing adverse biological reactions or undesirable interactions with other components of the composition.

As used herein, "pharmaceutically acceptable carrier" includes, but is not limited to, any adjuvant, carrier, excipient, glidant, sweetener, diluent, preservative, dye/coloring agent, flavoring agent, surfactant, wetting agent, dispersant, suspending agent, stabilizer, isotonic agent, solvent, or emulsifier approved by the relevant regulatory authorities for use in humans or animals.

Drugs or active ingredients that may be used in combination with the compound represented by formula (I) include, but are not limited to: dexamethasone, rituximab, trastuzumab, PD-1 inhibitors, PD-L1 inhibitors, pemetrexed, topotecan, doxorubicin, bortezomib, gemcitabine, dacarbazine, clarithromycin, vincristine, cytarabine, prednisone, docetaxel, clofarabine injection, HDAC inhibitors, kinase inhibitors, androgen receptor inhibitors, androgen biosynthesis inhibitors, erythropoietin, minocycline, elotuzumab, palbociclib, nivolumab, pembrolizumab, panobinostat, ublituximab, romidepsin, eltrombopag, CAR-T therapy, and melphalan.

Typical formulations are prepared by mixing the compounds represented by formula (I) with suitable carriers, diluents, or excipients. Suitable carriers and excipients are well known to those skilled in the art and include carbohydrates, waxes, water-soluble and/or swellable polymers, hydrophilic or hydrophobic materials, gelatin, oils, solvents, and water. The particular carrier, diluent, or excipient selected depends on the intended mode of administration and therapeutic purpose. Generally, solvents suitable for pharmaceutical use are selected based on safety and effectiveness for administration to mammals. Safe solvents include non-toxic aqueous solvents such as pharmaceutical water and other water-miscible solvents. Suitable aqueous solvents include one or more of water, ethanol, propylene glycol, and polyethylene glycol (e.g., PEG-400 or PEG-300). The formulation may further comprise buffers, stabilizers, surfactants, wetting agents, lubricants, emulsifiers, suspending agents, preservatives, antioxidants, light-protective agents, glidants, processing aids, coloring agents, sweeteners, flavoring agents, or other known additives.

When used in combination therapy, the compound represented by formula (I) and at least one additional drug may be administered separately or together, preferably in the form of a pharmaceutical composition. The compounds or compositions of the present invention may be administered by any known route, including oral administration, intravenous injection, rectal administration, vaginal administration, transdermal absorption, or other local or systemic routes.

These pharmaceutical compositions may further contain one or more buffers, stabilizers, surfactants, wetting agents, lubricants, emulsifiers, suspending agents, preservatives, antioxidants, light-protective agents, glidants, processing aids, coloring agents, sweeteners, flavoring agents, or other additives so that the pharmaceutical composition can be manufactured or used in an acceptable form.

Oral administration is preferred in the present invention. Solid dosage forms for oral administration include capsules, tablets, powders, or granules. In such solid dosage forms, the compound or pharmaceutical composition of the present invention may be mixed with inert excipients, diluents, or carriers. Suitable excipients include substances such as sodium citrate or dicalcium phosphate; starch, lactose, sucrose, mannitol, or silica; binders such as carboxymethyl cellulose, alginates, gelatin, polyvinylpyrrolidone, sucrose, or gum arabic; wetting agents such as glycerol; disintegrants such as agar, calcium carbonate, potato or tapioca starch, alginic acid, complex silicates, or sodium carbonate; absorption enhancers such as quaternary ammonium compounds; adsorbents such as kaolin or bentonite; and lubricants such as talc, calcium stearate, magnesium stearate, solid polyethylene glycol, or sodium lauryl sulfate, and the like. In the case of capsules and tablets, the dosage forms may also comprise buffering agents. Solid compositions of a similar type may also be employed as fillers in soft and hard-filled gelatin capsules, using excipients such as lactose and high-molecular-weight polyethylene glycols.

Liquid dosage forms suitable for oral administration include emulsions, solutions, suspensions, syrups, and elixirs. In addition to the compound or composition of the present invention, such dosage forms may contain inert diluents commonly used in the art, such as water or other solvents, solubilizers, and emulsifiers including ethanol, isopropanol, ethyl carbonate, ethyl acetate, benzyl alcohol, benzyl benzoate, propylene glycol, 1,3-butanediol, dimethylformamide, vegetable oils (e.g., cottonseed oil, peanut oil, corn oil, olive oil, castor oil, sesame oil), glycerol, tetrahydrofurfuryl alcohol, polyethylene glycol, sorbitan fatty acid esters, or mixtures thereof.

In addition to these inert diluents, the compositions may also include one or more excipients such as wetting agents, emulsifying agents, suspending agents, sweetening agents, flavoring agents, and perfuming agents.

For suspensions, in addition to the compound or composition of the present invention, there may be further included carriers such as suspending agents, e.g., ethoxylated isostearyl alcohol, polyoxyethylene sorbitol, sorbitan esters, microcrystalline cellulose, aluminum metahydroxide, bentonite, agar-agar, and tragacanth, or mixtures thereof.

For rectal or vaginal administration, the compositions are preferably prepared as suppositories by mixing the compound with suitable non-irritating excipients or carriers such as cocoa butter, polyethylene glycol, or suppository wax, which remain solid at room temperature but melt at body temperature.

The compounds or pharmaceutical compositions of the present invention may also be administered in other local dosage forms including ointments, powders, sprays, and inhalants. These preparations may be formulated under sterile conditions with pharmaceutically acceptable excipients, diluents, carriers, preservatives, buffers, or propellants. Ophthalmic formulations, eye ointments, powders, and solutions are also included within the scope of the present invention.

As used herein, the terms "administration," "delivery," or "dosing" refer to methods of delivering the compound or composition to a site where it exerts biological activity. These methods include, but are not limited to, oral administration, duodenal administration, parenteral injection (including intravenous, subcutaneous, intraperitoneal, intramuscular, or intra-arterial injection or infusion), topical administration, and rectal administration. Techniques for administration are well known in the art, for example as described in Goodman and Gilman, The Pharmacological Basis of Therapeutics, current ed.; Pergamon; and Remington's, Pharmaceutical Sciences (current edition), Mack Publishing Co., Easton, Pa. In preferred embodiments, the compounds and compositions described herein are administered orally.

The terms "drug combination," "combination therapy," "combined administration," or "administration of other therapeutic agents" refer to treatments obtained by mixing or combining more than one active ingredient. This includes both fixed combinations and non-fixed combinations. A fixed combination refers to simultaneous administration of at least one compound described herein and at least one synergistic drug in a single dosage form. A non-fixed combination refers to administration of at least one compound described herein together with at least one synergistic agent as separate entities, either simultaneously or sequentially with variable time intervals. Such approaches also include cocktail therapies, where three or more active components are administered.

The pharmaceutical compositions of the present invention contain the compound of the invention or a pharmaceutically acceptable salt thereof within a safe and effective dosage range, together with pharmaceutically acceptable excipients or carriers. The term "safe and effective amount" refers to an amount sufficient to significantly improve the disease condition without causing severe side effects. Typically, the pharmaceutical composition contains 1-2000 mg of the compound per dose, preferably 10-1000 mg per dose. Preferably, one "dose" corresponds to one capsule or tablet.

The compounds or pharmaceutical compositions of the present invention may be used to prepare medicaments for treating or preventing diseases involving the CRL4^{CRBN} E3 ubiquitin ligase. Such diseases include, but are not limited to, tumors, central nervous system diseases, and immune diseases.

In a preferred embodiment, the diseases include, but are not limited to: cancer, pain (including complex regional pain syndrome), skin diseases, immunodeficiency diseases, and injuries or dysfunctions of the central nervous system.

In another preferred embodiment, the cancers include, but are not limited to: skin cancers (such as melanoma), lymphatic cancers, breast cancer, cervical cancer, uterine cancer, gastrointestinal cancers, lung cancer, ovarian cancer, prostate cancer, colon cancer, rectal cancer, oral cancer, brain tumors, head and neck cancers, pharyngeal cancer, testicular cancer, kidney cancer, pancreatic cancer, splenic cancer, liver cancer, bladder cancer, laryngeal cancer, and AIDS-related cancers. The compounds of the present invention are also effective against hematological malignancies, including multiple myeloma, lymphoma, and acute or chronic leukemia. The compounds may also be used to prevent or treat primary tumors and metastatic tumors.

Diseases treatable by the compounds described herein include, but are not limited to: myelodysplastic syndrome, multiple myeloma, mantle cell lymphoma, non-Hodgkin lymphoma, chronic lymphocytic leukemia, chronic myelomonocytic leukemia, myelofibrosis, Burkitt lymphoma, Hodgkin lymphoma, diffuse large B-cell lymphoma, follicular lymphoma, ciliary body melanoma, iris melanoma, recurrent intraocular melanoma, T-cell lymphoma, erythroid lymphoma, monoblastic and monocytic leukemia, myeloid leukemia, central nervous system lymphoma, meningioma, spinal cord tumors, non-small cell lung cancer, ovarian cancer, skin cancer, renal cell carcinoma, astrocytoma, amyloidosis, complex regional pain syndrome type I, malignant melanoma, radiculopathy, glioblastoma, gliosarcoma, malignant glioma, refractory plasmacytoma, extraocular extension melanoma, papillary and follicular thyroid carcinoma, breast cancer, prostate cancer, hepatocellular carcinoma, and primary macroglobulinemia.

### Main advantages of the present invention include:

1. The present invention provides a class of molecular glue degraders targeting CRL4^{CRBN} E3 ubiquitin ligase with a novel indazolone scaffold, which improves antitumor efficacy, expands the therapeutic indications of molecular glue degraders, and provides ligands with improved physicochemical properties for designing degraders targeting disease-related proteins.
2. Existing CRBN ligands based on immunomodulatory imide drugs (IMiDs) are structurally unstable and prone to hydrolysis in body fluids. The present invention optimizes the structure through scaffold hopping, thereby developing molecular glue degraders with the indazolone scaffold represented by formula (I), which exhibit improved chemical stability.

The indazolone molecular glue scaffold represented by formula (I) can be rapidly and efficiently constructed via photo-induced cyclization of primary amines with o-nitrobenzyl alcohol (PANAC reaction).

The invention is further illustrated below with specific examples. These examples are provided for illustration only and are not intended to limit the scope of the invention. Unless otherwise specified, experimental procedures in the following examples were carried out under conventional conditions or according to the manufacturer's recommendations. Unless otherwise stated, percentages and parts are calculated by weight.

In all examples, ¹H NMR spectra were recorded on 400 MHz or 500 MHz Bruker NMR spectrometers, and chemical shifts are expressed as δ (ppm). Mass spectra were measured using UPLC-MS (ESI), where the UPLC instrument was Waters HPLC H-CLASS, and the MS detector was Waters SQ Detector 2. Anhydrous tetrahydrofuran was prepared by refluxing with benzophenone/sodium under oxygen-free conditions. Anhydrous toluene and dichloromethane were prepared by refluxing with calcium chloride. Petroleum ether, ethyl acetate, dichloromethane, and other solvents used as eluents for column chromatography were purchased from Sinopharm Chemical Reagent Co., Ltd. Thin-layer chromatography plates (HSGF254) used for reaction monitoring were also obtained from Sinopharm. Silica gel (200-300 mesh) for compound purification was likewise purchased from Sinopharm. The starting materials used in the present invention may be obtained commercially, for example from Sinopharm Chemical Reagent Co., Ltd., prepared by methods known in the art, or synthesized according to the methods described in the present invention.

### Example 1: (S)-3-(7-((4-(Morpholinomethyl)benzyl)oxy)-3-oxo-1,3-dihydro-2H-indazol-2-yl)piperidine-2,6-dione (D1)

### Synthetic Route:

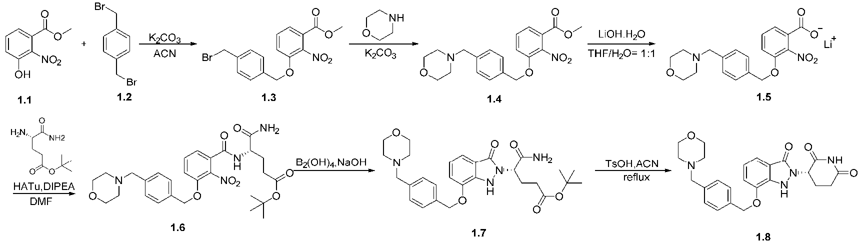

### Step 1:

A suspension of compound 1.1 (500 mg, 2.54 mmol), compound 1.2 (2.0 g, 7.60 mmol), and potassium carbonate (351 mg, 2.54 mmol) in acetonitrile was heated to 50 °C and stirred overnight. After the reaction was complete as monitored by TLC, the mixture was cooled to room temperature, filtered, and concentrated under reduced pressure. The residue was purified by column chromatography to afford a white solid (910 mg, 94% yield). ¹H NMR (500 MHz, CDCl3) δ 7.61 (dd, J = 7.9, 1.1 Hz, 1H), 7.47 - 7.39 (m, 3H), 7.34 (d, J = 8.2 Hz, 2H), 7.24 (dd, J = 8.4, 1.0 Hz, 1H), 5.20 (s, 2H), 4.48 (s, 2H), 3.90 (s, 3H).

### Step 2:

A suspension of compound 1.3 (350 mg, 0.9 mmol), morpholine (86 µL, 1.01 mmol), and potassium carbonate (191 mg, 1.38 mmol) in acetonitrile was stirred at room temperature overnight. After completion of the reaction (TLC monitoring), the mixture was filtered and concentrated under reduced pressure. Purification by column chromatography afforded a white solid (351 mg, 99% yield).

### Step 3:

Compound 1.4 (393 mg, 1.0 mmol) was dissolved in 10 mL anhydrous tetrahydrofuran, and 5 mL aqueous lithium hydroxide solution (63 mg, 1.5 mmol) was added. The mixture was stirred at room temperature for 2 h. After completion of the reaction (TLC monitoring), the mixture was concentrated under reduced pressure and dried under vacuum. The residue was used directly in the next step without further purification.

### Step 4:

Compound 1.5 (201 mg, 0.52 mmol), (S)-tert-butyl 4,5-diamino-5-oxopentanoate (124 mg, 0.52 mmol), and HATU (198 mg, 0.52 mmol) were dissolved in 10 mL anhydrous DMF. DIPEA (258 µL, 1.56 mmol) was added at room temperature, and the reaction mixture was stirred overnight. After completion (TLC monitoring), the reaction was quenched with water and extracted with ethyl acetate. The organic layer was washed several times with saturated sodium chloride solution, dried, filtered, and concentrated. Column chromatography afforded a gray solid (210 mg, 73% yield). UPLC-MS (ESI): Calculated for C₂₈H₃₆N₄O₈ [M+H]⁺: 557.62, Found: 557.62.

### Step 5:

Compound 1.6 (202 mg, 0.36 mmol) was dissolved in methanol (5 mL). At 0 °C, tetrahydroxydiboron (161 mg, 1.8 mmol) was added, followed by a methanolic solution of sodium hydroxide (144 mg, 3.6 mmol in 5 mL methanol). After stirring for 10 min at the same temperature, the reaction was warmed to 40 °C and stirred overnight. After completion (TLC monitoring), the reaction was quenched with saturated ammonium chloride solution and extracted with ethyl acetate. The organic layer was washed with water and saturated brine, dried over anhydrous sodium sulfate, filtered, and concentrated. Column chromatography afforded the product (160 mg, 85% yield).UPLC-MS (ESI): Calculated for C₂₈H₃₆N₄O₆ [M+H]⁺: 525.62, Found: 525.53. ¹H NMR (500 MHz, DMSO) δ 9.75 (s, 1H), 7.48 (t, *J =* 9.4 Hz, 3H), 7.34 (d, *J =* 7.9 Hz, 2H), 7.30 (s, 1H), 7.23 (d, *J =* 7.8 Hz, 1H), 7.18 (d, *J =* 7.9 Hz, 1H), 7.05 (t, *J =* 7.8 Hz, 1H), 5.22 (s, 2H), 4.87 (dd, *J =* 9.7, 6.0 Hz, 1H), 3.63 - 3.51 (m, 4H), 3.47 (s, 2H), 2.94 (d, *J =* 29.3 Hz, 2H), 2.34 (s, 4H), 2.20 (d, *J =* 14.5 Hz, 2H), 2.12 - 1.97 (m, 2H), 1.35 (s, 9H).

### Step 6:

Compound 1.7 (75 mg, 0.14 mmol) and p-toluenesulfonic acid monohydrate (160 mg, 0.14 mmol) were dissolved in acetonitrile (6 mL) and reacted at 80 °C overnight. The reaction mixture was concentrated, and the crude product was purified by HPLC to afford the final compound D1 (60 mg, 95% yield). UPLC-MS (ESI): Calculated for C₂₄H₂₆N₄O₅ [M+H]⁺: 451.50, Found: 451.53. ¹H NMR (500 MHz, DMSO) δ 10.98 (s, 1H), 10.09 (s, 1H), 7.49 (d, J = 8.0 Hz, 2H), 7.34 (d, J = 8.0 Hz, 2H), 7.25 (d, J = 7.7 Hz, 1H), 7.20 (d, J = 7.8 Hz, 1H), 7.05 (t, J = 7.8 Hz, 1H), 5.30 (dd, J = 13.1, 5.2 Hz, 1H), 5.22 (s, 2H), 3.60 - 3.53 (m, 4H), 3.47 (s, 2H), 2.92 (ddd, J = 17.2, 13.8, 5.4 Hz, 1H), 2.65 - 2.58 (m, 1H), 2.57 - 2.51 (m, 1H), 2.34 (s, 4H), 2.10 - 2.03 (m, 1H).¹³C NMR (126 MHz, DMSO) δ 172.78, 169.63, 165.05, 145.19, 137.75, 135.21, 132.16, 129.00, 127.91, 121.77, 118.55, 116.66, 114.81, 112.95, 69.48, 66.20, 62.14, 53.87, 53.16, 48.61, 30.94, 22.36.

### Example 2: (S)-4-(4-(4-(((2-(2,6-dioxopiperidin-3-yl)-3-oxo-2,3-dihydro-1H-indazol-7-yl)oxy)methyl)benzyl)piperazin-1-yl)-3-fluorobenzonitrile (D2)

### Synthetic Route:

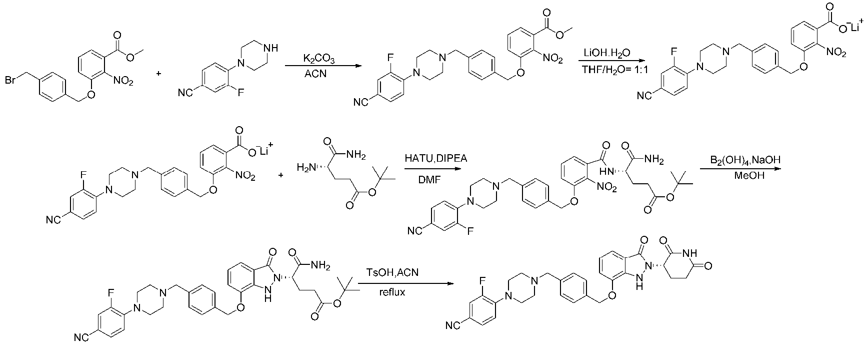

### Step 1:

3-((4-(bromomethyl)benzyl)oxy)-2-nitrobenzoate methyl ester (321 mg, 0.844 mmol), 3-fluoro-4-piperazinylbenzonitrile (191 mg, 0.93 mmol), and potassium carbonate (175 mg, 1.27 mmol) were dissolved in acetonitrile (15 mL) and stirred at room temperature overnight. After completion (TLC monitoring), the mixture was filtered and the filtrate was concentrated. Purification by column chromatography afforded the product (308 mg, white solid, 72% yield). UPLC-MS (ESI):Calculated for C₂₇H₂₅FN₄O₅ [M+H]⁺: 505.52, Found: 505.57.

### Step 2:

3-((4-((4-(4-cyano-2-fluorophenyl)piperazin-1-yl)methyl)benzyl)oxy)-2-nitrobenzoate methyl ester (345 mg, 0.68 mmol) was dissolved in THF (8 mL), followed by addition of aqueous lithium hydroxide monohydrate (43 mg, 1.03 mmol in 8 mL water). The reaction was stirred overnight at room temperature. The reaction mixture was concentrated under reduced pressure and dried to afford 315 mg crude product, which was used directly in the next step without purification.

### Step 3:

The lithium salt 3-((4-((4-(4-cyano-2-fluorophenyl)piperazin-1-yl)methyl)benzyl)oxy)-2-nitrobenzoate (315 mg, 0.68 mmol), (S)-tert-butyl 4,5-diamino-5-oxopentanoate hydrochloride (162 mg, 0.68 mmol), and HATU (259 mg, 0.68 mmol) were dissolved in DMF (10 mL). DIPEA (0.332 mL, 2.04 mmol) was added dropwise and the mixture was stirred at room temperature for 12 h. The reaction mixture was diluted with ethyl acetate, washed with water and saturated NaCl solution, dried over Na₂SO₄, filtered, and concentrated. Column chromatography afforded the product (423 mg, yellow oil, 98% yield). UPLC-MS (ESI): Calculated for C₃₅H₃₉FN₆O₇ [M+H]⁺: 675.73, Found: 675.61.

### Step 4:

(S)-5-amino-4-(3-((4-((4-(4-cyano-2-fluorophenyl)piperazin-1-yl)methyl)benzyl)oxy)-2-nitrobenzamido)-5-oxopentanoate tert-butyl ester (421 mg, 0.62 mmol) was dissolved in methanol (10 mL). At 0 °C, tetrahydroxydiboron (278 mg, 3.1 mmol) was added, followed by NaOH (248 mg, 6.2 mmol) in methanol (10 mL). After stirring 10 min at 0 °C, the mixture was heated to 40 °C and stirred overnight.

After completion (TLC monitoring), the reaction was quenched with saturated NH₄Cl, extracted with ethyl acetate, washed with water and brine, dried over Na₂SO₄, filtered, and concentrated. Column chromatography afforded 265 mg colorless oil (66% yield).

### Step 5:

(S)-5-amino-4-(7-((4-((4-(4-cyano-2-fluorophenyl)piperazin-1-yl)methyl)benzyl)oxy)-3-oxo-1,3-dihydro-2H-indazol-2-yl)-5-oxopentanoate tert-butyl ester (265 mg, 0.41 mmol) and p-toluenesulfonic acid monohydrate (471 mg, 2.47 mmol) were dissolved in acetonitrile (8 mL) and heated at 80 °C overnight. The mixture was concentrated and purified by HPLC to afford the final product D2 (47 mg, 20% yield). UPLC-MS (ESI):Calculated for C₃₁H₂₉FN₆O₄ [M+H]⁺: 569.61, Found: 569.55. ¹H NMR (600 MHz, DMSO-*d*₆) δ 10.97 (s, 1H), 10.10 (s, 1H), 7.68 (dd, *J =* 13.3, 1.5 Hz, 1H), 7.56 (dd, *J =* 8.5, 1.6 Hz, 1H), 7.50 (d, *J =* 7.7 Hz, 2H), 7.37 (d, *J* = 7.6 Hz, 2H), 7.25 (d, *J =* 7.8 Hz, 1H), 7.20 (d, *J =* 7.8 Hz, 1H), 7.11 (s, 1H), 7.06 (t, *J =* 7.8 Hz, 1H), 5.30 (dd, *J* = 13.1, 5.2 Hz, 1H), 5.23 (s, 2H), 3.55 (s, 2H), 3.18 (s, 4H), 2.92 (ddd, *J =* 17.3, 13.8, 5.4 Hz, 1H), 2.64 - 2.58 (m, 1H), 2.56 - 2.50 (m, 5H), 2.10 - 2.03 (m, 1H).¹³C NMR (151 MHz, DMSO-*d*₆) δ 172.66, 169.51, 162.67, 153.05 (d, *J* = 246.6 Hz), 145.07, 143.75, 137.65, 135.16, 129.80, 128.93, 127.84, 121.69, 119.67, 119.50, 119.31, 118.46, 118.30, 114.71, 112.86, 102.11, 69.37, 61.49, 53.75, 52.13, 49.13, 30.82, 22.24.

### Example 3: (S)-4-(4-((5-(((2-(2,6-dioxopiperidin-3-yl)-3-oxo-2,3-dihydro-1H-indazol-7-yl)oxy)methyl)pyridin-2-yl)thio)piperidin-1-yl)-3-fluorobenzonitrile (D3)

### Synthetic Route:

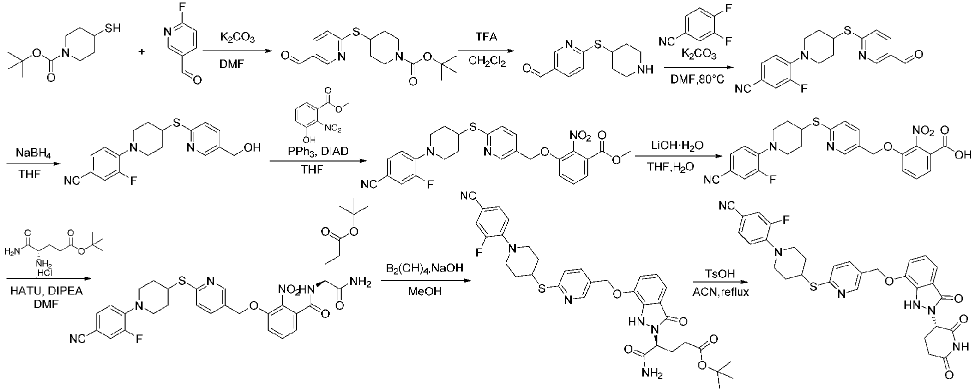

### Step 1:

N-Boc-4-mercaptopiperidine (1.05 g, 4.83 mmol), 2-fluoropyridine-5-carbaldehyde (664 mg, 5.31 mmol), and K₂CO₃ (1.67 g, 12.08 mmol) were dissolved in DMF (10 mL) and heated at 80 °C for 1 h. After solvent removal, the mixture was diluted with water and extracted with ethyl acetate. The organic layer was washed with brine, dried over Na₂SO₄, filtered, and concentrated. Column chromatography afforded 1.5 g product (96% yield). UPLC-MS (ESI) calculated C₁₆H₂₂N₂O₃S [M+H]⁺: 323.42,found 323.53. ¹H NMR (500 MHz, DMSO-d6) δ 10.00 (s, 1H), 8.92 (dd, J = 2.2, 0.8 Hz, 1H), 8.02 (dd, J = 8.4, 2.3 Hz, 1H), 7.48 (d, J = 8.4 Hz, 1H), 4.10 (tt, J = 10.4, 3.9 Hz, 1H), 3.85 - 3.77 (m, 2H), 3.08 (s, 2H), 2.07 - 1.98 (m, 2H), 1.53 (dtd, J = 13.1, 10.5, 4.1 Hz, 2H), 1.41 (s, 9H).

### Step 2:

Compound 4-((5-formylpyridin-2-yl)thio)piperidine-1-tert-butyl carboxylate (1.5 g) was dissolved in DCM (10 mL). TFA (3 mL) was added dropwise under ice bath conditions, and the reaction proceeded 1 h. The mixture was concentrated to afford 1.5 g crude product, which was used directly without purification. UPLC-MS (ESI): calculated C₁₁H₁₄N₂OS [M+H]⁺: 223.31,found 223.14.

### Step 3

A mixture of 6-(piperidin-4-ylthio)nicotinaldehyde trifluoroacetate (1.5 g, 4.46 mmol), 3,4-difluorobenzonitrile (1.24 g, 8.92 mmol), and potassium carbonate (1.85 g, 13.38 mmol) was dissolved in N,N-dimethylformamide (15 mL). The reaction mixture was stirred at 80 °C overnight. After completion of the reaction, the mixture was concentrated under reduced pressure, diluted with water, and extracted with ethyl acetate. The combined organic layers were washed with saturated aqueous sodium chloride solution, dried over anhydrous sodium sulfate, and filtered. The filtrate was concentrated under reduced pressure and the residue was purified by column chromatography to afford the product (1.33 g, 87% yield). UPLC-MS (ESI) calculated for C₁₈H₁₆FN₃OS [M+H]⁺: 342.40, found: 342.29. ¹H NMR (500 MHz, Chloroform-d) δ 10.00 (s, 1H), 8.83 (dd, J = 2.2, 0.9 Hz, 1H), 7.93 (dd, J = 8.4, 2.2 Hz, 1H), 7.37 (ddd, J = 8.4, 1.9, 0.8 Hz, 1H), 7.30 - 7.27 (m, 2H), 6.95 (t, J = 8.5 Hz, 1H), 4.21 (tt, J = 10.1, 4.1 Hz, 1H), 3.60 - 3.54 (m, 2H), 3.11 (ddd, J = 12.7, 10.1, 2.9 Hz, 2H), 2.32 - 2.22 (m, 2H), 1.95 (dtd, J = 13.7, 10.1, 3.7 Hz, 2H). ¹³C NMR (126 MHz, Chloroform-d) δ 189.95, 166.26, 154.00 (d, J = 248.6 Hz), 152.58, 144.28, 134.62, 129.42 (d, J = 3.4 Hz), 128.03, 122.59, 119.74 (d, J = 25.0 Hz), 119.01 (d, J = 3.8 Hz), 118.44, 103.61, 49.71, 49.68, 40.10, 31.91.

### Step 4

Under an ice bath, 3-fluoro-4-(4-((5-formylpyridin-2-yl)thio)piperidin-1-yl)benzonitrile (1.33 g, 3.90 mmol) was dissolved in tetrahydrofuran (20 mL). Sodium borohydride (294 mg, 7.79 mmol) was slowly added and the reaction was stirred for 1 hour. The reaction was quenched with water and extracted with ethyl acetate. The combined organic layers were washed with saturated sodium chloride solution, dried over anhydrous sodium sulfate, filtered, and concentrated under reduced pressure. The residue was purified by column chromatography to afford the product (1.24 g, 93% yield). UPLC-MS (ESI) calculated for C₁₈H₁₈FN₃OS [M+H]⁺: 344.42, found: 344.30. ¹H NMR (500 MHz, Chloroform-d) δ 8.41 (d, J = 2.2 Hz, 1H), 7.54 (dd, J = 8.2, 2.3 Hz, 1H), 7.35 (dd, J = 8.5, 1.9 Hz, 1H), 7.29 - 7.23 (m, 1H), 7.19 (d, J = 8.2 Hz, 1H), 6.93 (t, J = 8.5 Hz, 1H), 4.66 (s, 2H), 4.09 - 4.02 (m, 1H), 3.55 (dt, J = 11.8, 4.3 Hz, 2H), 3.07 (ddd, J = 12.7, 9.9, 2.9 Hz, 2H), 2.23 (dq, J = 12.8, 3.9 Hz, 2H), 1.90 (dtd, J = 13.5, 10.0, 3.6 Hz, 2H).

### Step 5

A mixture of 3-fluoro-4-(4-((5-(hydroxymethyl)pyridin-2-yl)thio)piperidin-1-yl)benzonitrile (376 mg, 1.1 mmol), methyl 2-nitro-3-hydroxybenzoate (180 mg, 0.91 mmol), and triphenylphosphine (287 mg, 1.1 mmol) was dissolved in dry tetrahydrofuran (5 mL). At 0 °C, diisopropyl azodicarboxylate (DIAD) (221 mg, 1.1 mmol) was added dropwise. The reaction mixture was stirred overnight. After concentration under reduced pressure, the residue was purified by column chromatography to afford the product (458 mg, 96% yield). UPLC-MS (ESI) calculated for C₂₆H₂₃FN₄O₅S [M+H]⁺: 523.55, found: 523.33. ¹H NMR (500 MHz, Chloroform-d) δ 8.45 - 8.41 (m, 1H), 7.64 (dd, J = 7.9, 1.1 Hz, 1H), 7.53 (dd, J = 8.3, 2.3 Hz, 1H), 7.50 (t, J = 8.2 Hz, 1H), 7.35 (ddd, J = 8.5, 2.1, 0.8 Hz, 1H), 7.31 (dd, J = 8.4, 1.2 Hz, 1H), 7.27 (dd, J = 12.6, 2.0 Hz, 1H), 7.20 (dd, J = 8.2, 0.9 Hz, 1H), 5.14 (s, 2H), 4.08 (tt, J = 9.9, 4.0 Hz, 1H), 3.91 (s, 3H), 3.59 - 3.52 (m, 2H), 3.09 (ddd, J = 12.7, 10.1, 2.9 Hz, 2H), 2.28 - 2.21 (m, 2H), 1.91 (dtd, J = 13.6, 10.0, 3.7 Hz, 2H).

### Step 6

Compound 3-((6-((1-(4-cyano-2-fluorophenyl)piperidin-4-yl)thio)pyridin-3-yl)methoxy)-2-nitro methyl benzoate (458 mg, 0.87 mmol) was dissolved in tetrahydrofuran (10 mL). An aqueous solution of lithium hydroxide monohydrate (37 mg, 0.87 mmol in 3 mL water) was added. The mixture was stirred at room temperature overnight. The reaction mixture was concentrated under reduced pressure to afford the product (450 mg), which was used in the next step without further purification.

### Step 7

Lithium 3-((6-((1-(4-cyano-2-fluorophenyl)piperidin-4-yl)thio)pyridin-3-yl)methoxy)-2-nitrobenzoate (450 mg, 0.87 mmol), (S)-tert-butyl 4,5-diamino-5-oxopentanoate hydrochloride (208 mg, 0.87 mmol), and HATU (332 mg, 0.87 mmol) were dissolved in N,N-dimethylformamide (10 mL). N,N-diisopropylethylamine (DIPEA) (0.457 mL, 2.62 mmol) was added dropwise and the mixture was stirred at room temperature for 12 hours. The reaction mixture was diluted with ethyl acetate, washed with water and saturated sodium chloride solution, dried over anhydrous sodium sulfate, filtered and concentrated. The residue was purified by column chromatography to give the product (550 mg, 91% yield). UPLC-MS (ESI) calculated for C₃₄H₃₇FN₆O₇S [M+H]⁺: 693.76, found: 693.43. ¹H NMR (500 MHz, Chloroform-d) δ 8.44 (d, J = 2.3 Hz, 1H), 7.57 - 7.44 (m, 3H), 7.35 (dd, J = 8.5, 1.9 Hz, 1H), 7.26 - 7.17 (m, 3H), 6.94 (t, J = 8.5 Hz, 1H), 5.14 (s, 2H), 4.59 (td, J = 8.1, 7.7, 4.7 Hz, 1H), 4.07 (dq, J = 9.5, 4.8, 4.2 Hz, 1H), 3.72 (tt, J = 6.3, 3.0 Hz, 1H), 3.55 (dt, J = 12.6, 4.3 Hz, 2H), 3.17 (dt, J = 11.8, 5.7 Hz, 1H), 3.12 - 3.05 (m, 2H), 2.67 - 2.51 (m, 1H), 2.40 (dt, J = 16.9, 6.5 Hz, 1H), 2.29 - 2.13 (m, 3H), 2.04 (q, J = 7.5 Hz, 1H), 1.95 - 1.86 (m, 2H), 1.79 (s, 1H), 1.44 (s, 9H).

### Step 8

5-amino-4-(3-((6-((1-(4-cyano-2-fluorophenyl)piperidin-4-yl)thio)pyridin-3-yl)methoxy)-2-nitrobenzamido)-5-oxopentanoate tert-butyl ester (550 mg, 0.79 mmol) was dissolved in methanol (7 mL). At 0 °C, bis(pinacolato)diboron (355 mg, 3.97 mmol) and a methanolic solution of sodium hydroxide (317 mg, 7.94 mmol) were added. The mixture was stirred for 10 minutes at 0 °C and then heated to 40 °C overnight. After concentration, the mixture was diluted with ethyl acetate, washed with water and saturated sodium chloride solution, dried over anhydrous sodium sulfate, filtered and concentrated. The residue was purified by column chromatography to afford the product (443 mg, 84% yield). UPLC-MS (ESI) calculated for C₃₄H₃₇FN₆O₅S [M+H]⁺: 661.77, found: 661.46.

### Step 9

5-amino-4-(7-((6-((1-(4-cyano-2-fluorophenyl)piperidin-4-yl)thio)pyridin-3-yl)methoxy)-3-oxo-1,3-dihydro-2H-indazol-2-yl)-5-oxopentanoate tert-butyl ester (150 mg, 0.23 mmol) and p-toluenesulfonic acid monohydrate (259 mg, 1.36 mmol) were dissolved in acetonitrile (3 mL). The reaction mixture was heated at 80 °C overnight. After concentration, the crude product was purified by preparative HPLC to afford the final product (35 mg, 26% yield). UPLC-MS (ESI) calculated for C₃₀H₂₇FN₆O₄S [M+H]⁺: 587.64, found: 587.33. ¹H NMR (500 MHz, DMSO-d6) δ 10.99 (s, 1H), 10.08 (s, 1H), 8.65 (d, J = 2.2 Hz, 1H), 7.81 (dd, J = 8.3, 2.3 Hz, 1H), 7.69 (dd, J = 13.3, 2.0 Hz, 1H), 7.56 (dd, J = 8.4, 2.0 Hz, 1H), 7.36 (d, J = 8.2 Hz, 1H), 7.27 (d, J = 7.8 Hz, 1H), 7.23 (d, J = 7.8 Hz, 1H), 7.16 (t, J = 8.8 Hz, 1H), 7.08 (t, J = 7.8 Hz, 1H), 5.32 (dd, J = 13.1, 5.3 Hz, 1H), 5.24 (s, 2H), 4.03 (tt, J = 10.2, 4.0 Hz, 1H), 3.52 (dt, J = 12.8, 4.1 Hz, 2H), 3.08 (ddd, J = 12.9, 10.3, 2.7 Hz, 2H), 2.93 (ddd, J = 17.2, 13.7, 5.4 Hz, 1H), 2.65 - 2.51 (m, 2H), 2.16 (dq, J = 12.1, 3.6 Hz, 2H), 2.07 (dtd, J = 12.9, 5.4, 2.4 Hz, 1H), 1.76 (dtd, J = 13.7, 10.3, 3.6 Hz, 2H). ¹³C NMR (126 MHz, DMSO-d6) δ 172.76, 169.62, 162.75, 157.46, 153.14 (d, J = 246.1 Hz), 149.18, 144.89, 143.98, 137.75, 136.68, 129.90, 128.28, 122.09, 121.80, 119.82, 119.67, 119.62, 118.58, 118.44, 115.07, 113.03, 102.07 (d, J = 9.9 Hz), 66.99, 53.87, 49.20, 49.16, 31.71, 30.93, 22.37.

### Example 4:4-(4-((5-(((2-(2,6-dioxopiperidin-3-yl)-3-oxo-2,3-dihydro-1H-indazol-7-yl)oxy)methyl)pyridin-2-yl)thio)piperidin-1-yl)-3-fluorobenzonitrile (D4)

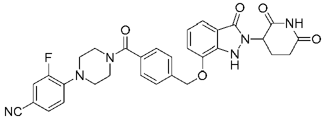

The synthetic method was carried out with reference to Example 1 to afford compound **D4.** ¹H NMR (500 MHz, DMSO-*d*₆) δ 11.00 (s, 1H), 10.14 (s, 1H), 7.74 (dd, *J =* 13.2, 2.0 Hz, 1H), 7.63 (d, *J =* 7.9 Hz, 2H), 7.60 (dd, *J =* 8.4, 1.9 Hz, 1H), 7.52 - 7.46 (m, 2H), 7.27 (d, *J =* 7.8 Hz, 1H), 7.21 (d, *J =* 7.8 Hz, 1H), 7.16 (t, *J =* 8.7 Hz, 1H), 7.07 (t, *J =* 7.8 Hz, 1H), 5.36 - 5.29 (m, 3H), 3.78 (s, 2H), 3.57 - 3.40 (m, 2H), 3.29 - 3.14 (m, 4H), 2.93 (ddd, *J =* 17.2, 13.8, 5.5 Hz, 1H), 2.66 - 2.54 (m, 2H), 2.12 - 2.00 (m, 1H). UPLC-MS ( ESI ) calculated for C₃₁H₂₈FN₆O₅ [M+H]⁺: 583.21, found 583.38.

### Example 5:3-(7-((4-(4-(2-Fluoro-4-methylphenyl)piperazine-1-carbonyl)benzyl)oxy)-3-oxo-1,3-dihydro-2H-indazol-2-yl)piperidine-2,6-dione (D5)

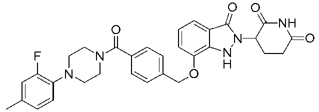

The synthetic method was carried out with reference to Example 1 to afford compound **D5.** ¹H NMR (500 MHz, DMSO-*d*₆) δ 11.00 (s, 1H), 10.14 (s, 1H), 7.62 (d, *J =* 7.9 Hz, 2H), 7.48 (d, *J =* 8.1 Hz, 2H), 7.27 (d, *J =* 7.8 Hz, 1H), 7.21 (d, *J =* 7.8 Hz, 1H), 7.07 (t, *J =* 7.8 Hz, 1H), 7.00 - 6.89 (m, 3H), 5.35 - 5.28 (m, 3H), 3.77 (s, 2H), 3.49 (s, 2H), 3.07 - 2.86 (m, 5H), 2.65 - 2.53 (m, 2H), 2.24 (s, 3H), 2.11 - 2.04 (m, 1H). UPLC-MS ( ESI ) calculated for C₃₁H₃₁FN₅O₅ [M+H]⁺: 572.23, found 572.32.

### Example 6: 3-(7-((4-(((2S,6R)-2,6-dimethylmorpholinyl)methyl)benzyl)oxy)-3-oxo-1,3-dihydro-2H-indazol-2-yl)piperidine-2,6-dione (D6)

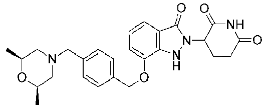

The synthetic method was carried out with reference to Example 1 to afford compound **D6.** ¹H NMR (500 MHz, DMSO-*d*₆) δ 11.00 (s, 1H), 10.11 (s, 1H), 7.65 (d, *J* = 7.9 Hz, 2H), 7.56 (d, *J =* 8.0 Hz, 2H), 7.28 (d, *J =* 7.7 Hz, 1H), 7.21 (d, *J =* 7.8 Hz, 1H), 7.07 (t, *J =* 7.8 Hz, 1H), 5.39 - 5.27 (m, 3H), 4.34 (s, 2H), 3.82 (tdd, *J =* 12.4, 6.1, 1.9 Hz, 3H), 3.27 (s, 1H), 2.94 (ddd, *J* = 17.1, 13.8, 5.4 Hz, 1H), 2.69 (t, *J* = 11.5 Hz, 2H), 2.63 (dt, *J* = 17.7, 3.2 Hz, 1H), 2.58 - 2.52 (m, 1H), 2.13 - 2.03 (m, 1H), 1.12 (d, *J* = 6.3 Hz, 6H). UPLC-MS ( ESI ) calculated for C₂₆H₃₁N₄O₅ [M+H]⁺: 479.22, found 479.45.

### Example 7: 3-(7-((4-((4-(2,4-difluorophenyl)piperazin-1-yl)methyl)benzyl)oxy)-3-oxo-1,3-dihydro-2H-indazol-2-yl)piperidine-2,6-dione (D7)

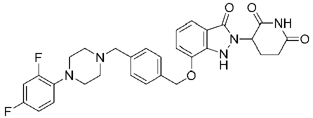

The synthetic method was carried out with reference to Example 1 to afford compound **D7.** ¹H NMR (500 MHz, DMSO) δ 10.98 (s, 1H), 10.11 (s, 1H), 7.50 (d, *J* = 7.6 Hz, 2H), 7.37 (d, *J* = 7.6 Hz, 2H), 7.25 (d, *J =* 7.8 Hz, 1H), 7.23 - 7.15 (m, 2H), 7.06 (td, *J =* 8.7, 5.7 Hz, 2H), 6.99 (t, *J* = 5.3 Hz, 1H), 5.31 (dd, *J =* 13.1, 5.1 Hz, 1H), 5.23 (s, 2H), 3.54 (s, 2H), 3.35 (s, 4H) , 2.96 (s, 4H), 2.93 - 2.87 (m, 1H), 2.66 - 2.57 (m, 1H), 2.54 - 2.51 (m, 1H) , 2.11 - 2.02 (m, 1H). UPLC-MS ( ESI ) calculated for C₃₀H₂₉F₂N₅O₄ [M+H]⁺: 562.22, found 562.37.

### Example 8: 3-(7-((4-((4-(3-chlorophenyl)piperazin-1-yl)methyl)benzyl)oxy)-3-oxo-1,3-dihydro-2H-indazol-2-yl)piperidine-2,6-dione (D8)

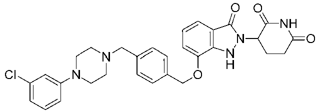

The synthetic method was carried out with reference to Example 1 to afford compound **D8.** ¹H NMR (500 MHz, DMSO) δ 10.98 (s, 1H), 10.10 (s, 1H), 7.51 (d, *J =* 7.5 Hz, 2H), 7.38 (d, *J* = 7.7 Hz, 2H), 7.26 (d, *J =* 7.9 Hz, 1H), 7.21 (d, *J =* 8.0 Hz, 2H), 7.07 (t, *J =* 7.8 Hz, 1H), 6.92 (s, 1H), 6.88 (d, *J* = 8.6 Hz, 1H), 6.78 (d, *J* = 7.8 Hz, 1H), 5.31 (dd, *J =* 13.1, 5.1 Hz, 1H), 5.24 (s, 2H), 3.54 (s, 2H), 3.34-3.29 (m, 4H), 3.17 (s, 4H), 2.98 - 2.87 (m, 1H), 2.63 (d, 1H), 2.54-2.51 (m, 1H), 2.10 - 2.04 (m, 1H). UPLC-MS ( ESI ) calculated for C₃₀H₃₁ClN₅O₄ [M+H]⁺: 560.20, found 560.28.

### Example 9: 3-(7-((4-((4-(4-chlorophenyl)piperazin-1-yl)methyl)benzyl)oxy)-3-oxo-1,3-dihydro-2H-indazol-2-yl)piperidine-2,6-dione (D9)

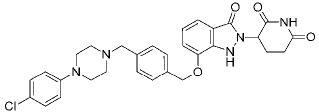

The synthetic method was carried out with reference to Example 1 to afford compound **D9.** ¹H NMR (500 MHz, DMSO) δ 10.98 (s, 1H), 10.10 (s, 1H), 7.51 (d, *J =* 7.7 Hz, 2H), 7.37 (d, *J =* 7.6 Hz, 2H), 7.26 (d, *J =* 7.8 Hz, 1H), 7.23 - 7.19 (m, 3H), 7.07 (t, *J =* 7.8 Hz, 1H), 6.96 - 6.90 (m, 2H), 5.31 (dd, *J =* 13.1, 5.2 Hz, 1H), 5.23 (s, 2H), 3.56 - 3.52 (m, 2H), 3.34-3.29 (m, 4H), 3.13 (t, *J* = 5.0 Hz, 4H), 2.98 - 2.87 (m, 1H), 2.66 - 2.58 (m, 1H), 2.54-2.51 (m, 1H), 2.10 - 2.04 (m, 1H).UPLC-MS ( ESI ) calculated for C₃₀H₃₁ClN₅O₄ [M+H]⁺: 560.20, found 560.32.

### Example 10: 3-(7-((4-((4-(4-fluorophenyl)piperazin-1-yl)methyl)benzyl)oxy)-3-oxo-1,3-dihydro-2H-indazol-2-yl)piperidine-2,6-dione (D10)

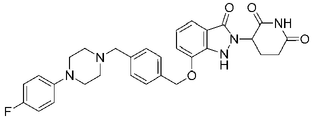

The synthetic method was carried out with reference to Example 1 to afford compound **D10.** ¹H NMR (500 MHz, DMSO) δ 10.98 (s, 1H), 10.10 (s, 1H), 7.51 (d, J = 7.7 Hz, 2H), 7.38 (d, J = 7.7 Hz, 2H), 7.26 (d, J = 7.8 Hz, 1H), 7.21 (d, J = 7.8 Hz, 1H), 7.08 (d, J = 7.8 Hz, 1H), 7.06 - 7.01 (m, 2H), 6.95 - 6.91 (m, 2H), 5.31 (dd, J = 13.1, 5.2 Hz, 1H), 5.23 (s, 2H), 3.54 (d, J = 2.6 Hz, 2H), 3.34-3.29 (m, 4H), 3.07 (s, 4H), 2.98 - 2.87 (m, 1H), 2.64 - 2.57 (m, 1H), 2.55 - 2.52 (m, 1H), 2.10 - 2.04 (m, 1H). UPLC-MS ( ESI ) calculated for C₃₀H₃₁FN₅O₄ [M+H]⁺: 544.23, found 544.32.

### Example 11: 3-(3-Oxo-7-((4-((4-(4-(trifluoromethyl)phenyl)piperazin-1-yl)methyl)benzyl)oxy)-1,3-dihydro-2H-indazol-2-yl)piperidine-2,6-dione (D11)

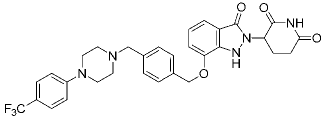

The synthetic method was carried out with reference to Example 1 to afford compound **D11.** ¹H NMR (500 MHz, DMSO) δ 11.00 (s, 1H), 10.18 (s, 1H), 7.63 (q, *J* = 8.2 Hz, 2H), 7.55 (d, *J =* 8.4 Hz, 1H), 7.42 (s, 2H), 7.32 (s, 2H), 7.26 (d, *J =* 8.1 Hz, 1H), 7.22 (d, *J =* 7.1 Hz, 2H), 7.09 (dd, *J =* 13.6, 8.1 Hz, 1H), 5.32 (dd, *J =* 12.9, 5.1 Hz, 1H), 5.28 (s, 2H), 4.22 (s, 2H), 3.57 (s, 2H), 3.51 (s, 2H), 3.49 (s, 3H), 3.47 (s, 2H), 2.89 (m, 1H), 2.67 - 2.55 (m, 1H), 2.08 (m, 1H). UPLC-MS ( ESI ) calculated for C₃₀H₃₁F₃N₅O₄ [M+H]⁺ 594.22, found 594.34.

### Example 12: 3-(7-((4-((4-(2,4-dichlorophenyl)piperazin-1-yl)methyl)benzyl)oxy)-3-oxo-1,3-dihydro-2H-indazol-2-yl)piperidine-2,6-dione (D12)

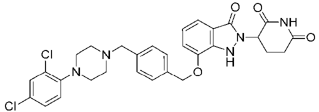

The synthetic method was carried out with reference to Example 1 to afford compound **D12.** ¹H NMR (500 MHz, DMSO) δ 11.01 (s, 1H), 10.12 (s, 1H), 7.70 - 7.64 (m, 2H), 7.64 - 7.56 (m, 3H), 7.42 (dd, *J =* 8.7, 2.5 Hz, 1H), 7.30 - 7.22 (m, 2H), 7.21 (d, *J =* 7.8 Hz, 1H), 7.07 (t, *J =* 7.8 Hz, 1H), 5.34 (d, *J* = 5.2 Hz, 1H), 5.31 (s, 2H), 4.45 (s, 2H), 3.57 (t, *J =* 5.5 Hz, 4H), 3.25 (s, 2H), 3.08 - 2.97 (m, 2H), 2.97 - 2.88 (m, 1H), 2.62 (m , 1H), 2.54-2.51(m, 1H), 2.09 (m , 1H). UPLC-MS ( ESI ) calculated for C₃₀H₃₀Cl₂N₅O₄ [M+H]⁺: 594.16, found 594.25.

### Example 13: 3-(7-((4-((3-morpholinoazetidin-1-yl)methyl)benzyl)oxy)-3-oxo-1,3-dihydro-2H-indazol-2-yl)piperidine-2,6-dione (D13)

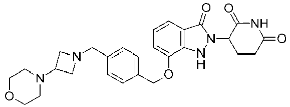

The synthetic method was carried out with reference to Example 1 to afford compound **D13.** ¹H NMR (500 MHz, DMSO) δ 11.00 (s, 1H), 7.62 (d, *J =* 8.0 Hz, 2H), 7.52 (d, *J =* 8.0 Hz, 2H), 7.27 (d, *J =* 7.8 Hz, 1H), 7.20 (d, *J =* 7.7 Hz, 1H), 7.07 (t, *J =* 7.8 Hz, 1H), 5.32 (dd, *J =* 13.2, 5.2 Hz, 1H), 5.29 (s, 2H), 4.45 (s, 2H), 4.20 (s, 4H), 3.73 (s, 5H), 3.42 (s, 1H) , 2.99 - 2.88 (m, 1H), 2.78 - 2.68 (m, 3H), 2.65 - 2.60 (m, 1H), 2.54-2.51(m, 1H) , 2.13 - 2.05 (m, 1H). UPLC-MS ( ESI ) calculated for C₂₇H₃₂N₅O₅ [M+H]⁺: 506.23, found 506.34.

### Example 14: 4-(4-(4-(((2-(2,6-dioxopiperidin-3-yl)-5-fluoro-3-oxo-2,3-dihydro-1H-indazol-7-yl)oxy)methyl)benzyl)piperazin-1-yl)-3-fluorobenzonitrile (D14)

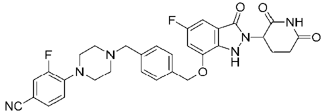

The synthetic method was carried out with reference to Example 1 to afford compound **D14.** ¹H NMR (400 MHz, DMSO) δ 11.00 (s, 1H), 10.11 (s, 1H), 7.78 (dd, *J =* 13.1, 1.9 Hz, 1H), 7.67 - 7.61 (m, 3H), 7.57 (d, *J =* 7.9 Hz, 2H), 7.22 (dd, *J =* 10.3, 7.2 Hz, 2H), 7.04 (dd, *J =* 7.6, 2.2 Hz, 1H), 5.31 (d, *J =* 12.8 Hz, 3H), 4.42 (s, 2H), 3.72 (d, *J =* 13.0 Hz, 2H) , 3.41 (s, 2H), 3.25 (s, 2H), 3.16 (d, *J =* 12.6 Hz, 2H), 2.98 - 2.90 (m, 1H) , 2.61 (d, *J =* 16.8 Hz, 1H), 2.54 - 2.51 (m, 1H) , 2.13 - 2.03 (m, 1H).UPLC-MS ( ESI ) calculated for C₃₁H₂₉F₂N₆O₄ [M+H]⁺: 587.21, found 587.62.

### Example 15: 3-(7-((4-((4-(2,4-difluorophenyl)piperazin-1-yl)methyl)benzyl)oxy)-5-fluoro-3-oxo-1,3-dihydro-2H-indazol-2-yl)piperidine-2,6-dione (D15)

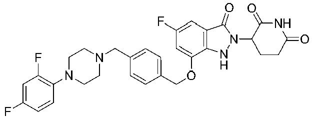

The synthetic method was carried out with reference to Example 1 to afford compound **D15.** ¹H NMR (500 MHz, DMSO) δ 11.02 (s, 1H), 10.14 (s, 1H), 7.66 (d, *J =* 7.7 Hz, 2H), 7.59 (d, *J =* 7.8 Hz, 2H), 7.32 - 7.20 (m, 2H), 7.15 (td, *J =* 9.5, 5.8 Hz, 1H), 7.05 (dd, *J =* 7.5, 2.5 Hz, 2H), 5.34 (s, 3H), 4.44 (s, 2H) , 4.2 (s, 2H), 3.44 (s, 4H), 3.26 (s, 2H) , 2.95 - 2.89 (m, 1H), 2.66 - 2.58 (m, 1H), 2.54 - 2.51 (m, 1H) , 2.14 - 2.06 (m, 1H). UPLC-MS ( ESI ) calculated for C₃₁H₂₉F₂N₆O₄ [M+H]⁺: 580.21, found 580.63.

### Example 16: 3-(7-((4-((4-(2,4-dichlorophenyl)piperazin-1-yl)methyl)benzyl)oxy)-5-fluoro-3-oxo-1,3-dihydro-2H-indazol-2-yl)piperidine-2,6-dione (D16)

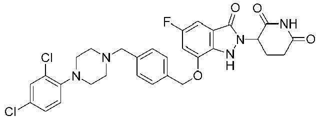

The synthetic method was carried out with reference to Example 1 to afford compound **D16.** ¹H NMR (500 MHz, DMSO) δ 11.00 (s, 1H), 10.15 (s, 1H), 7.56 - 7.48 (m, 3H), 7.41 - 7.36 (m, 3H), 7.26 - 7.20 (m, 1H), 7.17 (d, *J =* 8.7 Hz, 1H), 7.03 (dd, *J =* 7.5, 2.3 Hz, 1H), 5.32 (dd, *J =* 13.0, 5.1 Hz, 1H), 5.25 (s, 2H), 3.57 (s, 2H) , 3.35 (s, 4H) , 2.97 (s, 4H), 2.93 - 2.88 (m, 1H), 2.62 - 2.58 (m, 1H), 2.54 - 2.51 (m, 1H) , 2.10 - 2.04 (m, 1H).UPLC-MS ( ESI ) calculated for C₃₀H₂₉C₁₂FN₅O₄ [M+H]⁺: 612.15, found 612.30.

### Example 17: 3-(5-Fluoro-3-oxo-7-((4-((4-(4-(trifluoromethyl)phenyl)piperazin-1-yl)methyl)benzyl)oxy)-1,3-dihydro-2H-indazol-2-yl)piperidine-2,6-dione (D17)

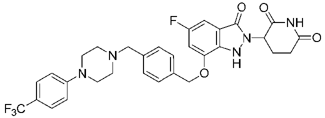

The synthetic method was carried out with reference to Example 1 to afford compound **D17.** ¹H NMR (500 MHz, DMSO) δ 11.01 (s, 1H), 10.15 (s, 1H), 7.51 (t, *J =* 8.0 Hz, 4H), 7.40 (d, *J =* 7.7 Hz, 2H), 7.23 (dd, *J =* 11.3, 2.3 Hz, 1H), 7.05 (dd, *J =* 8.1, 5.6 Hz, 3H), 5.32 (dd, *J =* 13.2, 5.2 Hz, 1H), 5.26 (s, 2H), 3.55 (d, *J =* 8.5 Hz, 2H), 3.35 (s, 4H) , 3.28 (t, *J =* 5.0 Hz, 4H), 2.98 - 2.87 (m, 1H), 2.65 - 2.57 (m, 1H), 2.54 - 2.51 (m, 1H) , 2.12 - 2.03 (m, 1H). UPLC-MS ( ESI ) calculated for C₃₁H₃₀F₄N₅O₄ [M+H]⁺: 612.22, found 612.36.

### Example 18: 3-(7-((4-((4-(4-chlorophenyl)piperazin-1-yl)methyl)benzyl)oxy)-5-fluoro-3-oxo-1,3-dihydro-2H-indazol-2-yl)piperidine-2,6-dione (D18)

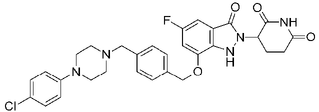

The synthetic method was carried out with reference to Example 1 to afford compound **D18.** ¹H NMR (500 MHz, DMSO) δ 11.01 (s, 1H), 10.15 (s, 1H), 7.51 (d, *J =* 7.7 Hz, 2H), 7.39 (d, *J =* 7.7 Hz, 2H), 7.23 (dd, *J* = 9.5, 2.9 Hz, 3H), 7.04 (dd, *J =* 7.6, 2.2 Hz, 1H), 6.96 - 6.90 (m, 2H), 5.32 (dd, *J =* 13.1, 5.2 Hz, 1H), 5.25 (s, 2H), 3.54 (d, *J* = 4.3 Hz, 2H), 3.35 (s, 4H) , 3.13 (t, *J =* 4.9 Hz, 4H), 2.98 - 2.87 (m, 1H), 2.65 - 2.58 (m, 1H), 2.54 - 2.51 (m, 1H) , 2.08 - 2.05 (m, 1H). UPLC-MS ( ESI ) calculated for C₃₀H₃₀ClFN₅O4 [M+H]⁺: 578.19, found 578.36.

### Example 19: 3-(5-Fluoro-7-((4-((4-(4-fluorophenyl)piperazin-1-yl)methyl)benzyl)oxy)-3-oxo-1,3-dihydro-2H-indazol-2-yl)piperidine-2,6-dione (D19)

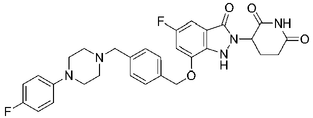

The synthetic method was carried out with reference to Example 1 to afford compound **D19.** ¹H NMR (500 MHz, DMSO) δ 11.01 (s, 1H), 10.15 (s, 1H), 7.51 (d, *J =* 7.7 Hz, 2H), 7.39 (d, *J =* 7.7 Hz, 2H), 7.23 (dd, *J =* 11.2, 2.2 Hz, 1H), 7.08 - 7.00 (m, 3H), 6.97 - 6.89 (m, 2H), 5.32 (dd, *J =* 13.2, 5.2 Hz, 1H), 5.25 (s, 2H), 3.54 (d, *J =* 6.3 Hz, 2H), 3.35 (s, 4H), 3.07 (d, *J =* 5.8 Hz, 4H), 2.97 - 2.87 (m, 1H), 2.64 - 2.57 (m, 1H), 2.54 - 2.51 (m, 1H) , 2.11 - 2.03 (m, 1H). UPLC-MS ( ESI ) calculated for C₃₀H₃₀F₂N₅O₄ [M+H]⁺: 562.22, found 562.33.

### Example 20: 3-(5-Fluoro-7-((4-((3-morpholinoazetidin-1-yl)methyl)benzyl)oxy)-3-oxo-1,3-dihydro-2H-indazol-2-yl)piperidine-2,6-dione (D20)

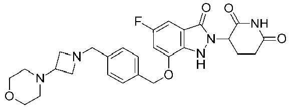

The synthetic method was carried out with reference to Example 1 to afford compound **D20.** ¹H NMR (500 MHz, DMSO) δ 11.01 (s, 1H), 10.15 (s, 1H), 7.48 (d, *J =* 7.8 Hz, 2H), 7.34 (d, *J =* 8.0 Hz, 2H), 7.21 (dd, *J =* 11.2, 2.3 Hz, 1H), 7.03 (dd, *J =* 7.6, 2.2 Hz, 1H), 5.32 (dd, *J =* 13.2, 5.2 Hz, 1H), 5.24 (s, 2H), 3.67 (s, 2H), 3.60 - 3.51 (m, 5H), 3.42 (s, 1H) , 2.98 - 2.86 (m, 4H), 2.65 - 2.56 (m, 1H), 2.54 - 2.51 (m, 1H) , 2.23 (s, 4H), 2.11 - 2.03 (m, 1H). UPLC-MS ( ESI ) calculated for C₂₇H₃₁FN₅O₅ [M+H]⁺: 524.22, found 524.47.

### Example 21: 3-(7-((4-((4-(4-chloro-2-fluorophenyl)piperazin-1-yl)methyl)benzyl)oxy)-5-fluoro-3-oxo-1,3-dihydro-2H-indazol-2-yl)piperidine-2,6-dione (D21)

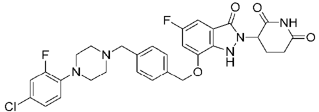

The synthetic method was carried out with reference to Example 1 to afford compound **D21.** ¹H NMR (500 MHz, DMSO) δ 11.02 (s, 1H), 10.17 (s, 1H), 7.66 (d, *J =* 8.0 Hz, 2H), 7.59 (d, *J =* 8.1 Hz, 2H), 7.42 (dd, *J =* 12.2, 2.4 Hz, 1H), 7.26 - 7.20 (m, 2H), 7.12 (t, *J =* 9.1 Hz, 1H), 7.05 (dd, *J =* 7.6, 2.2 Hz, 1H), 5.37 - 5.30 (m, 3H), 4.45 (s, 2H), 3.55 - 3.48 (m, 2H), 3.42 (s, 2H), 3.25(s, 2H) , 3.05 (s, 2H), 2.99 - 2.88 (m, 1H), 2.66 - 2.58 (m, 1H), 2.54 - 2.51 (m, 1H) , 2.14 - 2.05 (m, 1H). UPLC-MS ( ESI ) calculated for C₃₀H₂₉ClF₂N₅O₄ [M+H]⁺: 596.18, found 596.27.

### Example 22: 3-(1-Methyl-7-((4-(morpholinomethyl)benzyl)oxy)-3-oxo-1,3-dihydro-2H-indazol-2-yl)piperidine-2,6-dione (D22)

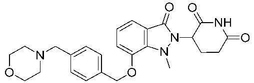

The synthetic method was carried out with reference to Example 1 to afford compound **D22.** ¹H NMR (500 MHz, DMSO) δ 11.03 (s, 1H), 7.61 (d, *J* = 7.7 Hz, 2H), 7.57 (d, *J* = 6.9 Hz, 2H), 7.33 (d, *J =* 8.0 Hz, 1H), 7.29 (d, *J =* 7.8 Hz, 1H), 7.20 (dd, *J =* 8.8, 6.8 Hz, 1H), 5.35 (s, 2H), 5.10 - 5.04 (m, 1H), 4.37 (s, 2H), 3.97 (d, *J =* 12.9 Hz, 4H), 3.65 (d, *J =* 12.3 Hz, 4H), 2.81 (t, *J =* 14.6 Hz, 1H), 2.72 - 2.58 (m, 2H), 2.04 (d, *J* = 11.9 Hz, 1H), 1.27 - 1.19 (m, 3H). UPLC-MS ( ESI ) calculated for C₂₅H₂₉N₄O₅ [M+H]⁺:465.52, found 465.37.

### Example 23: 3-(1-Ethyl-7-((4-(morpholinomethyl)benzyl)oxy)-3-oxo-1,3-dihydro-2H-indazol-2-yl)piperidine-2,6-dione (D23)

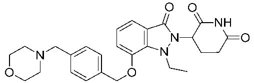

The synthetic method was carried out with reference to Example 1 to afford compound **D23.** ¹H NMR (500 MHz, DMSO) δ 11.03 (s, 1H), 7.61 (d, *J* = 8.0 Hz, 2H), 7.57 (d, *J* = 7.9 Hz, 2H), 7.33 (d, *J =* 7.9 Hz, 1H), 7.28 (d, *J =* 7.6 Hz, 1H), 7.20 (t, *J =* 7.8 Hz, 1H), 5.35 (s, 2H), 5.07 (dd, *J* = 12.6, 5.3 Hz, 1H), 4.37 (s, 2H), 4.11 (dq, *J =* 13.8, 6.7 Hz, 2H), 3.97 (d, *J =* 12.9 Hz, 4H), 3.64 (t, *J* = 12.2 Hz, 4H), 3.27 (d, *J =* 12.8 Hz, 2H), 2.87 - 2.76 (m, 1H), 2.72 - 2.57 (m, 2H), 2.07 - 1.99 (m, 1H), 1.23 (s, 3H). UPLC-MS ( ESI ) calculated for C₂₆H₃₁N₄O₅ [M+H]⁺: 479.55, found 479.86.

### Example 24: 4-(4-(4-(((2-(2,6-dioxopiperidin-3-yl)-1-methyl-3-oxo-2,3-dihydro-1H-indazol-7-yl)oxy)methyl)benzyl)piperazin-1-yl)-3-fluorobenzonitrile (D24)

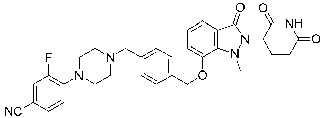

The synthetic method was carried out with reference to Example 1 to afford compound **D24.** ¹H NMR (500 MHz, DMSO) δ 11.05 (s, 1H), 7.83 - 7.76 (m, 1H), 7.64 (d, *J =* 10.5 Hz, 3H), 7.59 (d, *J* = 7.8 Hz, 2H), 7.34 (dd, *J* = 7.9, 1.0 Hz, 1H), 7.30 (dd, *J* = 7.8, 0.9 Hz, 1H), 7.22 (td, *J* = 8.2, 2.7 Hz, 2H), 5.35 (s, 2H), 5.14 (dd, *J* = 12.6, 5.3 Hz, 1H), 4.44 (s, 2H), 3.73 (d, *J* = 13.1 Hz, 3H), 3.26 (s, 3H), 3.19 (d, *J* = 12.6 Hz, 2H), 2.87 - 2.78 (m, 1H), 2.73 - 2.58 (m, 2H), 2.10 - 2.04 (m, 1H), 1.24 (s, 3H) UPLC-MS ( ESI ) calculated for C₃₂H₃₂FN₆O₄ [M+H]⁺: 583.64, found 583.49.

### Example 25: 4-(4-(4-(((2-(2,6-dioxopiperidin-3-yl)-1-methyl-3-oxo-2,3-dihydro-1H-indazol-7-yl)oxy)methyl)benzyl)piperazin-1-yl)-3-fluorobenzonitrile (D25)

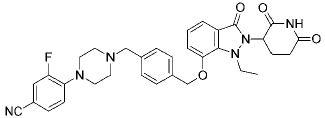

The synthetic method was carried out with reference to Example 1 to afford compound **D25.** ¹H NMR (500 MHz, DMSO) δ 11.04 (s, 1H), 7.79 (dd, *J =* 13.1, 1.9 Hz, 1H), 7.64 - 7.58 (m, 5H), 7.36 - 7.27 (m, 2H), 7.23 - 7.17 (m, 2H), 5.35 (s, 2H), 5.07 (dd, *J =* 12.5, 5.3 Hz, 1H), 4.43 (s, 2H), 4.12 (dq, *J =* 14.0, 6.9 Hz, 1H), 3.79 (dd, *J =* 15.0, 7.1 Hz, 2H), 3.44 (d, *J =* 12.2 Hz, 4H), 2.87 - 2.76 (m, 1H), 2.71 - 2.58 (m, 2H), 2.07 - 2.01 (m, 1H), 1.30 - 1.22 (m, 3H). UPLC-MS ( ESI ) calculated for C₃₃H₃₄FN₆O₄ [M+H]⁺: 597.66, found 597.35.

### Example 26: (S)-3-(3-Oxo-7-((2-(4-(trifluoromethoxy)phenyl)oxazol-5-yl)methoxy)-1,3-dihydro-2H-indazol-2-yl)piperidine-2,6-dione (D26)

### Synthetic Rout:

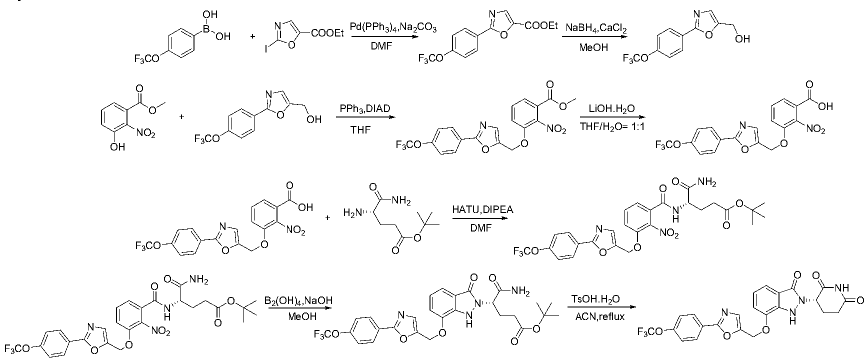

### Step 1:

4-(Trifluoromethoxy)phenylboronic acid (1 g, 4.89 mmol), ethyl 2-iodooxazole-5-carboxylate (1 g, 3.25 mmol), sodium carbonate (795 mg, 7.5 mmol), and tetrakis(triphenylphosphine)palladium (433 mg, 0.375 mmol) were added to a mixed solvent of 1,4-dioxane (15 mL) and water (3 mL). The reaction mixture was purged with nitrogen and stirred at 90 °C overnight under a nitrogen atmosphere. After completion of the reaction, the mixture was concentrated under reduced pressure to remove 1,4-dioxane. The residue was extracted with ethyl acetate. The combined organic layers were washed with saturated sodium chloride solution, dried over anhydrous sodium sulfate, filtered, and concentrated under reduced pressure. The residue was purified by silica gel column chromatography to afford the product (661 mg, 59% yield) as a yellow oil. UPLC-MS ( ESI ) calculated for C₁₃H₁₀F₃NO₄ [M + H ]⁺: 302.22, found 302.17.

### Step 2:

Ethyl 2-(4-(trifluoromethoxy)phenyl)oxazole-5-carboxylate (611 mg, 2 mmol) was dissolved in ethanol (15 mL), and calcium chloride (1.3 g, 12 mmol) was added. The reaction mixture was cooled to 0 °C, and sodium borohydride (227 mg, 6 mmol) was slowly added. The mixture was then warmed to room temperature and stirred overnight. The reaction was quenched by addition of saturated ammonium chloride solution (5 mL) and diluted with water. The mixture was extracted with ethyl acetate, and the combined organic layers were washed with saturated sodium chloride solution, dried over anhydrous sodium sulfate, filtered, and concentrated under reduced pressure. The residue was purified by silica gel column chromatography to give the product (311 mg, 59% yield) as a white solid. UPLC-MS ( ESI ) calculated for C₁₁H₈F₃NO₃ [M + H ]⁺: 260.18, found 260.26.

### Step 3:

(2-(4-(Trifluoromethoxy)phenyl)oxazol-5-yl)methanol (200 mg, 1 mmol), methyl 2-nitro-3-hydroxybenzoate (315 mg, 1.2 mmol), and triphenylphosphine (315 mg, 1.2 mmol) were dissolved in anhydrous tetrahydrofuran (8 mL). Diisopropyl azodicarboxylate (0.236 mL) was slowly added dropwise at 0 °C, and the reaction mixture was stirred overnight. The reaction mixture was concentrated under reduced pressure, and the residue was purified by column chromatography to afford the product (400 mg, 89% yield) as a yellow oil. UPLC-MS ( ESI ) calculated for C₁₉H₁₃F₃N₂O₇ [M + H ]⁺: 439.32, found 439.40.

### Step 4:

Methyl 2-nitro-3-((2-(4-(trifluoromethoxy)phenyl)oxazol-5-yl)methoxy)benzoate (440 mg, 1 mmol) was dissolved in tetrahydrofuran (8 mL), followed by addition of an aqueous solution of lithium hydroxide monohydrate (63 mg, 1.5 mmol in 4 mL water). The reaction mixture was stirred at room temperature overnight. The reaction mixture was concentrated under reduced pressure and dried to afford the product (430 mg), which was used directly in the next step without further purification.

### Step 5:

Lithium 2-nitro-3-((2-(4-(trifluoromethoxy)phenyl)oxazol-5-yl)methoxy)benzoate (431 mg, 1 mmol), (S)-tert-butyl 4,5-diamino-5-oxopentanoate hydrochloride (239 mg, 1 mmol), and N,N,N',N'-tetramethyl-O-(7-azabenzotriazol-1-yl)uronium hexafluorophosphate (381 mg, 1 mmol) were dissolved in N,N-dimethylformamide (10 mL). N,N-diisopropylethylamine (0.523 mL, 3 mmol) was added dropwise, and the mixture was stirred at room temperature for 12 h. The reaction mixture was diluted with ethyl acetate, washed with water and saturated sodium chloride solution, dried over anhydrous sodium sulfate, filtered, and concentrated under reduced pressure. The residue was purified by silica gel column chromatography to afford the product (530 mg, 87% yield).UPLC-MS ( ESI ) calculated for C₂₇H₂₇F₃N₄O₉ [M + H ]⁺: 609.53, found 609.50.

### Step 6:

(S)-tert-Butyl 5-amino-4-(2-nitro-3-((2-(4-(trifluoromethoxy)phenyl)oxazol-5-yl)methoxy)benzamido)-5-oxopentanoate (420 mg, 0.69 mmol) was dissolved in methanol (7 mL). At 0 °C, bis(pinacolato)diboron (309 mg, 3.45 mmol) was added, followed by addition of a methanolic sodium hydroxide solution (276 mg NaOH in 9 mL MeOH). The mixture was stirred at 0 °C for 10 min and then heated to 40 °C and stirred overnight. After completion of the reaction (monitored by TLC), the mixture was quenched with saturated ammonium chloride solution and extracted with ethyl acetate. The organic phase was washed with water and saturated sodium chloride solution, dried over anhydrous sodium sulfate, filtered, and concentrated under reduced pressure. The residue was purified by silica gel column chromatography to afford the product (123 mg, 31% yield) as a yellow oil. UPLC-MS ( ESI ) calculated for C₂₇H₂₇F₃N₄O₇ [M + H ]⁺: 577.53, found 577.56:

### Step 7:

(S)-tert-Butyl 5-amino-5-oxo-4-(3-oxo-7-((2-(4-(trifluoromethoxy)phenyl)oxazol-5-yl)methoxy)-1,3-dihydro-2H-indazol-2-yl)pentanoate (123 mg, 0.21 mmol) and p-toluenesulfonic acid monohydrate (240 mg, 1.26 mmol) were dissolved in acetonitrile (6 mL). The mixture was heated at 80 °C overnight. The reaction mixture was concentrated and purified by HPLC to afford the product (35 mg, 33% yield). UPLC-MS ( ESI ) calculated for C₂₃H₁₇F₃N₄O₆ [M + H ]⁺: 503.41, found 503.31.¹H NMR (500 MHz, DMSO-d6) δ 11.04 (s, 1H), 10.72 (s, 1H), 7.76 (d, J = 8.9 Hz, 2H), 7.47 (d, J = 8.2 Hz, 2H), 7.14 - 7.11 (m, 1H), 7.08 (t, J = 7.6 Hz, 1H), 7.05 (s, 1H), 7.03 - 7.00 (m, 1H), 5.37 (d, J = 16.3 Hz, 1H), 5.17 (dd, J = 12.4, 5.1 Hz, 1H), 5.09 (d, J = 16.4 Hz, 1H), 2.91 - 2.81 (m, 1H), 2.76 - 2.64 (m, 2H), 2.28 - 2.21 (m, 1H).

### Example 27: (S)-3-(7-((2-(4-chlorophenyl)oxazol-5-yl)methoxy)-3-oxo-1,3-dihydro-2H-indazol-2-yl)piperidine-2,6-dione (D27)

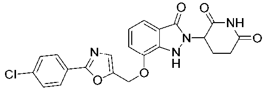

The synthetic method was carried out with reference to Example 26 to afford compound **D27.** ¹H NMR (500 MHz, DMSO) δ 11.02 (s, 1H), 10.59 (s, 1H), 7.61 - 7.55 (m, 2H), 7.15 - 7.05 (m, 3H), 7.05 - 6.97 (m, 3H), 5.40 - 5.31 (m, 2H), 5.17 (dd, J = 12.5, 5.2 Hz, 1H), 2.71 (dd, J = 17.2, 4.5 Hz, 2H), 2.27 (d, *J =* 21.1 Hz, 2H). UPLC-MS ( ESI ) calculated for C₂₂H₁₈ClN₄O₅ [M + H ]⁺: 453.85, found 453.23.

### Example 28: 3-(7-((2-(4-methoxyphenyl)oxazol-5-yl)methoxy)-3-oxo-1,3-dihydro-2H-indazol-2-yl)piperidine-2,6-dione (D28)

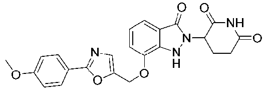

The synthetic method was carried out with reference to Example 26 to afford compound **D28.** ¹H NMR (500 MHz, DMSO) δ 11.04 (s, 1H), 10.69 (s, 1H), 7.61 - 7.55 (m, 2H), 7.15 - 7.05 (m, 3H), 7.05 - 6.97 (m, 3H), 6.96 (s, 1H), 5.40 - 5.31 (m, 2H), 5.03 (s, 1H), 3.80 (s, 3H), 2.26 (d, *J =* 11.0 Hz, 1H), 2.00 (dd, *J =* 16.0, 8.0 Hz, 2H). UPLC-MS ( ESI ) calculated for C₂₃H₂₁N₄O₆ [M + H ]⁺: 449.14, found 449.35.

### Example 29: N-(4-chlorobenzyl)-2-(2,6-dioxopiperidin-3-yl)-3-oxo-2,3-dihydro-1H-indazole-6-carboxamide (D29)

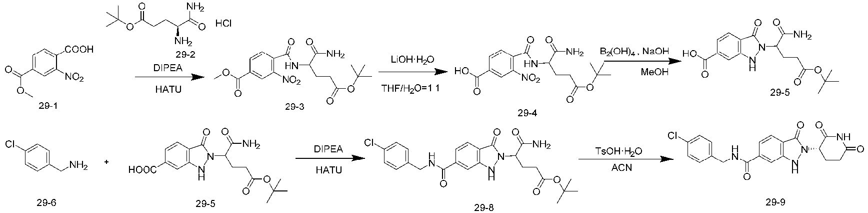

Compound 29-1 (1.0 g, 4.44 mmol) was placed in a round-bottom flask and dissolved in DMF (20 mL). Compound 29-2 (1.57 g, 6.67 mmol) and HATU (5.0 g, 13.32 mmol) were added, and the mixture was stirred for 3 min. DIPEA (5.0 mL, 22.2 mmol) was then added, and the reaction mixture was stirred at room temperature for 4 h. After completion of the reaction, the mixture was diluted with ethyl acetate and washed with saturated sodium chloride solution. The organic layer was dried over anhydrous sodium sulfate. The solvent was removed under reduced pressure, and the residue was purified by silica gel column chromatography to afford compound 29-3 (1.71 g, 94% yield).

### Synthesis of Compound 29-4:

Compound 29-3 (1.71 g, 4.1 mmol) was placed in a round-bottom flask and dissolved in tetrahydrofuran (10 mL). A solution of lithium hydroxide hydrate (0.984 g, 24.6 mmol) in water (10 mL) was added, and the mixture was stirred for 1 h. After completion of the reaction, the solvent was removed under reduced pressure to afford compound 29-4, which was obtained in quantitative yield (100%) and used directly in the next step.

### Synthesis of Compound 29-5:

Compound 29-4 (2.76 g, 6.74 mmol) was placed in a round-bottom flask and dissolved in methanol. The reaction mixture was cooled to 0 °C in an ice bath, and tetrahydroxydiboron (3.025 g, 33.75 mmol) was added, followed by addition of a methanolic solution of sodium hydroxide (2.7 g, 67.5 mmol). After stirring for 10 min, the reaction mixture was heated to 40 °C and stirred for 4 h. Upon completion, the mixture was extracted with ethyl acetate and washed with saturated sodium chloride solution. The organic phase was dried over anhydrous sodium sulfate. The solvent was removed under reduced pressure, and the residue was purified by silica gel column chromatography to afford compound 29-5 (2.4 g, 98% yield).

### Synthesis of Compound 29-8:

Compound 29-8 was prepared from compound 29-5 and compound 29-6 using the same molar equivalents and procedure as described for Compound 3.

### Synthesis of Compound 29:

Compound 29-8 (50 mg, 0.10 mmol) was placed in a round-bottom flask and dissolved in acetonitrile (5 mL). p-toluenesulfonic acid monohydrate (16 mg, 0.061 mmol) was added, and the reaction mixture was heated at 90 °C under reflux for 4 h. After completion of the reaction, the solvent was removed under reduced pressure. The residue was purified by preparative HPLC to afford compound 29 (10 mg) as a white solid (23% yield).¹H NMR (500 MHz, DMSO-*d*₆) δ 9.43 (t, *J* = 6.0 Hz, 1H), 8.75 (s, 1H), 7.96 (d, *J* = 8.1 Hz, 1H), 7.91 (d, *J* = 8.7 Hz, 2H), 7.49 (s, 1H), 7.43 - 7.33 (m, 4H), 5.13 (dd, *J* = 5.4, 3.0 Hz, 1H), 4.50 (d, *J* = 5.9 Hz, 2H), 2.78 (dt, *J* = 17.6, 3.8 Hz, 1H), 2.62 (ddd, *J* = 17.7, 12.6, 5.7 Hz, 1H), 2.47 (dd, *J* = 8.8, 3.9 Hz, 1H), 2.39 (ddq, J = 14.0, 6.0, 2.8 Hz, 1H). UPLC-MS ( ESI ) calculated for C₂₀H₁₈ClN₄O₄ [M + H ]⁺: 413.09, found 413.25.

### Example 30: N-((S)-1-(4-chlorophenyl)ethyl)-2-(2,6-dioxopiperidin-3-yl)-3-oxo-2,3-dihydro-1H-indazole-6-carboxamide (D30)

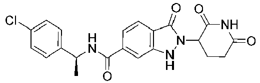

The synthetic method was carried out with reference to Example 29 to afford compound **D30.** ¹H NMR (500 MHz, DMSO-*d*₆) δ 11.06 (s, 1H), 10.25 (s, 1H), 9.01 (d, *J* = 7.8 Hz, 1H), 7.78 (d, *J* = 8.1 Hz, 1H), 7.70 (s, 1H), 7.62 (dd, *J =* 8.1, 1.4 Hz, 1H), 7.41 (q, *J* = 8.7 Hz, 4H), 5.39 (dd, *J =* 13.1, 5.2 Hz, 1H), 5.16 (p, *J* = 7.2 Hz, 1H), 2.98 - 2.92 (m, 1H), 2.69 - 2.64 (m, 1H), 2.46 - 2.38 (m, 1H), 2.14 - 2.09 (m, 1H), 1.48 (d, *J* = 7.1 Hz, 3H). UPLC-MS ( ESI ) calculated for C₂₁H₂₀ClN₄O₄ [M + H ]⁺: 427.11, found 427.26.

### Example 31: N-((S)-1-(4-chlorophenyl)ethyl)-2-(2,6-dioxopiperidin-3-yl)-3-oxo-2,3-dihydro-1H-indazole-6-carboxamide (D31)

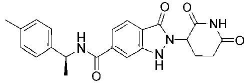

The synthetic method was carried out with reference to Example 29 to afford compound **D31.** ¹H NMR (500 MHz, DMSO-*d*₆) δ 11.06 (s, 1H), 8.94 (d, *J =* 8.0 Hz, 1H), 7.77 (d, *J =* 8.1 Hz, 1H), 7.70 (s, 1H), 7.64 - 7.55 (m, 1H), 7.29 (d, *J =* 7.9 Hz, 2H), 7.16 - 7.10 (m, 2H), 5.39 (dd, *J =* 13.1, 5.2 Hz, 1H), 5.13 (h, *J* = 8.1, 7.6 Hz, 1H), 3.70 (s, 3H), 2.95 (ddd, J = 17.3, 13.6, 5.4 Hz, 1H), 2.69 - 2.62 (m, 1H), 2.49 - 2.37 (m, 2H), 2.28 (s, 3H), 2.12 (dtt, *J =* 12.8, 5.4, 3.3 Hz, 1H), 1.46 (dd, *J =* 11.6, 7.0 Hz, 3H). UPLC-MS ( ESI ) calculated for C₂₂H₂₃N₄O₄ [M + H ]⁺: 407.16, found 407.33.

### Example 32: 2-(2,6-dioxopiperidin-3-yl)-3-oxo-N-((R)-1-phenylethyl)-2,3-dihydro-1H-indazole-6-carboxamide (D32)

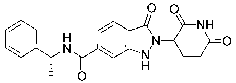

The synthetic method was carried out with reference to Example 29 to afford compound **D32.** ¹H NMR (500 MHz, DMSO-*d*₆) δ 11.07 (s, 1H), 8.99 (d, *J* = 8.0 Hz, 1H), 7.78 (d, *J =* 8.1 Hz, 1H), 7.71 (t, *J* = 1.0 Hz, 1H), 7.62 (dd, *J* = 8.1, 1.4 Hz, 1H), 7.44 - 7.36 (m, 2H), 7.36 - 7.29 (m, 2H), 7.27 - 7.19 (m, 1H), 5.39 (dd, *J* = 13.1, 5.2 Hz, 1H), 5.18 (p, *J* = 7.3 Hz, 1H), 2.95 (ddd, *J* = 17.2, 13.7, 5.4 Hz, 1H), 2.65 (dt, *J* = 17.1, 3.9 Hz, 1H), 2.43 (qd, *J* = 13.3, 4.6 Hz, 1H), 2.12 (dtd, *J* = 12.9, 5.4, 2.5 Hz, 1H), 1.50 (d, *J* = 7.1 Hz, 3H). UPLC-MS ( ESI ) calculated for C₂₁H₂₁N₄O₄ [M + H ]⁺: 393.15, found 393.32

### Example 33: 2-(2,6-dioxopiperidin-3-yl)-3-oxo-N-((S)-1-phenylethyl)-2,3-dihydro-1H-indazole-6-carboxamide (D33)

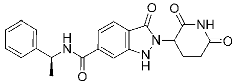

The synthetic method was carried out with reference to Example 29 to afford compound **D33.** ¹H NMR (500 MHz, DMSO-*d*₆) δ 11.06 (s, 1H), 10.24 (s, 1H), 8.99 (d, *J* = 8.0 Hz, 1H), 7.77 (dd, *J* = 8.1, 0.7 Hz, 1H), 7.71 (t, *J =* 1.1 Hz, 1H), 7.62 (dd, *J* = 8.2, 1.4 Hz, 1H), 7.44 - 7.38 (m, 2H), 7.37 - 7.31 (m, 2H), 7.27 - 7.20 (m, 1H), 5.39 (dd, *J* = 13.2, 5.2 Hz, 1H), 5.18 (p, *J* = 7.2 Hz, 1H), 2.95 (ddd, *J* = 17.1, 13.6, 5.4 Hz, 1H), 2.69 - 2.62 (m, 1H), 2.43 (qd, *J =* 13.2, 4.5 Hz, 1H), 1.50 (d, *J =* 7.1 Hz, 3H). UPLC-MS ( ESI ) calculated for C₂₁H₂₁N₄O₄ [M + H ]⁺: 393.15, found 393.32

### Example 34: N-((R)-1-(4-chlorophenyl)ethyl)-2-(2,6-dioxopiperidin-3-yl)-3-oxo-2,3-dihydro-1H-indazole-6-carboxamide (D34)

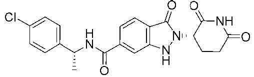

The synthetic method was carried out with reference to Example 29 to afford compound **D34.** ¹H NMR (500 MHz, DMSO-*d*₆) δ 11.07 (s, 1H), 10.25 (s, 1H), 9.01 (d, *J* = 7.9 Hz, 1H), 7.89 - 7.58 (m, 3H), 7.41 (qd, *J =* 8.5, 1.5 Hz, 4H), 5.39 (dd, *J =* 13.1, 5.2 Hz, 1H), 5.17 (pd, *J* = 7.2, 3.0 Hz, 1H), 2.95 (ddd, *J* = 17.3, 13.7, 5.4 Hz, 1H), 2.71 - 2.61 (m, 1H), 2.41 (td, *J* = 13.3, 4.6 Hz, 1H), 1.48 (d, *J* = 7.1 Hz, 3H). UPLC-MS ( ESI ) calculated for C₂₁H₂₀ClN₄O₄ [M + H ]⁺: 427.11, found 427.37

### Example 35: 2-(2,6-dioxopiperidin-3-yl)-3-oxo-N-((S)-2,2,2-trifluoro-1-phenylethyl)-2,3-dihydro-1H-indazole-6-carboxamide (D35)

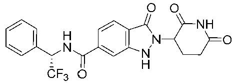

The synthetic method was carried out with reference to Example 29 to afford compound **D35.** UPLC-MS ( ESI ) calculated for C₂₁H₂₀ClN₄O₄ [M + H ]⁺: 447.12, found 447.30.

### Example 36: 2-(2,6-dioxopiperidin-3-yl)-N-(4-methylbenzyl)-3-oxo-2,3-dihydro-1H-indazole-6-carboxamide (D36)

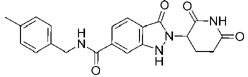

The synthetic method was carried out with reference to Example 29 to afford compound **D36.** ¹H NMR (500 MHz, DMSO-*d*₆) δ 9.34 (t, *J* = 6.0 Hz, 1H), 8.77 - 8.72 (m, 1H), 7.95 (d, *J* = 8.1 Hz, 1H), 7.90 (dd, *J* = 8.2, 1.4 Hz, 1H), 7.87 (s, 1H), 7.53 - 7.45 (m, 1H), 7.25 - 7.20 (m, 2H), 7.18 - 7.10 (m, 2H), 5.12 (dd, *J* = 5.4, 3.0 Hz, 1H), 4.47 (d, *J* = 6.0 Hz, 2H), 2.89 (s, 1H), 2.78 (dt, *J =* 17.5, 3.9 Hz, 1H), 2.65 - 2.59 (m, 1H), 2.40 (dt, *J =* 6.1, 2.8 Hz, 1H), 2.28 (s, 3H). UPLC-MS ( ESI ) calculated for C₂₁H₂₁N₄O₄ [M + H ]⁺: 393.15, found 393.15.

### Example 37: 2-(2,6-dioxopiperidin-3-yl)-3-oxo-N-((R)-2,2,2-trifluoro-1-phenylethyl)-2,3-dihydro-1H-indazole-6-carboxamide (D37)

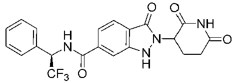

The synthetic method was carried out with reference to Example 29 to afford compound **D37.** ¹H NMR (500 MHz, DMSO-*d*₆) δ 11.07 (s, 1H), 10.32 (s, 1H), 9.71 (s, 1H), 7.71 (d, *J =* 7.2 Hz, 4H), 7.45 (q, *J* = 7.8, 6.8 Hz, 6H), 6.07 (s, 1H), 5.41 (s, 1H), 3.69 (s, 5H), 2.95 (s, 2H), 2.13 (s, 2H). UPLC-MS ( ESI ) calculated for C₂₁H₁₈F₃N₄O₄ [M + H ]⁺: 447.12, found 447.34.

### Example 38: N-((S)-1-(2,4-difluorophenyl)ethyl)-2-(2,6-dioxopiperidin-3-yl)-3-oxo-2,3-dihydro-1H-indazole-6-carboxamide (D38)

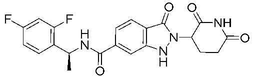

The synthetic method was carried out with reference to Example 29 to afford compound **D38.** ¹H NMR (500 MHz, DMSO-*d*₆) δ 9.25 (dd, *J =* 12.4, 7.5 Hz, 1H), 8.70 (dd, *J =* 3.8, 1.2 Hz, 1H), 8.01 - 7.85 (m, 2H), 7.62 - 7.43 (m, 2H), 7.22 (dddd, *J =* 15.4, 12.3, 6.2, 2.5 Hz, 1H), 7.09 (tt, *J =* 8.7, 2.7 Hz, 1H), 5.43 - 5.29 (m, 2H), 2.88 (dt, *J =* 17.0, 5.1 Hz, 1H), 2.76 (ddd, J = 17.3, 10.3, 4.5 Hz, 1H), 2.66 - 2.58 (m, 1H), 2.41 - 2.36 (m, 1H), 1.50 (dd, *J =* 7.1, 3.8 Hz, 3H). UPLC-MS ( ESI ) calculated for C₂₁H₁₉F₂N₄0₄ [M + H ]⁺: 429.13, found 429.31.

### Example 39: N-((S)-1-(3-chlorophenyl)ethyl)-2-(2,6-dioxopiperidin-3-yl)-3-oxo-2,3-dihydro-1H-indazole-6-carboxamide (D39)

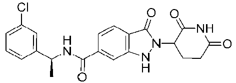

The synthetic method was carried out with reference to Example 29 to afford compound **D39.** ¹H NMR (500 MHz, DMSO-*d*₆) δ 9.20 (d, *J =* 7.8 Hz, 1H), 8.71 (s, 1H), 7.97 (d, *J =* 8.1 Hz, 1H), 7.91 (dd, *J =* 8.1, 1.4 Hz, 1H), 7.87 (s, 1H), 7.48 (d, *J =* 4.6 Hz, 2H), 7.38 (d, *J =* 4.8 Hz, 2H), 7.32 (qd, *J =* 4.2, 2.0 Hz, 1H), 5.19 (p, *J =* 7.2 Hz, 1H), 5.13 (dd, *J =* 5.5, 2.9 Hz, 1H), 3.06 (s, 1H), 2.78 (dt, *J* = 17.4, 3.8 Hz, 1H), 2.62 (dd, *J* = 11.4, 6.4 Hz, 1H), 2.40 (dt, *J* = 6.9, 3.5 Hz, 1H), 1.50 (d, *J* = 7.0 Hz, 3H). UPLC-MS ( ESI ) calculated for C₂₁H₂₀ClN₄O₄ [M + H ]⁺: 427.11, found 427.33.

### Example 40: N-(tert-butyl)-2-(2,6-dioxopiperidin-3-yl)-3-oxo-2,3-dihydro-1H-indazole-6-carboxamide (D40)

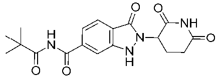

The synthetic method was carried out with reference to Example 29 to afford compound **D40**. ¹H NMR (500 MHz, DMSO-*d*₆) δ 10.33 (s, 1H), 8.63 - 8.56 (m, 1H), 8.13 (d, *J* = 13.6 Hz, 1H), 7.95 - 7.87 (m, 1H), 7.90 - 7.81 (m, 1H), 5.31 (t, *J =* 5.4 Hz, 1H), 2.77 (q, *J =* 5.7 Hz, 1H), 2.69 - 2.58 (m, 1H), 2.41 - 2.30 (m, 1H), 2.29 - 2.16 (m, 1H), 1.43 - 1.34 (m, 9H). UPLC-MS ( ESI ) calculated for C₁₇H₂₁N₄O₄ [M + H ]⁺: 345.15, found 345.38.

### Example 41: N-((R)-6-chlorochroman-4-yl)-2-(2,6-dioxopiperidin-3-yl)-3-oxo-2,3-dihydro-1H-indazole-6-carboxamide (D41)

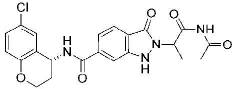

The synthetic method was carried out with reference to Example 29 to afford compound **D41.** ¹H NMR (500 MHz, DMSO-*d*₆) δ 10.87 (s, 1H), 9.28 (dd, *J =* 12.8, 8.0 Hz, 1H), 8.77 (t, *J* = 1.7 Hz, 1H), 8.03 - 7.80 (m, 2H), 7.38 - 7.11 (m, 2H), 6.94 - 6.71 (m, 1H), 5.31 (q, *J =* 8.4, 7.5 Hz, 1H), 4.30 (qdd, *J* = 10.7, 6.8, 3.3 Hz, 2H), 2.89 (dt, *J =* 17.0, 5.1 Hz, 1H), 2.77 (ddd, *J* = 12.5, 9.1, 4.6 Hz, 1H), 2.70 - 2.56 (m, 1H), 2.43 - 2.34 (m, 1H), 2.18 - 2.06 (m, 2H). UPLC-MS ( ESI ) calculated for C₂₂H₂₀ClN₄O₅ [M + H ]⁺: 455.10, found 455.29.

### Example 42: 2-(2,6-dioxopiperidin-3-yl)-N-((R)-8-methylchroman-4-yl)-3-oxo-2,3-dihydro-1H-indazole-6-carboxamide (D42)

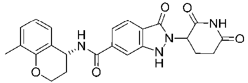

The synthetic method was carried out with reference to Example 29 to afford compound **D42.** ¹H NMR (500 MHz, DMSO-*d*₆) δ 9.24 (dd, *J* = 12.0, 8.1 Hz, 1H), 8.76 (s, 1H), 8.01 - 7.84 (m, 2H), 7.09 - 6.98 (m, 2H), 6.79 (dd, *J* = 8.0, 6.3 Hz, 1H), 5.30 (q, *J* = 6.8 Hz, 1H), 5.13 (dd, *J* = 5.4, 2.9 Hz, 1H), 4.33 (dddd, *J* = 33.2, 10.7, 7.3, 3.3 Hz, 2H), 2.92 - 2.83 (m, 1H), 2.77 (dt, *J =* 17.4, 4.3 Hz, 1H), 2.61 (dd, *J =* 11.3, 6.5 Hz, 1H), 2.40 (dt, *J =* 6.2, 3.0 Hz, 1H), 2.18 - 2.05 (m, 5H). UPLC-MS ( ESI ) calculated for C₂₃H₂₃N₄O₅ [M + H ]⁺: 435.16, found 435.29.

### Example 43: N-butyl-2-(2,6-dioxopiperidin-3-yl)-3-oxo-2,3-dihydro-1H-indazole-6-carboxamide (D43)

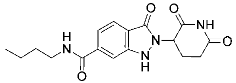

The synthetic method was carried out with reference to Example 29 to afford compound **D43**. ¹H NMR (500 MHz, DMSO-*d*₆) δ 8.77 (t, *J =* 5.7 Hz, 1H), 8.69 (t, *J =* 1.0 Hz, 1H), 7.95 - 7.91 (m, 1H), 7.90 - 7.83 (m, 2H), 7.49 (s, 1H), 5.12 (dd, *J* = 5.4, 3.0 Hz, 1H), 3.31 - 3.28 (m, 2H), 2.78 (dd, *J =* 17.4, 4.0 Hz, 1H), 2.65 - 2.59 (m, 1H), 2.49 - 2.43 (m, 1H), 2.41 - 2.36 (m, 1H), 1.54 (tt, *J* = 7.8, 6.3 Hz, 3H), 1.39 - 1.28 (m, 3H), 0.92 (t, *J* = 7.3 Hz, 4H). UPLC-MS ( ESI ) calculated for C₁₇H₂₁N₄O₄ [M + H ]⁺: 345.15, found 345.41.

### Example 44: (S)-2-(4-chlorophenyl)-N-((2-(2,6-dioxopiperidin-3-yl)-3-oxo-2,3-dihydro-1H-indazol-6-yl)methyl)-2,2-difluoroacetamide (D44)

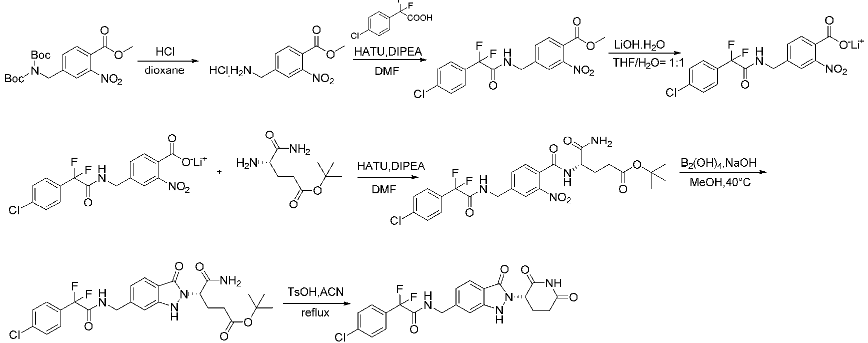

### Step 1:

Methyl 4-((bis(tert-butoxycarbonyl)amino)methyl)-2-nitrobenzoate (300 mg, 0.73 mmol) was dissolved in dichloromethane (2 mL). A 4 mol/L hydrogen chloride solution in 1,4-dioxane (6 mL) was slowly added, and the reaction mixture was stirred at room temperature for 12 h. After completion of the reaction, the solvent was removed under reduced pressure, and the residue was used directly in the next step without further purification.

### Step 2:

Methyl 4-(aminomethyl)-2-nitrobenzoate hydrochloride (246 mg, 1 mmol), 2-(4-chlorophenyl)-2,2-difluoroacetic acid (227 mg, 1.1 mmol), and N,N,N',N'-tetramethyl-O-(7-azabenzotriazol-1-yl)uronium hexafluorophosphate (HATU, 418 mg, 1.1 mmol) were added to N,N-dimethylformamide (10 mL). N,N-diisopropylethylamine (1.39 mL, 8 mmol) was added, and the mixture was stirred at room temperature for 12 h. The reaction mixture was diluted with ethyl acetate, washed with water and saturated sodium chloride solution, and dried over anhydrous sodium sulfate. After filtration and concentration under reduced pressure, the residue was purified by column chromatography to give the product (100 mg) as a colorless oil in 25% yield. UPLC-MS ( ESI ) calculated for C₁₇H₁₃ClF₂N₂O₅ [M + H ]⁺: 399.75, found 399.66.

### Step 3:

Methyl 4-((2-(4-chlorophenyl)-2,2-difluoroacetamido)methyl)-2-nitrobenzoate (100 mg, 0.25 mmol) was dissolved in tetrahydrofuran (3 mL). An aqueous solution (3 mL) of lithium hydroxide monohydrate (16 mg, 0.376 mmol) was added, and the mixture was stirred at room temperature overnight. The reaction mixture was concentrated under reduced pressure to give the product (97 mg), which was used directly in the next step without purification.

### Step 4:

Lithium 4-((2-(4-chlorophenyl)-2,2-difluoroacetamido)methyl)-2-nitrobenzoate (97 mg), (S)-tert-butyl 4,5-diamino-5-oxopentanoate hydrochloride (60 mg, 0.25 mmol), and N,N,N',N'-tetramethyl-O-(7-azabenzotriazol-1-yl)uronium hexafluorophosphate (95 mg, 0.25 mmol) were added to N,N-dimethylformamide (3 mL). N,N-diisopropylethylamine (0.131 mL, 0.75 mmol) was added dropwise and the mixture was stirred at room temperature for 12 h. The reaction mixture was diluted with ethyl acetate, washed with water and saturated sodium chloride solution, and dried over anhydrous sodium sulfate. After filtration and concentration, the residue was purified by column chromatography to afford the product (126 mg) in 89% yield. UPLC-MS (ESI) calculated for C₂₅H₂₇ClF₂N₄O₇ [M + H ]⁺: 568.96, found 568.91.

### Step 5:

(S)-tert-Butyl 5-amino-4-(4-((2-(4-chlorophenyl)-2,2-difluoroacetamido)methyl)-2-nitrobenzamido)-5-oxopentanoate (30 mg, 0.053 mmol) was dissolved in methanol (3 mL). At 0 °C, bis(pinacolato)diboron (24 mg, 0.264 mmol) was added, followed by a methanolic solution (3 mL) of sodium hydroxide (20 mg, 0.5 mmol). The mixture was stirred at 0 °C for 10 min and then heated to 40 °C overnight. After completion as monitored by TLC, the reaction was quenched with saturated ammonium chloride solution and extracted with ethyl acetate. The organic layer was washed with water and saturated sodium chloride solution, dried over anhydrous sodium sulfate, filtered, and concentrated. The residue was purified by column chromatography to give the product (19 mg) in 67% yield. UPLC-MS ( ESI ) calculated for C₂₅H₂₇ClF₂N₄O₅ [M + H ]⁺: 537.96, found 537.88.

### Step 6:

(S)-tert-Butyl 5-amino-4-(6-((2-(4-chlorophenyl)-2,2-difluoroacetamido)methyl)-3-oxo-1,3-dihydro-2H-indazol-2-yl)-5-oxopentanoate (19 mg, 0.035 mmol) and p-toluenesulfonic acid monohydrate (40 mg, 0.212 mmol) were added to acetonitrile (2 mL). The mixture was stirred at 80 °C overnight. The reaction mixture was concentrated and purified by preparative HPLC to afford the product (7 mg) in 42% yield. UPLC-MS ( ESI ) calculated for C₂₁H₁₇ClF₂N₄O₄ [M + H ]⁺: 463.84, found 463.36.¹H NMR (500 MHz, DMSO) δ 10.76 (s, 1H), 9.78 (s, 1H), 9.43 (s, 1H), 8.18 (s, 1H), 7.84 (s, 1H), 7.79 (d, J = 7.9 Hz, 1H), 7.61 (s, 2H), 7.45 (s, 1H), 7.30 (d, J = 9.1 Hz, 1H), 5.08 (d, J = 2.9 Hz, 1H), 4.49 (d, J = 6.1 Hz, 2H), 2.74 (d, J = 17.7 Hz, 1H), 2.64 - 2.57 (m, 2H), 2.36 (d, J = 1.9 Hz, 1H).

### Example 45: (S)-1-(3-Chloro-4-methylphenyl)-3-((2-(2,6-dioxopiperidin-3-yl)-3-oxo-2,3-dihydro-1H-indazol-6-yl)methyl)urea (D45)

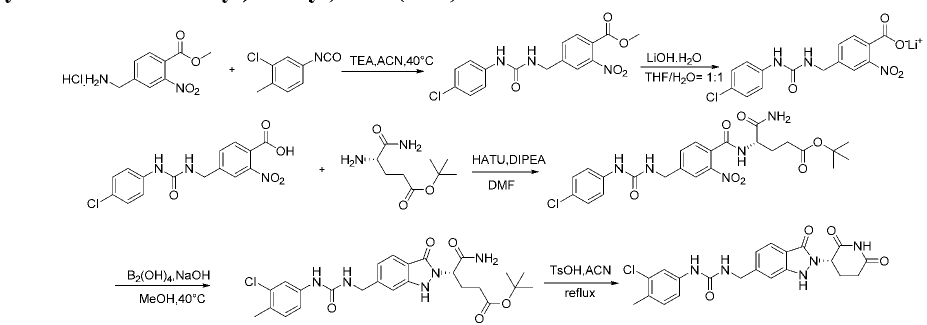

### Step 1:

Methyl 4-(aminomethyl)-2-nitrobenzoate hydrochloride (180 mg, 0.73 mmol) and 3-chloro-4-methylphenyl isocyanate (135 mg, 0.80 mmol) were added to acetonitrile (8 mL). Triethylamine (0.507 mL, 3.65 mmol) was then added, and the reaction mixture was heated to 40 °C for 6 h. After completion of the reaction, the mixture was concentrated under reduced pressure, diluted with water, and extracted with ethyl acetate. The combined organic phases were washed with saturated sodium chloride solution, dried over anhydrous sodium sulfate, filtered, and concentrated. The residue was purified by column chromatography to give the product (247 mg, white solid, 89% yield).UPLC-MS ( ESI ) calculated for C₁₆H₁₄ClN₃O₅ [M + H ]⁺: 364.75, found 364.77.

### Step 2:

The above compound (247 mg, 0.65 mmol) was dissolved in tetrahydrofuran (3 mL). An aqueous solution of lithium hydroxide monohydrate (41 mg, 0.98 mmol in 2 mL water) was added, and the reaction mixture was stirred overnight at room temperature. The reaction mixture was concentrated under reduced pressure and dried to give 241 mg crude product, which was used directly in the next step without purification. The resulting lithium salt of 4-((3-(3-chloro-4-methylphenyl)ureido)methyl)-2-nitrobenzoic acid (241 mg), (S)-tert-butyl 4,5-diamino-5-oxopentanoate hydrochloride (171 mg, 0.72 mmol), and HATU (274 mg, 0.72 mmol) were dissolved in DMF (10 mL). DIPEA (0.34 mL, 1.95 mmol) was added dropwise, and the reaction mixture was stirred at room temperature for 12 h. The mixture was diluted with ethyl acetate, washed with water and saturated NaCl solution, dried over Na₂SO₄, filtered, and concentrated. Column chromatography afforded the product (322 mg, 90% yield).UPLC-MS ( ESI ) calculated for C₂₄H₂₈ClN₅O₇ [M + H ]⁺: 534.97, found 534.89.

### Step 3:

(S)-5-amino-4-(4-((3-(3-chloro-4-methylphenyl)ureido)methyl)-2-nitrobenzamido)-5-oxopentanoate tert-butyl ester (358 mg, 0.67 mmol) was dissolved in methanol (5 mL). At 0 °C, tetrahydroxydiboron (300 mg, 3.35 mmol) was added, followed by a methanolic solution of sodium hydroxide (268 mg, 6.7 mmol in 5 mL methanol). The reaction mixture was stirred 10 min at 0 °C, then warmed to 40 °C and stirred overnight. After completion (TLC monitoring), the reaction was quenched with saturated ammonium chloride solution, extracted with ethyl acetate, and the organic phase was washed with water and brine, dried over Na₂SO₄, filtered, and concentrated. Column chromatography afforded the product (272 mg, 81% yield). UPLC-MS ( ESI ) calculated for C₂₅H₃₀ClN₅O₅[M + H ]⁺: 517.00, found 517.03.

### Step 4:

(S)-5-amino-4-(6-((3-(3-chloro-4-methylphenyl)ureido)methyl)-3-oxo-1,3-dihydro-2H-indazol-2-yl)-5-oxopentanoate tert-butyl ester (272 mg, 0.53 mmol) and p-toluenesulfonic acid monohydrate (601 mg, 3.16 mmol) were dissolved in acetonitrile and heated at 80 °C overnight. The reaction mixture was concentrated and purified by HPLC to afford the final product D45 (60 mg, 26% yield). UPLC-MS ( ESI ) calculated for C₂₁H₂₀ClN₅O₄ [M + H ]⁺: 442.87, found 442.91.

### Example 46: N-Benzyl-2-(2,6-dioxopiperidin-3-yl)-3-oxo-2,3-dihydro-1H-indazol-6-carboxamide (D46)

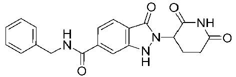

The synthetic method was carried out with reference to Example 29 to afford compound **D46.** ¹H NMR (500 MHz, DMSO) δ 11.06 (s, 1H), 10.26 (s, 1H), 9.21 (t, *J* = 5.9 Hz, 1H), 7.77 (d, *J =* 8.1 Hz, 1H), 7.72 (s, 1H), 7.62 (d, *J =* 8.2 Hz, 1H), 7.33 (d, *J =* 4.7 Hz, 4H), 7.25 (dd, *J* = 8.8, 4.4 Hz, 1H), 5.39 (dd, *J =* 13.1, 5.1 Hz, 1H), 4.50 (d, *J =* 5.9 Hz, 2H), 2.93 (dd, *J* = 22.3, 8.7 Hz, 1H), 2.65 (d, *J =* 12.5 Hz, 1H), 2.40 (dd, *J =* 19.8, 15.6 Hz, 1H), 2.14 - 2.06 (m, 1H). UPLC-MS ( ESI ) calculated for C₂₀H₁₉N₄O₄ [M + H ]⁺: 379.13, found: 379.25.

### Example 47: 2-(2,6-Dioxopiperidin-3-yl)-N-((S)-1-(4-ethylphenyl)ethyl)-3-oxo-2,3-dihydro-1H-indazol-6-carboxamide (D47)

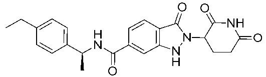

The synthetic method was carried out with reference to Example 29 to afford compound **D47.** ¹H NMR (500 MHz, DMSO) δ 11.06 (s, 1H), 10.22 (s, 1H), 8.93 (d, *J =* 8.0 Hz, 1H), 7.76 (d, *J =* 8.2 Hz, 1H), 7.69 (s, 1H), 7.61 (d, *J =* 8.2 Hz, 1H), 7.30 (d, *J =* 8.1 Hz, 2H), 7.16 (d, *J =* 8.1 Hz, 2H), 5.38 (dd, *J =* 13.1, 5.2 Hz, 1H), 5.14 (p, *J =* 7.1 Hz, 1H), 2.99 - 2.89 (m, 1H), 2.64 (d, *J =* 16.6 Hz, 1H), 2.57 (q, *J =* 7.6 Hz, 2H), 2.41 (qd, *J =* 13.3, 4.5 Hz, 1H), 2.15 - 2.05 (m, 1H), 1.47 (d, *J =* 7.1 Hz, 3H), 1.16 (t, *J =* 7.6 Hz, 3H). UPLC-MS ( ESI ) calculated for C₂₃H₂₅N₄O₄ [M + H ]⁺: 421.18, found: 421.16.

### II. Experimental Examples

The compounds described in the Examples of the present invention were further evaluated for their biological activity against three major hematological tumor cell lines. Representative cell lines include: Multiple myeloma cell line (MM.1S), Mantle cell lymphoma cell line (Mino), Acute myeloid leukemia cell line (MV-4-11). The inhibitory activity of the compounds on the proliferation of these representative cell lines was evaluated. Unless otherwise specified, the experimental materials required for the pharmacological experiments were commercially purchased.

### 1. Effect of Compounds on the Proliferation of MM.1S Cells

MM.1S cells were cultured in RPMI-1640 medium supplemented with 10% fetal bovine serum and collected. The cell concentration was diluted according to a 7-day incubation period, and 180 µL of cell suspension was added to each well of a 96-well plate, with 20,000 cells per well. For the control group, 20 µL of DMSO was added to each control well to give a final concentration of 0.2% DMSO. The test compounds were prepared from a 10 mM stock solution and serially diluted in a 5-fold gradient, and 20 µL of the diluted solution was added to each compound-treated well (final DMSO concentration also 0.2%). The cells were incubated in a 37 °C, 5% CO₂ incubator for 7 days. After incubation, the reaction solution was prepared according to the MTS assay kit instructions (Promega, G5430), and 20 µL was added to each well. The plate was incubated at 37 °C with 5% CO₂ for 3-4 hours. The absorbance at 490 nm was measured using a microplate reader, and the absorbance at 690 nm was used as the background value. The final raw data were calculated as OD490 - OD690.The inhibition rate of the compounds was calculated using the following formula: Inhibition rate= (OD_{DMSO}-OD_{componnd})/(OD_{DMSO}-OD_{blank})×100%. The IC₅₀ values for cell proliferation inhibition were determined using GraphPad Prism 5.0. Each experiment was repeated three times, and three parallel replicates were performed in each experiment to calculate the mean value and standard deviation.

The cell proliferation inhibition results are shown in Table 1, where: A indicates cell activity IC₅₀ < 200 nM; B indicates 200 nM < IC₅₀ < 1 µM; C indicates 1 µM < IC₅₀ < 10 µM; D indicates IC₅₀ > 10 µM

**Table 1. Inhibitory Activity of Compounds on MM.1S Cell Proliferation**

| **Compound No.** | **MM1.S** | **Compound No.** | **MM1.S** |
|---|---|---|---|
| **D1** | **A** | **D25** | **C** |
| **D2** | **A** | **D26** | **C** |
| **D3** | **A** | **D27** | **C** |
| **D4** | **A** | **D28** | **C** |
| **D5** | **A** | **D29** | **D** |
| **D7** | **A** | **D30** | **D** |
| **D8** | **A** | **D31** | **D** |
| **D9** | **A** | **D32** | **D** |
| **D10** | **A** | **D33** | **D** |
| **D11** | **A** | **D34** | **D** |
| **D12** | **A** | **D35** | **D** |
| **D13** | **A** | **D36** | **D** |
| **D14** | **A** | **D37** | **D** |
| **D15** | **A** | **D38** | **D** |
| **D16** | **A** | **D39** | **D** |
| **D17** | **A** | **D40** | **D** |
| **D18** | **A** | **D41** | **D** |
| **D19** | **A** | **D42** | **D** |
| **D20** | **A** | **D44** | **D** |
| **D21** | **A** | **D46** | **D** |
| **D22** | **A** | **D47** | **B** |
| **D23** | **D** | **Lenalidomide** | **A** |
| **D24** | **A** | | |

Based on the results of the above cell proliferation inhibition assays, some compounds of the present invention exhibit excellent inhibitory activity against the growth of multiple myeloma MM.1S cells. Certain compounds demonstrate greater activity than the marketed drugs lenalidomide or pomalidomide. On the other hand, the development of these structurally diverse compounds provides additional structural options for obtaining drug molecules with higher biological activity and improved pharmaceutical properties. Therefore, the compounds of the present invention may be used for the prevention and treatment of diseases associated with regulation of CRBN (CRL4^{CRBN} E3 ubiquitin ligase) activity, such as multiple myeloma, and without limitation may also include other potential tumor diseases, pain, neurological diseases, and immune system diseases.

### 2. Inhibitory Activity of Compounds on Mino Cell Proliferation

Mino cells were cultured in RPMI-1640 medium supplemented with 15% fetal bovine serum and collected. The cell concentration was diluted according to a 3-day incubation period, and 90 µL of cell suspension was added to each well of a 96-well plate, resulting in 8,000 cells per well. For the control wells, 10 µL of DMSO was added to give a final concentration of 0.2% DMSO. The test compounds were prepared from a 10 mM stock solution and serially diluted in a five-fold gradient. Similarly, 10 µL of the diluted compound solution was added to each compound-treated well (final DMSO concentration 0.2%).

The cells were incubated in a 37 °C, 5% CO₂ incubator for 3 days. After incubation, the reaction solution was prepared according to the MTS assay kit instructions (Promega, G5430), and 20 µL was added to each well. The plate was further incubated at 37 °C with 5% CO₂ for 3-4 hours. The absorbance at 490 nm was measured using a microplate reader, and the absorbance at 690 nm was used as the background value. The final raw data were calculated as OD490 - OD690. The inhibition rate of the compounds was calculated using the following formula: Inhibition rate= (OD_{DMSO}-OD_{componnd})/(OD_{DMSO}-OD_{blank})×100%. The IC₅₀ values for cell proliferation inhibition were determined using GraphPad Prism 5.0. Each experiment was repeated three times, and three parallel replicates were performed in each experiment to calculate the mean value and standard deviation.

The cell activity test results are shown in Table 2, where: A indicates IC₅₀ < 200 nM; B indicates 200 nM < IC₅₀ < 1 µM; C indicates 1 µM < IC₅₀ < 10 µM; D indicates IC₅₀ > 10 µM.

**Table 2. Inhibitory Activity of Compounds on Mino Cell Proliferation**

| **Compound No.** | **Mino** | **Compound No.** | **Mino** |
|---|---|---|---|
| **D1** | **C** | **D25** | **D** |
| **D2** | **A** | **D26** | **C** |
| **D3** | **A** | **D27** | **D** |
| **D4** | **A** | **D28** | **D** |
| **D5** | **A** | **D29** | **D** |
| **D7** | **A** | **D30** | **C** |
| **D8** | **A** | **D31** | **D** |
| **D9** | **A** | **D32** | **D** |
| **D10** | **A** | **D33** | **D** |
| **D11** | **B** | **D34** | **D** |
| **D12** | **B** | **D35** | **D** |
| **D13** | **C** | **D36** | **D** |
| **D14** | **A** | **D37** | **D** |
| **D15** | **A** | **D38** | **D** |
| **D16** | **A** | **D39** | **D** |
| **D17** | **A** | **D40** | **D** |
| **D18** | **A** | **D41** | **D** |
| **D19** | **A** | **D42** | **D** |
| **D20** | **B** | **D44** | **D** |
| **D21** | **B** | **D46** | **D** |
| **D22** | **D** | **D47** | **A** |
| **D23** | **D** | **Lenalidomide** | **D** |
| **D24** | **A** | | |

Based on the cell proliferation inhibition assay results of certain example compounds described above, it was found that some compounds of the present invention exhibit excellent inhibitory activity against the growth of mantle cell lymphoma Mino cells, and the activity of the vast majority of compounds is superior to that of the marketed drugs lenalidomide or pomalidomide. On the other hand, the development of these structurally diverse compounds provides an expanded material basis and molecular sources for obtaining drug molecules with higher biological activity and improved pharmaceutical properties. Therefore, the compounds of the present invention expand the application scope of IMiD-type drugs in the treatment of hematologic malignancies and may be further extended to other hematological tumor indications. For example, the compounds may serve as active agents for mantle cell lymphoma and may be used in the preparation of medicaments or diagnostic reagents for preventing or treating such diseases. Accordingly, the compounds of the present invention can serve as potent and novel CRBN modulators, useful for the prevention and treatment of diseases associated with the regulation of CRBN (CRL4^{CRBN} E3 ubiquitin ligase) activity, such as multiple myeloma, mantle cell lymphoma, and, without limitation, other potential tumor diseases, pain, neurological diseases, and immune system diseases.

### 3. Inhibitory Activity of Compounds on MV-4-11 Cell Proliferation

MV-4-11 cells were cultured in IMDM medium supplemented with 10% fetal bovine serum and collected. The cell concentration was diluted according to a 7-day incubation period, and 180 µL of cell suspension was added to each well of a 96-well plate, resulting in 2,000 cells per well. For the control wells, 20 µL of DMSO was added to give a final concentration of 0.2% DMSO. The test compounds were prepared from a 10 mM stock solution and serially diluted in a five-fold gradient, and 20 µL of the diluted compound solution was added to each compound-treated well (final DMSO concentration 0.2%). The cells were incubated in a 37 °C, 5% CO₂ incubator for 7 days. After incubation, the reaction solution was prepared according to the MTS assay kit instructions (Promega, G5430), and 20 µL was added to each well. The plate was further incubated at 37 °C with 5% CO₂ for 3-4 hours. The absorbance at 490 nm was measured using a microplate reader, and the absorbance at 690 nm was used as the background value. The final raw data were calculated as OD490 - OD690. The inhibition rate of the compounds was calculated using the following formula: Inhibition rate= (OD_{DMSO}-OD_{componnd})/(OD_{DMSO}-OD_{blank})×100%. The IC₅₀ values for cell proliferation inhibition were determined using GraphPad Prism 5.0. Each experiment was repeated three times, and three parallel replicates were performed in each experiment to calculate the mean value and standard deviation.

The cell activity test results are shown in Table 3, where: A indicates IC₅₀ < 200 nM; B indicates 200 nM < IC₅₀ < 1 µM; C indicates 1 µM ≤ IC₅₀ ≤ 20 µM; D indicates IC₅₀ > 20 µM.

**Table 3. Inhibitory Activity of Compounds on MV-4-11 Cell Proliferation**

| **Compound No.** | **MV-411** | **Compound No.** | **MV-411** |
|---|---|---|---|
| **D1** | **D** | **D25** | **C** |
| **D2** | **A** | **D26** | **C** |
| **D3** | **B** | **D27** | **D** |
| **D4** | **C** | **D28** | **D** |
| **D5** | **C** | **D29** | **D** |
| **D7** | **A** | **D30** | **C** |
| **D8** | **A** | **D31** | **D** |
| **D9** | **A** | **D32** | **D** |
| **D10** | **A** | **D33** | **D** |
| **D11** | **A** | **D34** | **D** |
| **D12** | **A** | **D35** | **D** |
| **D13** | **B** | **D36** | **D** |
| **D14** | **A** | **D37** | **D** |
| **D15** | **A** | **D38** | **D** |
| **D16** | **A** | **D39** | **D** |
| **D17** | **A** | **D40** | **D** |
| **D18** | **A** | **D41** | **D** |
| **D19** | **A** | **D42** | **D** |
| **D20** | **B** | **D44** | **C** |
| **D21** | **A** | **D46** | **D** |
| **D22** | **D** | **D47** | **B** |
| **D23** | **D** | **Lenalidomide** | **D** |
| **D24** | **D** | | |

Based on the cell proliferation inhibition assay results obtained in the acute myeloid leukemia cell line (MV-4-11) for some of the example compounds described above, it was found that several compounds of the present invention exhibit excellent inhibitory activity against MV-4-11 leukemia cells. The IC₅₀ values of certain compounds fall within the nanomolar range, and the most active compounds listed in the table exhibit IC₅₀ values of < 100 nM. In contrast, the reference compounds, including the marketed drugs lenalidomide and pomalidomide, as well as the clinical-stage compound CC-220, all show cell growth inhibitory activity (IC₅₀) greater than 20 µM in MV-4-11 cells. Accordingly, in the compounds of the present invention, the IMiD parent scaffold was modified into an indazolone scaffold, resulting in unexpectedly improved activity in the acute myeloid leukemia cell line (MV-4-11). The compounds demonstrate significantly superior cellular activity compared with marketed drugs and compounds currently under clinical investigation.

### 4. CRBN/DDB1 binding TR-FRET assay

### I. Experimental Procedure

1. 0.1 µL of the reference solution was added to a 384-well assay plate. The final detection concentration was 100 µM, followed by 3-fold serial dilution for 10 points.
2. 2.5 µL of CRBN/DDB1 was added to the 384-well plate and centrifuged at 1000 rpm for 1 minute.
3. 2.5 µL of Thalidomide-BODIPY was added to the plate, centrifuged at 1000 rpm for 1 minute, and incubated at 25 °C for 15 minutes.
4. 5 µL of His-Tb was added to the plate, centrifuged at 1000 rpm for 1 minute, and incubated at 25 °C for 60 minutes.
5. The TR-FRET signal was measured using a BMG plate reader with a fluorescence ratio of 520/490 nm.

The inhibition rates of the compounds on CRBN/DDB 1 binding are shown in Table 4, where: A indicates inhibition rate > 90%; B indicates 50% < inhibition rate < 90%; C indicates inhibition rate < 50%.

**Table 4. Inhibition Rate of Compounds on CRBN/DDB1 Binding**

| **Compound No.** | **AVE_Inhibition% (1 µM)** | **Compound No.** | **AVE_Inhibition% (1 µM)** |
|---|---|---|---|
| **D1** | **B** | **D18** | **A** |
| **D2** | **A** | **D19** | **A** |
| **D4** | **A** | **D23** | **C** |
| **D5** | **B** | **D25** | **C** |
| **D7** | **A** | **D40** | **C** |
| **D8** | **A** | **D44** | **C** |
| **D9** | **A** | **D46** | **C** |
| **D10** | **A** | **D47** | **C** |
| **D14** | **A** | **Lenalidomide** | **B** |
| **D15** | **B** | | |

Based on the test results of the inhibition rates of certain example compounds on CRBN/DDB1 binding at a concentration of 10 µM, it was found that most of the compounds of the present invention exhibit strong inhibitory activity against CRBN/DDB1 binding. Moreover, the inhibitory activity of most compounds is superior to or comparable with that of lenalidomide.

All references mentioned in the present invention are incorporated herein by reference, as if each individual reference were specifically and individually incorporated herein. Furthermore, it should be understood that, after reading the foregoing description of the present invention, those skilled in the art may make various changes or modifications to the invention. Such equivalent forms or modifications are also intended to fall within the scope of the appended claims of the present application.

## Claims

1. A compound of Formula (I), or a pharmaceutically acceptable salt, tautomer, stereoisomer, isotopically labeled compound, pharmaceutically acceptable ester, prodrug, or hydrate thereof: 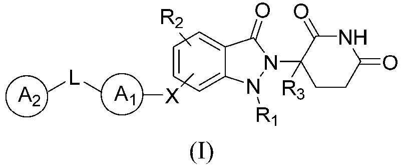 wherein:
R₁ is hydrogen, methyl, or ethyl;
R₂ is hydrogen or fluorine;
R₃ is hydrogen, deuterium, or fluorine;
L is absent, -CH₂-, S, -CO-, or -CH(CH₃)-;
X-A₁ is selected from -(CH₂)ₙ-O-CH₂-A₁, -(CH₂)ₙ-NHCONH-A₁, -(CH₂)ₙ-NHCOCF₂-A₁, - CONH-(CH₂)ₙ-A₁, -CONH-CO-A₁, -CONHCH(CH₃)-A₁, and -CONHCH(CF₃)-A₁;
n is 0 or 1;
when X-A₁ is -(CH₂)ₙ-O-CH₂-A₁, L is absent, -CH₂-, S, -CO-, or -CH(CH₃)-;
A₁ is selected from halogen-substituted or unsubstituted phenyl, halogen-substituted or unsubstituted pyridyl, and a five-membered heteroaryl containing N, O, or S;
A₂ is selected from phenyl, pyridyl, piperazinyl, piperidinyl, morpholinyl, and azetidinyl; wherein the phenyl, pyridyl, piperazinyl, piperidinyl, and morpholinyl are optionally substituted with one or more substituents selected from hydrogen, deuterium, halogen, cyano, nitro, hydroxy, carboxy, aminocarbonyl, 4-10 membered heterocyclyl, C₁-C₆ alkyl, C₁-C₆ alkoxy, halogen-substituted C₁-C₆ alkyl, halogen-substituted C₁-C₆ alkoxy, substituted or unsubstituted phenyl, substituted or unsubstituted pyridyl, C₃-C₈ heterocyclyloxy, C₃-C₈ cycloalkyloxy, hydroxy-substituted C₁-C₆ alkoxy, and C₁-C₆ alkoxy-substituted C₁-C₆ alkoxy;
wherein the substituted or unsubstituted phenyl and substituted or unsubstituted pyridyl are optionally substituted with one or more substituents selected from hydrogen, halogen, cyano, 4-10 membered heterocyclyl, C₁-C₆ alkyl, C₁-C₆ alkoxy, halogen-substituted C₁-C₆ alkyl, and halogen-substituted C₁-C₆ alkoxy;
when X-A₁ is -(CH₂)n-NHCONH-A₁ or -(CH₂)n-NHCOCF₂-A₁, L and A₂ are both absent;
A₁ is phenyl, wherein the phenyl is optionally substituted with one or more substituents selected from hydrogen, halogen, cyano, and C₁-C₆ alkyl;
when X-A₁ is -CONHCH(CH₃)-A₁, -CONH-(CH₂)n-A₁, -CONH-CO-A₁, or -CONHCH(CF₃)-A₁, L and A₂ are both absent;
A₁ is selected from phenyl, pyridyl, 5-10 membered heteroaryl, C₃-C₁₀ cycloalkyl, 4-6 membered heterocyclyl, benzo-fused 4-6 membered heterocyclyl, and C₁-C₆ alkyl; wherein the phenyl, pyridyl, 5-10 membered heteroaryl, C₃-C₁₀ cycloalkyl, 4-6 membered heterocyclyl, and C₁-C₆ alkyl are optionally substituted with one or more substituents selected from hydrogen, deuterium, halogen, cyano, 4-10 membered heterocyclyl, C₁-C₆ alkyl, C₁-C₆ alkoxy, halogen-substituted C₁-C₆ alkyl, and halogen-substituted C₁-C₆ alkoxy.

2. The compound according to claim 1, or a pharmaceutically acceptable salt, tautomer, stereoisomer, isotopically labeled compound, pharmaceutically acceptable ester, prodrug, or hydrate thereof, wherein the compound has a structure represented by formulae (I-1)-(I-11): 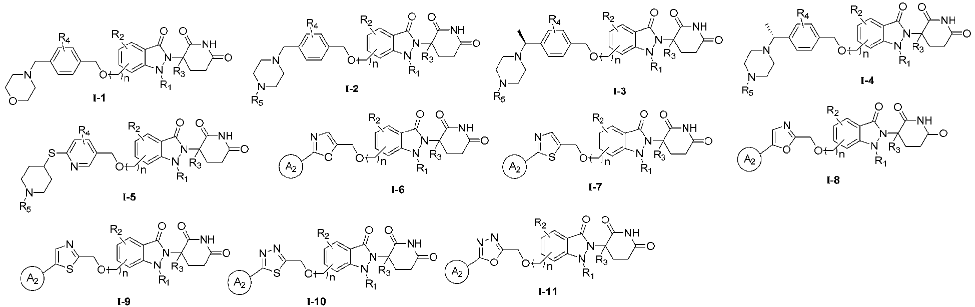 wherein:
R₁ is hydrogen, methyl or ethyl;
R₂ is hydrogen or fluorine;
R₃ is hydrogen, deuterium or fluorine;
R₄ is H or halogen;
n is 0 or 1;
R₅ is selected from phenyl, pyridinyl or morpholinyl, wherein the phenyl, pyridinyl or morpholinyl is optionally substituted with one or more groups selected from hydrogen, halogen, cyano, a 4-10 membered heterocyclic group, C₁-C₆ alkyl, C₁-C₆ alkoxy, halogen-substituted C₁-C₆ alkyl, and halogen-substituted C₁-C₆ alkoxy;
A₂ is selected from phenyl or pyridinyl; wherein the phenyl or pyridinyl is optionally substituted with one or more groups selected from hydrogen, deuterium, halogen, cyano, a 4-10 membered heterocyclic group, C₁-C₆ alkyl, C₁-C₆ alkoxy, halogen-substituted C₁-C₆ alkyl, halogen-substituted C₁-C₆ alkoxy, C₃-C₈ heterocycloalkoxy, C₃-C₈ cycloalkoxy, hydroxy-substituted C₁-C₆ alkoxy, and C₁-C₆ alkoxy-substituted C₁-C₆ alkoxy.

3. The compound according to claim 1, or a pharmaceutically acceptable salt, tautomer, stereoisomer, isotopic derivative, ester, prodrug, or hydrate thereof, wherein the compound has a structure represented by formulae (I-29)-(I-30): 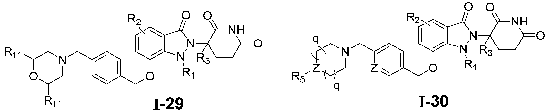 wherein:
R₁ is hydrogen, methyl or ethyl;
R₂ is hydrogen or fluorine;
R₃ is hydrogen, deuterium or fluorine;
R₁₁ is hydrogen or methyl;
each q is independently 0 or 1;
Z is N or -CH-;
R₅ is selected from phenyl, pyridyl or morpholinyl, wherein the phenyl, pyridyl or morpholinyl is optionally substituted with one or more groups selected from hydrogen, halogen, cyano, a 4-10 membered heterocyclic group, C₁-C₆ alkyl, C₁-C₆ alkoxy, halogen-substituted C₁-C₆ alkyl, and halogen-substituted C₁-C₆ alkoxy;
A₂ is selected from phenyl or pyridyl, wherein the phenyl or pyridyl is optionally substituted with one or more groups selected from hydrogen, deuterium, halogen, cyano, a 4-10 membered heterocyclic group, C₁-C₆ alkyl, C₁-C₆ alkoxy, halogen-substituted C₁-C₆ alkyl, halogen-substituted C₁-C₆ alkoxy, C₃-C₈ heterocycloalkoxy, C₃-C₈ cycloalkoxy, hydroxy-substituted C₁-C₆ alkoxy, and C₁-C₆ alkoxy-substituted C₁-C₆ alkoxy.

4. The compound according to claim 1, or a pharmaceutically acceptable salt, tautomer, stereoisomer, isotopic derivative, ester, prodrug, or hydrate thereof, wherein the compound has a structure represented by formulae (I-12)-(I-13): 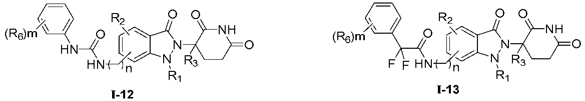 wherein:
R₁ is hydrogen, methyl or ethyl;
R₂ is hydrogen or fluorine;
R₃ is hydrogen, deuterium or fluorine;
n is 0 or 1;
each R₆ is independently selected from hydrogen, halogen, cyano, or C₁-C₆ alkyl;
m is 0, 1, 2, 3, 4 or 5.

5. The compound according to claim 1, or a pharmaceutically acceptable salt, tautomer, stereoisomer, isotopic derivative, ester, prodrug, or hydrate thereof, wherein the compound has a structure represented by formulae (I-31)-(I-32): 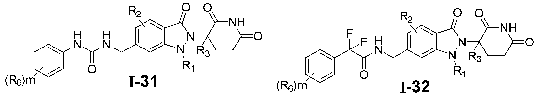 wherein:
R₁ is hydrogen, methyl or ethyl;
R₂ is hydrogen or fluorine;
R₃ is hydrogen, deuterium or fluorine;
each R₆ is independently selected from hydrogen, halogen, cyano, or C₁-C₆ alkyl;
m is 0, 1, 2, 3, 4 or 5.

6. The compound according to claim 1, or a pharmaceutically acceptable salt, tautomer, stereoisomer, isotopic derivative, ester, prodrug, or hydrate thereof, wherein the compound has a structure represented by formulae (I-20)-(I-26): 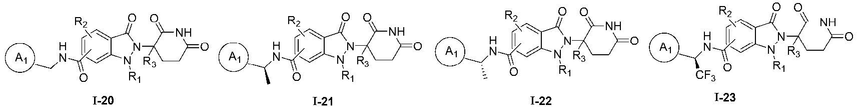 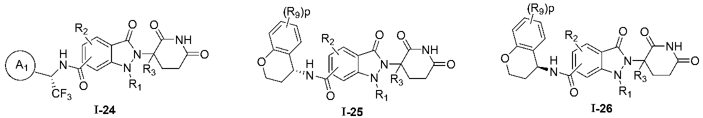 wherein:
R₁ is hydrogen, methyl or ethyl;
R₂ is hydrogen or fluorine;
R₃ is hydrogen, deuterium or fluorine;
each R₉ is independently selected from hydrogen, halogen, cyano, C₁-C₆ alkyl, C₁-C₆ alkoxy, halogen-substituted C₁-C₆ alkyl, or halogen-substituted C₁-C₆ alkoxy;
p is 0, 1, 2, 3, 4 or 5;
A₁ is selected from phenyl, pyridyl, substituted or unsubstituted C₃-C₁₀ cycloalkyl, 4-6 membered heterocyclyl, or C₁-C₆ alkyl; wherein the phenyl, pyridyl, substituted or unsubstituted C₃-C₁₀ cycloalkyl, 4-6 membered heterocyclyl, or C₁-C₆ alkyl is substituted with one or more groups selected from hydrogen, deuterium, halogen, cyano, a 4-10 membered heterocyclic group, C₁-C₆ alkyl, C₁-C₆ alkoxy, halogen-substituted C₁-C₆ alkyl, halogen-substituted C₁-C₆ alkoxy, C₃-C₈ heterocycloalkoxy, and C₃-C₈ cycloalkoxy.

7. The compound according to claim 1, or a pharmaceutically acceptable salt, tautomer, stereoisomer, isotopic derivative, ester, prodrug, or hydrate thereof, wherein from: 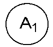 is selected 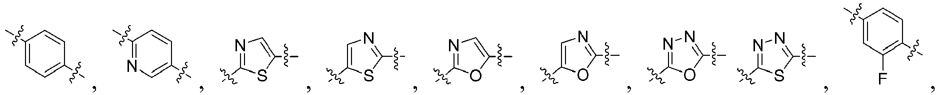 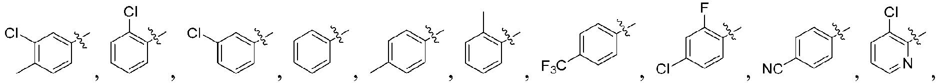 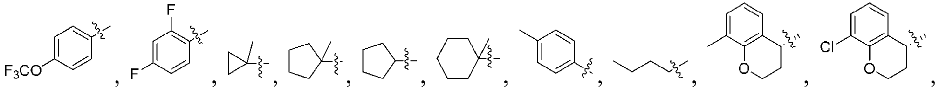 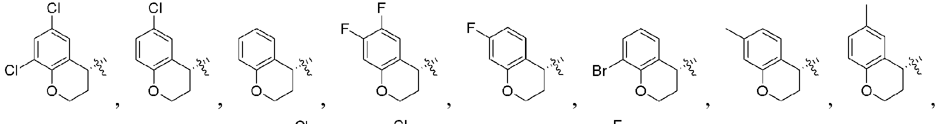 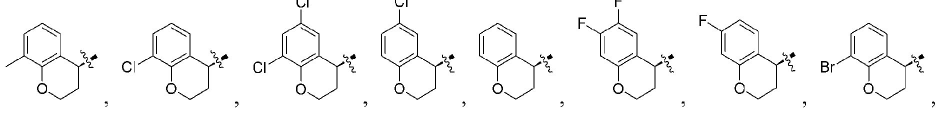 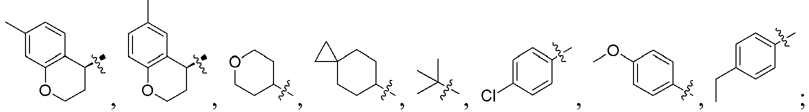 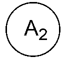 is selected from:

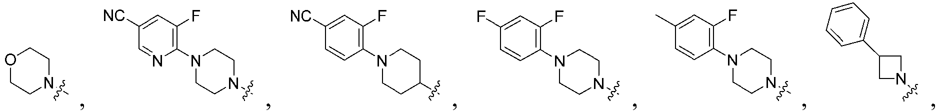

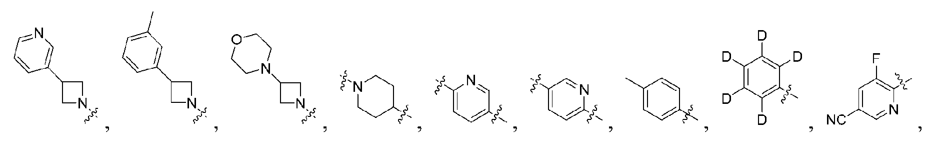

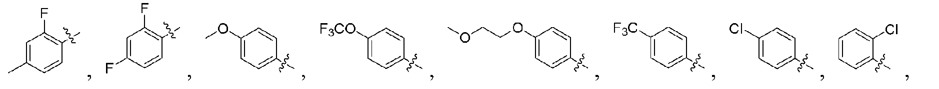

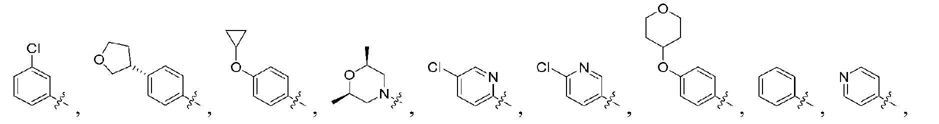

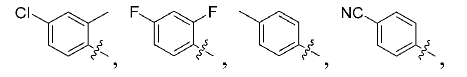

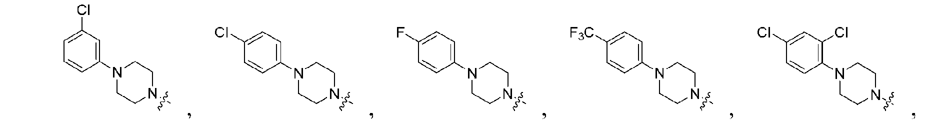

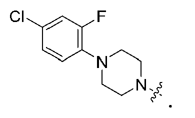

8. The compound according to claim 1, or a pharmaceutically acceptable salt, tautomer, stereoisomer, isotopic derivative, ester, prodrug, or hydrate thereof, wherein the compound is selected from the following compounds:
| **Compound No.** | **Structure** | **Compound No.** | **Structure** |
|---|---|---|---|
| **D1** | 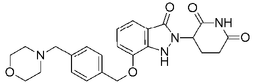 | **D2** | 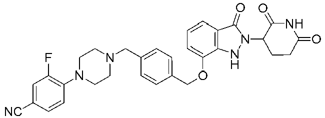 |
| **D3** | 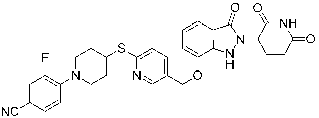 | **D4** | 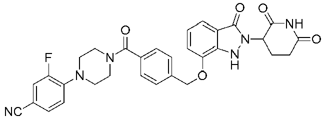 |
| **D5** | 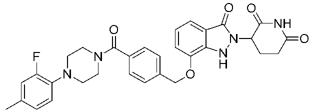 | **D6** | 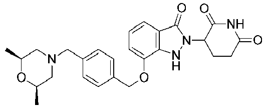 |
| **D7** | 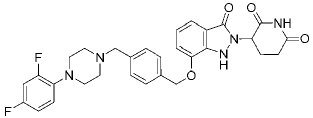 | **D8** | 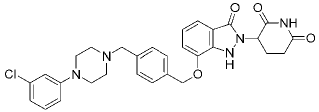 |
| **D9** | 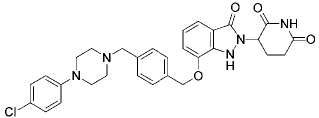 | **D10** | 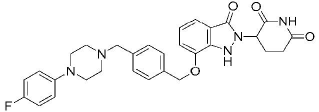 |
| **D11** | 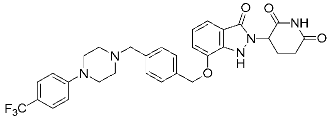 | **D12** | 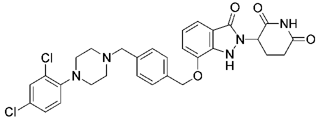 |
| **D13** | 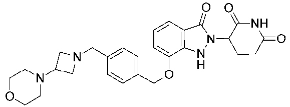 | **D14** | 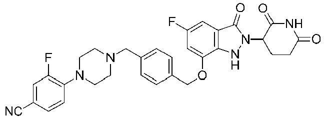 |
| **D15** | 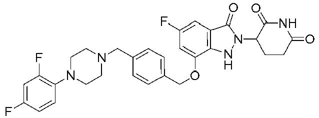 | **D16** | 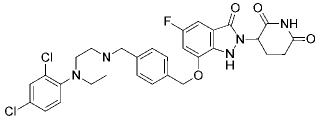 |
| **D17** | 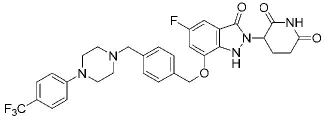 | **D18** | 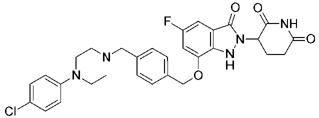 |
| **D19** | 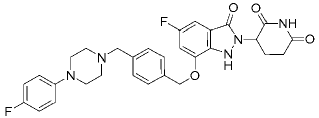 | **D20** | 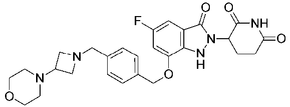 |
| **D21** | 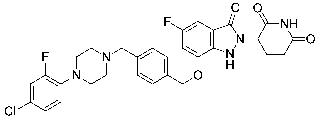 | **D22** | 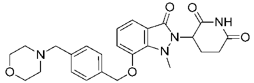 |
| **D23** | 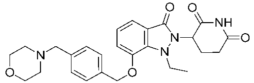 | **D24** | 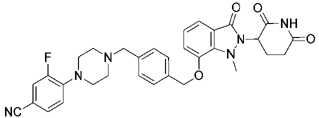 |
| **D25** | 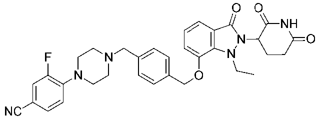 | **D26** | 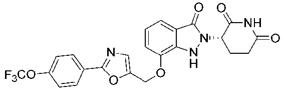 |
| **D27** | 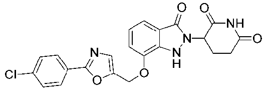 | **D28** | 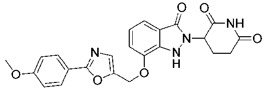 |
| **D29** | 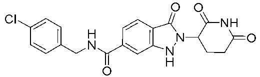 | **D30** | 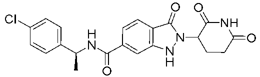 |
| **D31** | 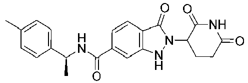 | **D32** | 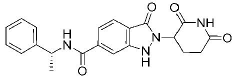 |
| **D33** | 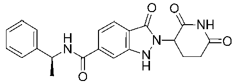 | **D34** | 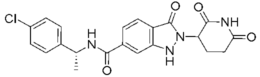 |
| **D35** | 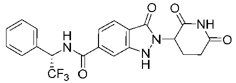 | **D36** | 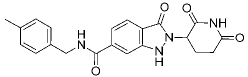 |
| **D37** | 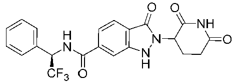 | **D38** | 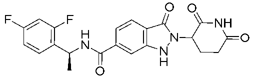 |
| **D39** | 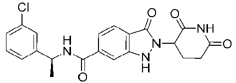 | **D40** | 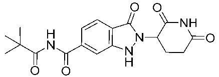 |
| **D41** | 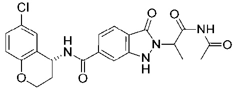 | **D42** | 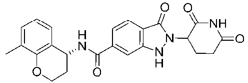 |
| **D43** | 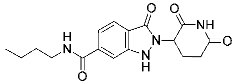 | **D44** | 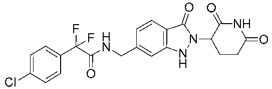 |
| **D45** | 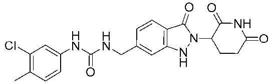 | **D46** | 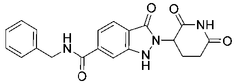 |
| **D47** | 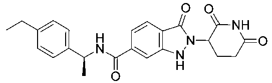 | | |
.

9. A method for preparing the compound according to claim 1, wherein the method is selected from one of the following methods:
Synthetic Method 1: 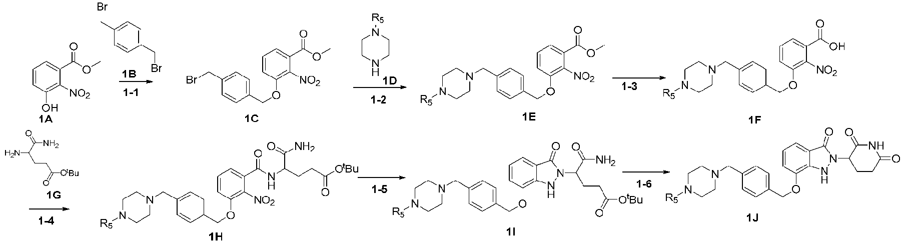
Step 1-1: Compound 1A and compound 1B undergo a nucleophilic substitution reaction to give compound 1C;
Step 1-2: Compound 1C and compound 1D undergo a nucleophilic substitution reaction to give compound 1E;
Step 1-3: Compound 1E is subjected to alkaline hydrolysis to give compound 1F;
Step 1-4: Compound 1F and compound 1G undergo a condensation reaction to give compound 1H;
Step 1-5: Compound 1H reacts with tetrahydroxydiboron and sodium hydroxide to give compound 1I;
Step 1-6: Compound 1I undergoes a reflux reaction with p-toluenesulfonic acid to give compound 1J;
R₅ is as defined in claim 2;
Synthetic Method 2: 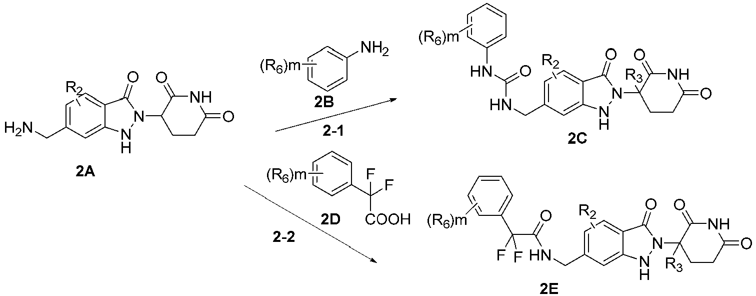
Step 2-1: Compound 2A and compound 2B react in the presence of N,N'-carbonyldiimidazole and triethylamine to give compound 2C;
Step 2-2: Compound 2A and compound 2D react in the presence of HATU and N,N-diisopropylethylamine to give compound 2E;
R₂, R₃, R₆, and m are as defined in claim 4;
Synthetic Method 3: 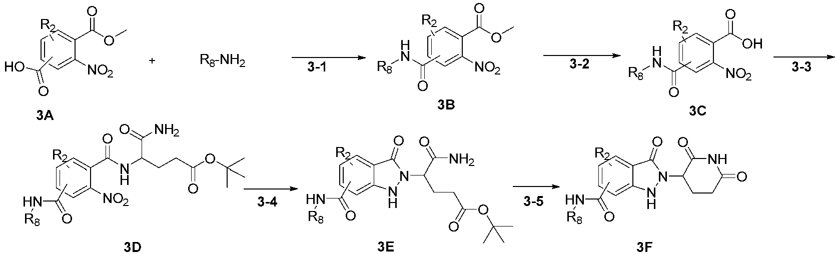
Step 3-1: Compound 3A and R8-NH₂ undergo a condensation reaction to give compound 3B;
Step 3-2: Compound 3B is hydrolyzed in the presence of lithium hydroxide to give compound 3C;
Step 3-3: Compound 3C and compound 1G undergo a condensation reaction to give compound 3D;
Step 3-4: Compound 3D reacts with tetrahydroxydiboron and sodium hydroxide to give compound 3E;
Step 3-5: Compound 3E undergoes a reflux reaction with p-toluenesulfonic acid to give compound 3F;
R₈-NH₂ is selected from: 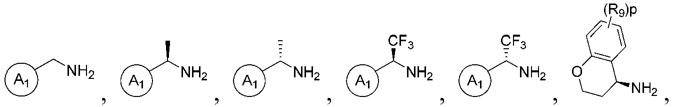 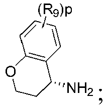 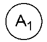 and R₉ is as defined in claim 6.

10. A pharmaceutical composition comprising a therapeutically effective amount of the compound according to the claims, or a tautomer, enantiomer, diastereomer, racemate, isotopic derivative, pharmaceutically acceptable salt, ester, prodrug, or hydrate thereof, and one or more pharmaceutically acceptable carriers.

11. The pharmaceutical composition according to claim 10, wherein the pharmaceutical composition further comprises one or more agents selected from dexamethasone, rituximab, trastuzumab, a PD-1 inhibitor, a PD-L1 inhibitor, pemetrexed, topotecan, doxorubicin, bortezomib, gemcitabine, dacarbazine, clarithromycin, vincristine, cytarabine, prednisone, docetaxel, clofarabine injection, an HDAC inhibitor, an androgen receptor inhibitor, an androgen biosynthesis inhibitor, a BTK inhibitor, erythropoietin, minocycline, elotuzumab, palbociclib, nivolumab, pembrolizumab, panobinostat, ublituximab, romidepsin, eltrombopag, CAR-T, and melphalan.

12. Use of the compound according to claim 1, or a tautomer, enantiomer, diastereomer, racemate, isotopic derivative, pharmaceutically acceptable salt, ester, prodrug, or hydrate thereof, in the manufacture of a medicament for preventing or treating a disease associated with the CRL4 ^{CRBN} E3 ubiquitin ligase.

13. The use according to claim 12, wherein the disease associated with the CRL4^{CRBN} E3 ubiquitin ligase is cancer, pain, a central nervous system disease, or an immune system disease.

14. The use according to claim 11, wherein the disease is a hematologic tumor or a solid tumor.

15. The use according to claim 14, wherein the disease is selected from myelodysplastic syndrome, multiple myeloma, mantle cell lymphoma, non-Hodgkin's lymphoma, chronic lymphocytic leukemia, chronic myelomonocytic leukemia, myelofibrosis, Burkitt lymphoma, Hodgkin's lymphoma, diffuse large B-cell lymphoma, follicular lymphoma, ciliary body and choroidal melanoma, iris melanoma, recurrent intraocular melanoma, T-cell lymphoma, erythroid lymphoma, monoblastic and monocytic leukemia, acute myeloid leukemia, central nervous system lymphoma, meningioma, spinal cord tumor, non-small cell lung cancer, ovarian cancer, skin cancer, renal cell carcinoma, astrocytoma, amyloidosis, complex regional pain syndrome type I, malignant melanoma, radiculopathy, glioblastoma, gliosarcoma, malignant glioma, refractory plasmacytoma, extraocular extension melanoma, papillary and follicular thyroid carcinoma, breast cancer, prostate cancer, hepatocellular carcinoma, primary macroglobulinemia, large cell lymphoma, myeloid leukemia, brain tumor, or thyroid cancer.
